# EUROPEAN PATENT APPLICATION

(11) **EP 4 786 455 A1**
(43) Date of publication of application: **05.08.2026**
(21) Application number: 24872658.0
(22) Date of filing: 28.06.2024
(51) Int. Cl.: C07D 237/34, A61K 31/5377, A61P 35/00

(54) **NOVEL PHTHALAZINE DERIVATIVE COMPOUND AS SOS1 INHIBITOR, AND USE THEREOF**

(30) Priority: 27.09.2023 KR 20230130062
(71) Applicant: Hanmi Pharmaceutical Co., Ltd., Hwaseong-si, Gyeonggi-do 18536 (KR)
(72) Inventor: KONG, Tae Yeon, Hwaseong-si Gyeonggi-do 18469 (KR); PARK, Won Gi, Hwaseong-si Gyeonggi-do 18469 (KR); BYUN, Joo Yun, Hwaseong-si Gyeonggi-do 18469 (KR); JUNG, Seung Hyun, Hwaseong-si Gyeonggi-do 18469 (KR); CHOI, Jae Yul, Hwaseong-si Gyeonggi-do 18469 (KR); KIM, Min Jeong, Hwaseong-si Gyeonggi-do 18469 (KR); AHN, Young Gil, Hwaseong-si Gyeonggi-do 18469 (KR)
(74) Representative: Schnappauf, Georg
(86) International application number: PCT/KR2024/009122
(87) International publication number: WO 2025/070947

(57) **Abstract**

The present invention relates to a novel q phthalazine derivative compound as a SOS1 inhibitor and to uses thereof. More particularly, the present invention relates to a novel phthalazine derivative compound having inhibitory activity on SOS1 binding to RAS family proteins and/or RAC1, to pharmacologically acceptable salts thereof, and to pharmaceutical compositions comprising such compounds.

## Description

### Technical Field

The present invention relates to a novel phthalazine derivative compound and a use thereof as a SOS1 inhibitor and, more particularly, to a novel phthalazine derivative compound having an activity of inhibiting SOS1 binding to RAS family proteins and/or RAC1, and to a pharmaceutical composition comprising a compound selected from the group consisting of the phthalazine derivative compound, optical isomers thereof, diastereomers thereof, hydrates thereof, solvates thereof, and pharmaceutically acceptable salts thereof.

### Background Art

RAS family proteins are found in approximately 20 to 30% of human cancers, and are known to include KRAS (Kirsten rat sarcoma viral oncogene homolog), NRAS (neuroblastoma RAS viral oncogene homolog), and HRAS (Harvey murine sarcoma virus oncogene). RAS regulates cell proliferation through, for example, the RAF/MEK/ERK pathway leading to activation of mitogen-activated protein kinase (MAPK) and the PI3K/Akt/mTOR pathway through phosphatidylinositide 3-kinase (PI3K). In the case of cancer-associated mutations in RAS family proteins, intrinsic and/or GAP-stimulated GTPase activity is suppressed, thereby increasing the pool of GTP-bound (active) RAS family proteins.

Meanwhile, RAS proteins function as molecular switches, in which GTP and GDP exist in cells in active (GTP-bound) and inactive (GDP-bound) states, respectively. Activated GTP-bound RAS recruits other proteins through binding of the homologous RAS-binding domain (RBD) to activate effector proteins, and then generates downstream signals having diverse functions. The activation state of RAS is regulated by guanine nucleotide exchange factors (GEFs) and GTPase activating proteins (GAPs). Binding of GAPs, such as NF1, increases the GTPase activity of RAS family proteins.

Binding of GEFs, such as SOS1 (Son of Sevenless 1), promotes GDP release from RAS family proteins, thereby allowing RAS family proteins to bind to and be activated by GTP. Son of Sevenless (SOS) proteins exist as two isoforms, SOS1 and SOS2, and only SOS1 is phosphorylated by ERK. Growth factor-induced phosphorylation of SOS1 is largely mediated by ERK, which phosphorylates at least four serine residues in the C-terminal region of SOS1. This suggests that SOS1 plays an important role in negative feedback regulation of the KRAS pathway.

The SOS1 protein consists of 1333 amino acids (150 kDa). SOS1 is a multi-domain protein having tandem N-terminal histone domains (HD), followed by a Dbl homology domain (DH), a plextrin homology domain (PH), a helical linker (HL), a RAS exchange motif (REM), a CDC25 homology domain, and a C-terminal proline rich domain (PR). SOS1 has two binding sites for RAS family proteins: a catalytic site that binds to GDP-bound RAS family proteins to promote guanine nucleotide exchange, and an allosteric site that binds to GTP-bound RAS family proteins to further increase the catalytic GEF function of SOS1. Selective pharmacological inhibition of SOS1 binding at the catalytic site to RAS family proteins is expected to prevent SOS1-mediated activation of RAS family proteins in the GTP-bound form.

Such SOS1 inhibitor compounds are consequently expected to inhibit downstream cellular signaling of RAS family proteins (e.g., ERK phosphorylation). Accordingly, novel SOS1 inhibitor compounds that bind to the catalytic site of SOS1 (confirmed by crystallography) and simultaneously prevent binding to and activation of RAS family proteins have been under development. Compounds having a significant inhibitory effect (low IC₅₀ values) on the interaction between SOS1 and RAS family proteins, particularly KRAS, and consequently causing a marked decrease in ERK phosphorylation in KRAS-mutant cancer cell lines have been under development.

The present inventors confirmed that a novel phthalazine derivative compound as a SOS1 inhibitor has an activity of inhibiting SOS1 binding to RAS family proteins and/or RAC1, thereby completing the present invention.

Related prior art is as follows.
1. Simanshu DK., et al. (2017) RAS proteins and their regulators in human disease. Cell, 170, 17-33.
2. Amy Y., et al. (2012) Oncogenic and wild type Ras play divergent roles in the regulation of mitogen-activated protein kinase signaling. Cancer Discov, 3(1), 112-23.

Pierre S., et al., (2011) Understanding SOS (Son of Sevenless). Biochem. Pharmacol., 82(9), 1049-56.

### DISCLOSURE

### Technical Problem

An object of the present invention is to provide a novel phthalazine derivative compound having excellent activity of inhibiting SOS1 binding to RAS family proteins and/or RAC1.

Another object of the present invention is to provide a pharmaceutical composition comprising the compound in a therapeutically effective amount.

### Technical Solution

According to one embodiment of the present invention, there is provided a compound selected from the group consisting of a compound represented by the following Formula 1, optical isomers thereof, diastereomers thereof, hydrates thereof, solvates thereof, and pharmaceutically acceptable salts thereof: 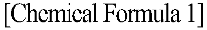 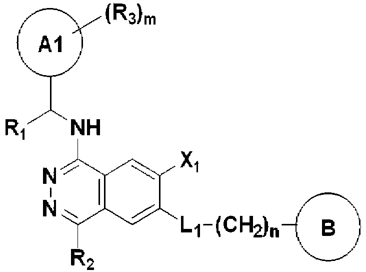 In Chemical Formula 1,
R₁ is hydrogen or C₁₋₄ alkyl;
R₂ is hydrogen, C₁₋₄ alkyl, halo C₁₋₄ alkyl, C₂₋₆ alkenyl or C₂₋₆ alkynyl;
R₃ is R₃ₐ or -L₂- 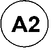 ;
R₃ₐ are each independently any one selected from halogen, hydroxy, cyano, amino, amine, nitro, oxo (=O), C₁₋₆ alkyl, C₂₋₆ alkenyl, C₂₋₆ alkynyl, halo C₁₋₆ alkyl, amino C₁₋₆ alkyl, C₁₋₆ alkoxy, hydroxy-C₁₋₄ alkyl, -CF₂H, -(CH₂)ᵣ-NH(CO)-Rₐ, -(CH₂)ᵣ-NRₐR_{b}, and Rₐ and R_{b} are each independently any one selected from the group consisting of hydrogen, C₁₋₆ alkyl, halo C₁₋₆ alkyl, hydroxy-C₁₋₆ alkyl, C₂₋₆ alkenyl, C₂₋₆ alkynyl and C₃₋₈ carbocyclyl; and wherein the C₁₋₆ alkyl, C₂₋₆ alkenyl, C₂₋₆ alkynyl, amino C₁₋₆ alkyl, C1-6 alkoxy, and hydroxy-C₁₋₄ alkyl may be substituted with one or more halogen atoms;
r is an integer in the range of 0 to 1;
m is an integer in the range of 0 to 5;
L₂ is a direct bond, -O-(CH₂)ₚ, or -CH=CH-(CH₂)_{q};
p is an integer in the range of 0 to 3;
q is an integer in the range of 0 to 2;
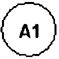 and 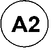 are each independently C₆₋₁₀ aryl, C₄₋₁₀ heteroaryl, C₃₋₁₀ carbocyclyl, C₂₋₁₀ heterocyclyl, or C₉₋₁₂ bicyclic heterocyclyl, wherein C₆₋₁₀ aryl, C₄₋₁₀ heteroaryl, C₃₋₁₀ carbocyclyl, C₂₋₁₀ heterocyclyl, or C₉₋₁₂ bicyclic heterocyclyl of 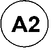 is unsubstituted or substituted with one or more R₃ₐ;
X₁ is -O(R₄) or -N(R₅)(R₆);
R₄ is hydrogen, C₁₋₆ alkyl, hydroxy C₁₋₆ alkyl, C₂₋₆ alkenyl, C₂₋₆ alkynyl C₃₋₁₀ carbocyclyl, C₂₋₉ heterocyclyl, C₆₋₁₀ aryl, C₄₋₁₀ heteroaryl, C₈₋₁₆ spirocarbocyclyl, C₆₋₁₄ heterospirocarbocyclyl, C₈₋₁₆ fused carbocyclyl, C₆₋₁₄ fused heterocyclyl, C₈₋₁₆ bridge carbocyclyl, or C₆₋₁₄ bridged heterocyclyl, which is unsubstituted or substituted with halogen, hydroxy, nitro, oxo (=O), cyano, halo C₁₋₆ alkyl, C₁₋₆ alkyl, C₂₋₆ alkenyl, C₂₋₆ alkynyl, halo C₁₋₆ alkoxy, -S(O)-C₁₋₄ alkyl, -S(O)₂-C₁₋₄ alkyl, -C(O)-NR_{c}R_{d}, -C(O)OR_{c}, -OR_{c}, or -NR_{c}R_{d}, wherein R_{c} and R_{d} are each independently hydrogen or C₁₋₆ alkyl;
R₅ and R₆ are each hydrogen, C₁₋₆ alkyl, hydroxy C₁₋₆ alkyl, C₂₋₆ alkenyl, C₂₋₆ alkynyl, C₃₋₁₀ carbocyclyl, C₂₋₉ heterocyclyl, C₆₋₁₀ aryl, C₄₋₁₀ heteroaryl, C₈₋₁₆ spirocarbocyclyl, C₆₋₁₄ heterospirocarbocyclyl, C₈₋₁₆ fused carbocyclyl, C₆₋₁₄ fused heterocyclyl, C₈₋₁₆ crosslinked car bocyclyl, or C₆₋₁₄ bridged heterocyclyl, wherein C₁₋₆ alkyl, hydroxy C₁₋₆ alkyl, C₂₋₆ alkenyl, C₂₋₆ alkynyl, C₃₋₁₀ carbocyclyl, C₂₋₉ heterocyclyl, C₆₋₁₀ aryl, C₄₋₁₀ heteroaryl, C₈₋₁₆ spirocarbocyclyl, C₆₋₁₄ heterospirocarbocyclyl, C₈₋₁₆ fused carbocyclyl, C₆₋₁₄ fused heterocyclyl, C₈₋₁₆ bridged carbocyclyl, or C₆₋₁₄ bridged heterocyclyl is unsubstituted or substituted with one or more functional groups selected from the group consisting of halogen, hydroxy, nitro, oxo (=O), cyano, halo C₁₋₆ alkyl, C₁₋₆ alkyl, C₂₋₆ alkenyl, C₂₋₆ alkynyl, C₁₋₆ alkoxy, halo C₁₋₆ alkoxy, -S(O)-C₁₋₄ alkyl, -S(O)₂-C₁₋₄ alkyl, - C(O)-NRₑR_{f}, -C(O)ORₑ, -ORₑ, and -NRₑR_{f}, wherein Rₑ and R_{f} are each independently hydrogen or C₁₋₆ alkyl, and
alternatively, the -N(R₅)(R₆) is C₂₋₉ heterocyclyl, C₆₋₁₄ heterospirocarbocyclyl, C₆₋₁₄ fused heterocyclyl, C₆₋₁₄ bridged heterocyclyl, or C₄₋₁₀ heteroaryl in each of which R₅ and R₆ are linked to each other and form a ring in conjunction with a nitrogen atom, wherein C₂₋₉ heterocyclyl, C₆₋₁₄ heterospirocarbocyclyl, C₆₋₁₄ fused heterocyclyl, C₆₋₁₄ bridged heterocyclyl, or C₄₋₁₀ heteroaryl is unsubstituted or substituted with one or more functional groups selected from the group consisting of halogen, hydroxy, nitro, oxo (=O), cyano, halo C₁₋₆ alkyl, C₁₋₆ alkyl, C₂₋₆ alkenyl, C₂₋₆ alkynyl, C₁₋₆ alkoxy, halo C₁₋₆ alkoxy, -S(O)-C₁₋₄ alkyl, -S(O)₂-C₁₋₄ alkyl, -C(O)-NR_{g}Rₕ, -C(O)OR_{g}, -OR_{g}, and -NR_{g}Rₕ, wherein R_{g} and Rₕ are each independently hydrogen, C₁₋₆ alkyl, hydroxy-C₁₋₆ alkyl, C₂₋₆ alkenyl, C₂₋₆ alkynyl, C₁₋₆ alkoxy, or C₃₋₈ carbocyclyl;
L₁ is a direct bond, -C(O)-, -O-, -NH-, -C(O)NH-, or -C(O)NCH₃-;
n is an integer in the range of 0 to 2; and
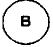 iis C₃₋₁₀ carbocyclyl, C₂₋₉ heterocyclyl, C₆₋₁₀ aryl, C₄₋₁₀ heteroaryl, C₄₋₁₄ heterospirocarbocyclyl, C₄₋₁₄ fused heterocyclyl, or C₄₋₁₄ bridged heterocyclyl, wherein C₃₋₁₀ carbocyclyl, C₂₋₉ heterocyclyl, C₆₋₁₀ aryl, C₄₋₁₀ heteroaryl, C₄₋₁₄ heterospirocarbocyclyl, C₄₋₁₄ fused heterocyclyl, or C₄₋₁₄ bridged heterocyclyl is unsubstituted or substituted with one or more functional groups selected from the group consisting of halogen, hydroxy, nitro, oxo (=O), halo C₁₋₆ alkyl, C₁₋₆ alkyl, C₂₋₆ alkenyl, C₂₋₆ alkynyl, C₁₋₆ alkoxy, -S(O)-C₁₋₄ alkyl, -S(O)₂-C₁₋₄ alkyl, -C(O)-NRᵢRⱼ, - C(O)ORᵢ, -ORᵢ, -NRᵢRⱼ, _{C3-10} carbocyclyl, and C₂₋₉ heterocyclyl, wherein Rᵢ and Rⱼ are each independently hydrogen or C₁₋₆ alkyl.

According to another embodiment of the present invention, there is provided a pharmaceutical composition and pharmaceutical preparation for the prevention or treatment of various diseases related to inhibition of SOS1 binding to RAS family proteins and/or RAC1, the composition and preparation including the compound described above in a therapeutically effective amount.

According to a further embodiment of the present invention, there is provided a method of inhibiting SOS1 binding to a RAS family protein and/or RAC1 in a subject or cell, the method including administering a pharmaceutically effective amount of the compound to the subject.

According to a yet further embodiment of the present invention, there is provided a method of inhibiting tyrosine kinase in a subject or cell, the method including administering a pharmaceutically effective amount of the compound to the subject.

According to a yet further embodiment of the present invention, there is provided a method of for preventing or treating cancer or tumor in a subject, the method including administering to the subject a pharmaceutically effective amount of the compound.

According to a yet further embodiment of the present invention, there are provided uses of the compounds or pharmaceutically acceptable salts described above for the prevention or treatment of cancer or tumors.

### Advantageous Effects

The phthalazine derivative compound of Chemical Formula 1 in the present invention has excellent ability to inhibit SOS1 binding to RAS family proteins and/or RAC1, has anticancer activity against cancer associated with cell proliferation attributable to abnormal SOS1 activity, and can be usefully used as a therapeutic agent.

### Mode for Invention

All technical terms used in the present invention, unless otherwise defined, have the meanings commonly understood by the ordinarily skilled in the related art of the present invention. In addition, although preferred methods and samples are described herein, similar or equivalent ones also fall within the scope of the present invention. In addition, the numerical values described herein are considered to include the meaning of "about" even if not specified. The contents of all publications incorporated herein by reference are hereby incorporated by reference in their entirety.

In Chemical Formula 1, residues listed as R₁ to R₆ are used as commonly understood by those skilled in the art.

In the present invention, the term "halogen" refers to fluorine, chlorine, bromine, or iodine, unless otherwise specified. Specifically, the term "halogen" may refer to fluorine or chlorine but may not be limited thereto.

As used herein, the term "alkyl" refers to a saturated, straight-chain or branched monovalent hydrocarbon radical, unless otherwise specified.

In the present invention, the term "alkenyl", unless otherwise specified, refers to a monovalent hydrocarbon radical containing at least one carbon-carbon double bond, each double bond having a three-dimensional E- or Z-arrangement.

The term "alkynyl" as used herein, unless otherwise specified, refers to a monovalent group derived from an unsaturated, straight-chain or branched hydrocarbon moiety having at least one carbon-carbon triple bond.

These alkyl, alkenyl, and alkynyl groups may be straight (i.e., straight-chained) or branched. Depending on the definition, the number of carbon atoms in the alkyl group may be 1, 2, 3, 4, 5 or 6 or may be 1, 2, 3, or 4. Examples of alkyl include: methyl; ethyl; propyl including n-propyl and isopropyl; butyl including n-butyl, sec-butyl, isobutyl, and tert-butyl; pentyl including n-pentyl, 1-methylbutyl, isopentyl, neopentyl, and tert-pentyl; and hexyl including n-hexyl, 3,3-dimethylbutyl, and isohexyl. Each of the double and triple bonds in the alkenyl group and the alkynyl group may be present at any position. Examples of alkenyl and alkynyl are ethenyl, prop-1-enyl, prop-2-enyl (= allyl), but-2-enyl, 2-methylprop-2-enyl, 3-methylbut- 2-enyl, hex-3-enyl, hex-4-enyl, prop-2-ynyl (= propargyl), but-2-ynyl, but-3-ynyl, hex-4-ynyl, or hex-5-ynyl. However, each of the alkyl, alkenyl, and alkynyl groups may substitute at any position, as long as each compound is sufficiently stable and suitable for a desired purpose such as use as a pharmaceutical substance.

In the present invention, the term "carbocyclyl" refers to cyclic alkyl which may be substituted or unsubstituted and may refer to mono or bicycloaliphatic unless otherwise specified. Preferably, examples of the carbocyclyl include, but are not limited to, aryl, carbocyclyl, spirocarbocyclyl, fused carbocyclyl, and bridged carbocyclyl. More preferably, examples of the - 1carbocyclyl include cyclopropyl, cyclobutyl, cyclopentyl, cyclohexyl, cyclohexenyl, cycloheptyl, cyclohepsenyl, cyclooctyl, cyclooctenyl, 2,5-cyclohexadienyl, bicyclo[2.2.2]octyl, adamant-1-yl, decahydronaphthyl, oxocyclohexyl, dioxocyclohexyl, thiocyclohexyl, 2-oxobicyclo[2.2.1]hept-1-enyl, or all possible isomers thereof without limitation.

In the present invention, the term "heterocyclyl" refers to a monocyclic, bicyclic, or multicyclic alkyl that contains one or more (specifically 1, 2, 3, or 4) heteroatoms selected from O, N, and S and which may be substituted or unsubstituted. Preferably, examples of the heterocyclyl include, but are not limited to, heteroaryl, heterocyclyl, heterospirocarbocyclyl, fused heterocyclyl, and bridged heterocyclyl. More preferably, examples of the heterocyclyl include, but are not limited to, piperazinyl, piperidinyl, piperazinyl-1-oxide, morpholinyl, thiamorpholinyl, pyrrolidinyl, imidazolinyl, tetrahydrofuranyl, diazabicyclo octanyl, diazaspirooctanyl, and similar groups thereto. For example, in the case of C₂₋₁₀ heterocyclyl, C₂₋₁₀ representing a carbon number means a ring size of a 3-membered or greater-numbered ring including one or more heteroatoms.

In the present invention, the term "aryl" refers to an aromatic group that may be substituted or unsubstituted, unless otherwise specified. For example, the aryl includes phenyl, biphenyl, naphthyl, toluyl, naphthalenyl, anthracenyl, and all isomers thereof without limitation.

In the present invention, the term "heteroaryl" refers to a monocyclic, bicyclic, or multicyclic aromatic group that contains one or more (specifically 1, 2, 3, or 4) heteroatoms selected from O, N, and S and which may be substituted or unsubstituted. Preferably, examples of the monocyclic heteroaryl include thiazolyl, oxazolyl, thiophenyl, furanyl, pyrrolyl, imidazolyl, isoxazolyl, pyrazolyl, triazolyl, thiadiazolyl, tetrazolyl, oxadiazolyl, pyridinyl, pyridazinyl, pyrimidinyl, pyrazinyl, and the like but are not limited thereto. Preferably, examples of the bicyclic heteroaryl include indolyl, benzothiophenyl, benzofuranyl, benzimidazolyl, benzoxazolyl, benzisoxazolyl, benzthiazolyl, benzthiadiazolyl, benztriazolyl, quinolineyl, isoquinolinyl, purinyl, puropyridinyl, and the like, but are not limited thereto.

In the present invention, the numerical range indicated using the term "to" refers to a range including the numerical values described before and after the term "to" as the lower limit and the upper limit, respectively.

As used herein, the term "SOS1 binding to RAS family protein and/or RAC1" refers to binding between SOS1 and RAS family protein at the catalytic site of SOS1, and "activity inhibiting binding" refers to that a GTP-binding form prevents SOS1-mediated activation of RAS-family proteins.

As used herein, the term "SOS1 inhibitor compound" refers to a compound that inhibits signal transduction to lower cells by RAS-family proteins, for example, inhibits ERK phosphorylation. Specifically, "SOS1 inhibitor compound" means a compound that binds to the SOS1 catalytic site, thereby preventing SOS1 from binding to RAS family proteins or preventing activation of RAS family proteins.

In the present invention, the term "enantiomer" refers to various possible stereoisomers and geometric isomers of the compound according to the present invention, unless otherwise stated. Since the compounds of Chemical Formula 1 according to an aspect of the present invention may have an asymmetric carbon center (absent carbon), the compounds may exist as enantiomers (R or S isomers), racemates, diastereomers, or any mixtures thereof. All these isomers and mixtures fall within the scope of the present invention. The optically active (R)- and (S)- isomers can be resolved using conventional techniques or prepared using chiral synthons or chiral reagents. When the compounds contain a double bond, the substituent may be in an E or Z form. When the compound contains a disubstituted carbocyclyl, the compound may be in a cis- or trans form. In addition, when the compound of Chemical Formula 1 includes a bridged ring, the compound may exist as an exo or endo isomer. In addition, all tautomers may also be included.

In the present invention, the term "asymmetric carbon atom" refers to a carbon atom in a molecule that contains four different atoms, atomic groups, or functional groups bonded to the carbon atom, unless otherwise stated. In the case of a compound containing such an asymmetric carbon atom, the compound has a photorotation property or optical isomers. Specifically, the compound having the structure of Chemical Formula 1 having the asymmetric carbon atom may be a compound having the structure of Chemical Formula 1a or Chemical Formula 1b below. On the other hand, a 1:1 mixture of a pair of enantiomers is referred to as a "racemic" mixture.

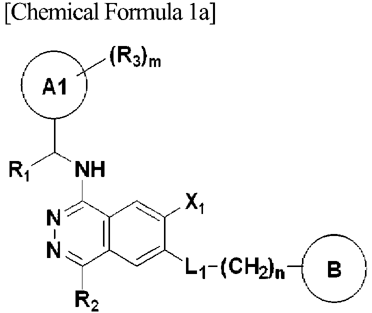

In Chemical Formula 1a, 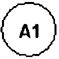 , R₁ to R₃, X₁, L₁, 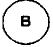 , m, and n are defined in the same manner as in Chemical Formula 1.

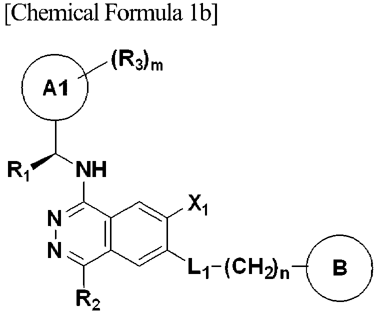

In one aspect, the compound of Chemical Formula 1, an optical isomer thereof, and a diastereomer thereof may exist in the form of a solvate. The term "solvate" may include molecular complexes, each containing the compound and one or more pharmaceutically acceptable solvent molecules, such as ethanol or water. Complexes in which the solvent molecule is water are referred to as "hydrates".

In one aspect, the compound of Chemical Formula 1, an optical isomer thereof, and a diastereomer thereof, and a solvate thereof may exist in the form of a pharmaceutically acceptable salt.

In the present invention, the term "pharmaceutically acceptable salt" may refer to, for example, inorganic acid salts, organic acid salts, and metal salts that are generally used by pharmaceutical manufacturers in preparing pharmaceuticals. Pharmaceutically acceptable salts include acid addition salts formed by a pharmaceutically acceptable free acid and a base compound of Chemical Formula 1, alkali metal salts (such as sodium salts) and alkaline earth metal salts (such as calcium salts), organic base addition salts formed by an organic base and a carboxylic acid structure of Chemical Formula 1, and amino acid addition salts, but are not limited thereto.

Preferred salt forms of the compounds according to the invention include salts with inorganic or organic acids. In this case, as the inorganic acid, hydrochloric acid, sulfuric acid, nitric acid, phosphoric acid, perchloric acid, bromic acid, and the like may be used. In addition, as the organic acid, acetic acid, methanesulfonic acid, ethanesulfonic acid, p-toluenesulfonic acid, fumaric acid, maleic acid, malonic acid, phthalic acid, succinic acid, lactic acid, citric acid, citric acid, gluconic acid, tartaric acid, salicylic acid, malic acid, oxalic acid, benzoic acid, embonic acid, aspartic acid, glutamic acid, and the like may be used. Organic bases that can be used to prepare organic base addition salts are tris(hydroxymethyl)methylamine, dicyclohexylamine, and the like. Amino acids that can be used to prepare amino acid addition salts are natural amino acids such as alanine and glycine. It will be apparent to those of ordinary skill in the art that other acids or bases may be used aside from the inorganic acids, organic acids, organic bases, and amino acids exemplified above.

The salts may be prepared by a conventional method. For example, the salts can be prepared by: dissolving the compound of Chemical Formula 1 in a solvent miscible with water, such as methanol, ethanol, acetone, and 1,4-dioxane; adding a free acid or a free base to the resulting solution; and crystallizing the resulting product.

The details in the description of the pharmaceutical composition according to one aspect of the present invention are applicable, as they are, to a prevention or treatment method according to another aspect of the present invention.

In the present invention, the term "treatment" is used as a concept including all meanings of therapy, improvement, amelioration, or management of a disease.

As used herein, the term "preventing" or "prevention" refers to preventing a disease, condition, or disorder in a subject who may be predisposed to the disease, condition, or disorder but does not yet experience or exhibit the pathology or signs of the disease.

As used herein, the term "subject" or "patient" refers to any mammal, for example, an animal, including mice, rats, other rodents, rabbits, dogs, cats, pigs, cattle, sheep, horses, primates, and humans.

Hereinafter, the present invention will be described in more detail.

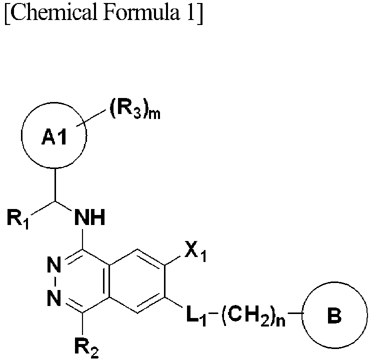

In Chemical Formula 1,
R₁ is hydrogen or C₁₋₄ alkyl;
R₂ is hydrogen, C₁₋₄ alkyl, halo C₁₋₄ alkyl, C₂₋₆ alkenyl or C₂₋₆ alkynyl;
R₃ is R₃ₐ or -L₂- 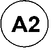
R₃ₐ are each independently halogen, hydroxy, cyano, amino, amine, nitro, oxo (=O), C₁₋₆ alkyl, C₂₋₆ alkenyl, C₂₋₆ alkynyl, halo C₁₋₆ alkyl, amino C₁₋₆ alkyl, C₁₋₆ alkoxy, hydroxy-C₁₋₄ alkyl, - CF₂H, -(CH₂)ᵣ-NH(CO)-Rₐ, or -(CH₂)ᵣ-NRₐR_{b}, in which Rₐ and R_{b} are each independently any one selected from the group consisting of hydrogen, C₁₋₆ alkyl, halo C₁₋₆ alkyl, hydroxy-C₁₋₆ alkyl, C₂₋₆ alkenyl, C₂₋₆ alkynyl, and C₃₋₈ carbocyclyl; and wherein the _{C1-6} alkyl, C₂₋₆ alkenyl, C₂₋₆ alkynyl, amino C₁₋₆ alkyl, C₁₋₆ alkoxy, and hydroxy-C₁₋₄ alkyl may be substituted with one or more halogen atoms;
r is an integer in the range of 0 to 1;
m is an integer in the range of 0 to 5;
L₂ is a direct bond, -O-(CH₂)ₚ, or -CH=CH-(CH₂)_{q};
p is an integer in the range of 0 to 3;
q is an integer in the range of 0 to 2;
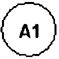 and 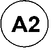 are each independently C₆₋₁₀ aryl, C₄₋₁₀ heteroaryl, C₃₋₁₀ carbocyclyl, C₂₋₁₀ heterocyclyl, or C₉₋₁₂ bicyclic heterocyclyl, in which C₆₋₁₀ aryl, C₄₋₁₀ heteroaryl, C₃₋₁₀ carbocyclyl, C₂₋₁₀ heterocyclyl, or C₉₋₁₂ bicyclic heterocyclyl of 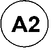 is unsubstituted or substituted with one or more R₃ₐ;
X₁ is -O(R₄) or -N(R₅)(R₆);
R₄ is hydrogen, C₁₋₆ alkyl, hydroxy C₁₋₆ alkyl, C₂₋₆ alkenyl, C₂₋₆ alkynyl, C₃₋₁₀ carbocyclyl, C₂₋₉ heterocyclyl, C₆₋₁₀ aryl, C₄₋₁₀ heteroaryl, C₈₋₁₆ spirocarbocyclyl, C₆₋₁₄ heterospirocarbocyclyl, C₈₋₁₆ fused carbocyclyl, C₆₋₁₄ fused heterocyclyl, C₈₋₁₆ bridge carbocyclyl, or C₆₋₁₄ bridged heterocyclyl each of which is unsubstituted or substituted with halogen, hydroxy, nitro, oxo (=O), cyano, halo C₁₋₆ alkyl, C₁₋₆ alkyl, C₂₋₆ alkenyl, C₂₋₆ alkynyl, C₁₋₆ alkoxy, halo C₁₋₆ alkoxy, -S(O)-C₁₋₄ alkyl, -S(O)₂-C₁₋₄ alkyl, -C(O)-NR_{c}R_{d}, -C(O)OR_{c}, -OR_{c}, or -NR_{c}R_{d}, in which R_{c} and R_{d} are each independently hydrogen or C₁₋₆ alkyl;
R₅ and R₆ are each hydrogen, C₁₋₆ alkyl, hydroxy C₁₋₆ alkyl, C₂₋₆ alkenyl, C₂₋₆ alkynyl, C₃₋₁₀ carbocyclyl, C₂₋₉ heterocyclyl, C₆₋₁₀ aryl, C₄₋₁₀ heteroaryl, C₈₋₁₆ spirocarbocyclyl, C₆₋₁₄ heterospirocarbocyclyl, C₈₋₁₆ fused carbocyclyl, C₆₋₁₄ fused heterocyclyl, C₈₋₁₆ bridged carbocyclyl, or C₆₋₁₄ bridged heterocyclyl, wherein C₁₋₆ alkyl, hydroxy C₁₋₆ alkyl, C₂₋₆ alkenyl, C₂₋₆ alkynyl, C₃₋₁₀ carbocyclyl, C₂₋₉ heterocyclyl, C₆₋₁₀ aryl, C₄₋₁₀ heteroaryl, C₈₋₁₆ spirocarbocyclyl, C₆₋₁₄ heterospirocarbocyclyl, C₈₋₁₆ fused carbocyclyl, C₆₋₁₄ fused heterocyclyl, C₈₋₁₆ bridged carbocyclyl, or C₆₋₁₄ bridged heterocyclyl is unsubstituted or substituted with one or more functional groups selected from the group consisting of halogen, hydroxy, nitro, oxo (=O), cyano, halo C₁₋₆ alkyl, C₁₋₆ alkyl, C₂₋₆ alkenyl, C₂₋₆ alkynyl, C₁₋₆ alkoxy, halo C₁₋₆ alkoxy, -S(O)-C₁₋₄ alkyl, -S(O)₂-C₁₋₄ alkyl, - C(O)-NRₑR_{f}, -C(O)ORₑ, -ORₑ, and -NRₑR_{f}, wherein Rₑ and R_{f} are each independently hydrogen or C₁₋₆ alkyl, or
alternatively, the -N(R₅)(R₆) is C₂₋₉ heterocyclyl, C₆₋₁₄ heterospirocarbocyclyl, C₆₋₁₄ fused heterocyclyl, C₆₋₁₄ bridged heterocyclyl, or C₄₋₁₀ heteroaryl in each of which R₅ and R₆ are linked to each other and form a ring in conjunction with a nitrogen atom contained in the -N(R₅)(R₆), in which C₂₋₉ heterocyclyl, C₆₋₁₄ heterospirocarbocyclyl, C₆₋₁₄ fused heterocyclyl, C₆₋₁₄ bridged heterocyclyl, or C₄₋₁₀ heteroaryl is unsubstituted or substituted with one or more functional groups selected from the group consisting of halogen, hydroxy, nitro, oxo (=O), cyano, halo C₁₋₆ alkyl, C₁₋₆ alkyl, C₂₋₆ alkenyl, C₂₋₆ alkynyl, C₁₋₆ alkoxy, halo C₁₋₆ alkoxy, -S(O)-C₁₋₄ alkyl, -S(O)₂-C₁₋₄ alkyl, -C(O)-NR_{g}Rₕ, -C(O)OR_{g}, -OR_{g}, and -NR_{g}Rₕ, wherein R_{g} and Rₕ are each independently hydrogen, C₁₋₆ alkyl, hydroxy-C₁₋₆ alkyl, C₂₋₆ alkenyl, C₂₋₆ alkynyl, C₁₋₆ alkoxy, or C₃₋₈ carbocyclyl;
L₁ is a direct bond, -C(O)-, -O-, -NH-, -C(O)NH-, or -C(O)NCH₃-;
n is an integer in the range of 0 to 2; and
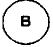 is C₃₋₁₀ carbocyclyl, C₂₋₉ heterocyclyl, C₆₋₁₀ aryl, C₄₋₁₀ heteroaryl, C₄₋₁₄ heterospirocarbocyclyl, C₄₋₁₄ fused heterocyclyl, or C₄₋₁₄ bridged heterocyclyl, in which C₃₋₁₀ carbocyclyl, C₂₋₉ heterocyclyl, C₆₋₁₀ aryl, C₄₋₁₀ heteroaryl, C₄₋₁₄ heterospirocarbocyclyl, C₄₋₁₄ fused heterocyclyl, or C₄₋₁₄ bridged heterocyclyl is unsubstituted or substituted with one or more functional groups selected from the group consisting of halogen, hydroxy, nitro, oxo (=O), halo C₁₋₆ alkyl, C₁₋₆ alkyl, C₂₋₆ alkenyl, C₂₋₆ alkynyl, C₁₋₆ alkoxy, -S(O)-C₁₋₄ alkyl, -S(O)₂-C₁₋₄ alkyl, -C(O)-NRᵢRⱼ, - C(O)ORᵢ, -ORᵢ, -NRᵢRⱼ, C₃₋₁₀ carbocyclyl, and C₂₋₉ heterocyclyl,wherein Rᵢ and Rⱼ are each independently hydrogen or C₁₋₆ alkyl.

Preferably, in a compound selected from the compound of Chemical Formula 1 of the present invention, optical isomers thereof, diastereomers thereof, hydrates thereof, and solvates thereof, and pharmaceutically acceptable salts thereof, 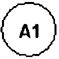 and 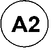 are each independently C₆₋₁₀ aryl or C₄₋₁₀ heteroaryl.

Preferably, in a compound selected from the compound of Chemical Formula 1 of the present invention, optical isomers thereof, diastereomers thereof, hydrates thereof, and solvates thereof, pharmaceutically acceptable salts thereof, R₃ₐ may be each independently halogen, hydroxy, cyano, amino, amine, nitro, C₁₋₆ alkyl, amino C₁₋₆ alkyl, C₁₋₆ alkoxy, -CF₂H, -(CH₂)ᵣ-NH(CO)-Rₐ, or -(CH₂)ᵣ-NRₐR_{b}, in which Rₐ and R_{b} may be each independently hydrogen, C₁₋₆ alkyl, -CF₃, or -CF₂H, and wherein the C₁₋₆ alkyl may be substituted with one or more halogen atoms.

Preferably, in a compound selected from the compound of Chemical Formula 1 of the present invention, optical isomers thereof, diastereomers thereof, hydrate thereof, and solvates thereof, pharmaceutically acceptable salts thereof, and R₄ is hydrogen, C₁₋₆ alkyl, C₂₋₆ alkenyl, C₂₋₆ alkynyl, C₃₋₁₀ carbocyclyl, or C₂₋₉ heterocyclyl, R₅ and R₆ are each independently hydrogen, C₁₋₆ alkyl, C₂₋₆ alkenyl, C₂₋₆ alkynyl, C₃₋₁₀ carbocyclyl, or C₂₋₉ heterocyclyl, or the -N(R₅)(R₆) is C₂₋₉ heterocyclyl.

According to another embodiment of the present invention, the compound represented by Chemical Formula 1 can be represented as Chemical Formula 2 below: 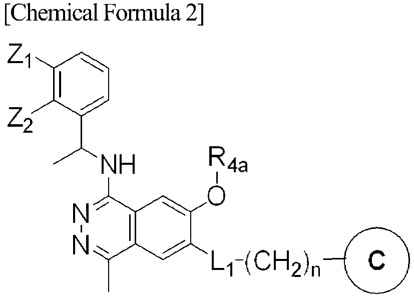 In Chemical Formula 2,
L₁ is a direct bond, -C(O)-, -O-, -NH-, -C(O)NH-, or -C(O)NCH₃-;
n is an integer in the range of 0 to 2; and
Z₁ and Z₂ are each independently hydrogen, -CN, -F, -CF₂H, -CF₃, -CH₃, or -NH₂, but for the case in which Z₁ and Z₂ are both hydrogen;
R₄ₐ is hydrogen, C₁₋₆ alkyl, C₃₋₁₀ carbocyclyl, or C₂₋₉ heterocyclyl;
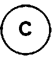 is morpholinyl, thiomorpholinyl, dioxothiomorpholinyl, piperazinyl, thiazolidinyl, tetrahydropyranyl, hexahydro-1*H*-furo[3,4-c]pyrrolyl, oxetanyl or azetidinyl, wherein the morpholinyl, thiomorpholinyl, dioxothiomorpholinyl, piperazinyl, thiazolidinyl, tetrahydropyranyl, hexahydro-1*H*-furo[3,4-c]pyrrolyl, oxetanyl or azetidinyl is unsubstituted or substituted with one or more functional groups selected from the group consisting of halogen, -C₁₋₃ alkyl, C₃₋₁₀ carbocyclyl, and C₂₋₉ heterocycly.

According to a further embodiment of the present invention, the compound represented by Chemical Formula 1 can be represented as Chemical Formula 3 below: 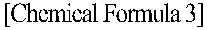 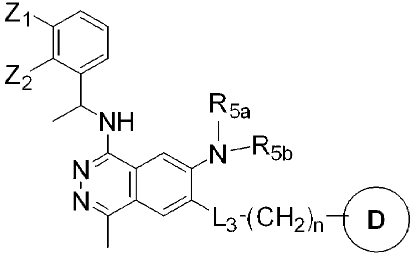 In Chemical Formula 3,
L₃ is a direct bond, -C(O)-, -C(O)NH-, or -C(O)NCH₃-;
n is an integer in the range of 0 to 2; and
Z₁ and Z₂ are each independently hydrogen, -CN, -F, -CF₂H, -CF₃, -CH₃, or -NH₂, but for the case in which Z₁ and Z₂ are both hydrogen;
R₅ₐ and R_{5b} are each independently hydrogen, C₁₋₆ alkyl, C₃₋₁₀ carbocyclyl, or C₂₋₉ heterocyclyl, or
alternatively the -N(R₅ₐ)(R_{5b}) is a C₂₋₉ heterocyclyl in which R₅ₐ and R_{5b} are linked to each other and form a ring in conjunction with a nitrogen atom contained in the -N(R₅ₐ)(R_{5b});
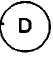 is morpholinyl, dioxothiomorpholinyl, thiomorpholinyl, piperazinyl, thiazolidinyl, tetrahydropyranyl, hexahydro-1*H*-furo[3,4-c]pyrrolyl, oxetanyl or azetidinyl, in which morpholinyl, dioxothiomorpholinyl, thiomorpholinyl, piperazinyl, thiazolidinyl, tetrahydropyranyl, hexahydro-1*H*-furo[3,4-c]pyrrolyl, oxetanyl or azetidinyl is unsubstituted or substituted with one or more functional groups selected from the group consisting of halogen, -C₁₋₃ alkyl, C₃₋₁₀ carbocyclyl, and C₂₋₉ heterocyclyl.

According to a yet further embodiment of the present invention, the compound represented by Chemical Formula 2 can be represented as Chemical Formula 4 below: 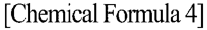 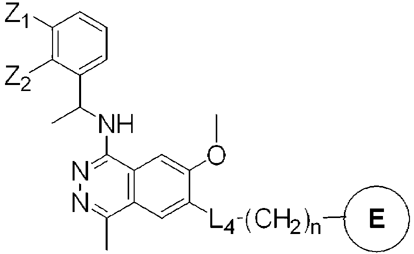 In Chemical Formula 4,
L₄ is a direct bond, -C(O)-, -O-, -C(O)NH-, or -C(O)NCH₃-;
n is an integer in the range of 0 to 2; and
Z₁ and Z₂ are each independently hydrogen, -CN, -F, -CF₂H, -CF₃, -CH₃, or -NH₂, but for the case in which Z₁ and Z₂ are both hydrogen;
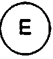 is morpholinyl, thiomorpholinyl, piperazinyl, thiazolidinyl, or tetrahydropyranyl, wherein the morpholinyl, thiomorpholinyl, piperazinyl, thiazolidinyl, or tetrahydropyranyl is unsubstituted or substituted with one or more functional groups selected from the group consisting of halogen, -C₁₋₃ alkyl, C₃₋₁₀ carbocyclyl, and C₂₋₉ heterocyclyl.

According to a still yet further embodiment of the present invention, the compound represented by Chemical Formula 3 can be represented as Chemical Formula 5 below: 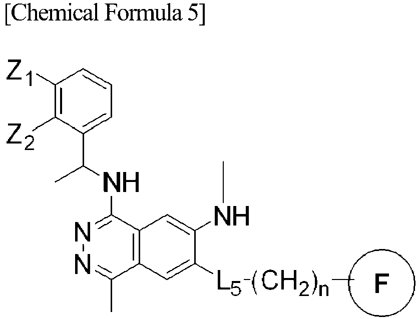 In Chemical Formula 5,
L₅ is a direct bond, -C(O)-, -C(O)NH-, or -C(O)NCH₃-;
n is an integer in the range of 0 to 2; and
Z₁ and Z₂ are each independently hydrogen, -F, -CN, -CF₂H, -CF₃, -CH₃, or -NH₂, but for the case in which Z₁ and Z₂ are both hydrogen;
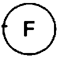 is morpholinyl, dioxothiomorpholinyl, thiomorpholinyl, piperazinyl, thiazolidinyl, or hexahydro-1*H*-furo[3,4-c]pyrrolyl, wherein the morpholinyl, dioxothiomorpholinyl, thiomorpholinyl, piperazinyl, thiazolidinyl, or hexahydro-1*H*-furo[3,4-c]pyrrolyl is unsubstituted or substituted with one or more functional groups selected from the group consisting of halogen, -C₁₋₃ alkyl, C₃₋₁₀ carbocyclyl, and C₂₋₉ heterocyclyl..

In addition, preferred examples of the compound of Chemical Formula 1 according to the present invention are as follows, but not limited thereto:
(*R*)-2-methyl-3-(1-((4-methyl-7-(methylamino)-6-(morpholine-4-carbonyl)phthalazin-1-yl)amino)ethyl)benzonitrile;
(*R*)-2-methyl-3-(1-((4-methyl-7-(methylamino)-6-(thiomorpholine-4-carbonyl)phthalazin-1-yl)amino)ethyl)benzonitrile;
(*R*)-2-methyl-3-(1-((4-methyl-7-(methylamino)-6-(thiazolidine-3-carbonyl)phthalazin-1-yl)amino)ethyl)benzonitrile;
(*R*)-3-(1-((6-(1,1-dioxothiomorpholine-4-carbonyl)-4-methyl-7-(methylamino)phthalazin-1-yl)amino)ethyl)-2-methylbenzonitrile;
(*R*)-1-((1-(3-cyano-2-methylphenyl)ethyl)amino)-4-methyl-7-(methylamino)-N-(tetrahydro-2H-pyran-4-yl)phthalazin-6-carboxamide;
(*R*)-1-((1-(3-cyano-2-methylphenyl)ethyl)amino)-N,4-dimethyl-7-(methylamino)-N-(tetrahydro-2H-pyran-4-yl)phthalazin-6-carboxamide;
3-((1R)-1-((6-(hexahydro-1H-furo[3,4-c]pyrrole-5-carbonyl)-4-methyl-7-(methylamino)phthalazin-1-yl)amino)ethyl)-2-methylbenzonitrile;
(*R*)-2-methyl-3-(1-((4-methyl-7-(methylamino)-6-(4-methylpiperazin-1-carbonyl)phthalazin-1-yl)amino)ethyl)benzonitrile;
(*R*)-2-methyl-3-(1-((4-methyl-7-(methylamino)-6-(piperazin-1-carbonyl)phthalazin-1-yl)amino)ethyl)benzonitrile;
(*R*)-3-(1-((6-(4,4-difluoropiperidin-1-carbonyl)-4-methyl-7-(methylamino)phthalazin-1-yl)amino)ethyl)-2-methylbenzonitrile;
(*R*)-2-methyl-3-(1-((4-methyl-7-(methylamino)-6-(2-oxa-6-azaspiro[3.3]heptane-6-carbonyl)phthalazin-1-yl)amino)ethyl)benzonitrile;
(*R*)-3-(1-((6-(4-ethylpiperazin-1-carbonyl)-4-methyl-7-(methylamino)phthalazin-1-yl)amino)ethyl)-2-methylbenzonitrile;
(*R*)-3-(1-((6-(4-cyclopropylpiperazin-1-carbonyl)-4-methyl-7-(methylamino)phthalazin-1-yl)amino)ethyl)-2-methylbenzonitrile;
(*R*)-2-methyl-3-(1-((4-methyl-7-(methylamino)-6-(pyrrolidin-1-carbonyl)phthalazin-1-yl)amino)ethyl)benzonitrile;
(*R*)-2-methyl-3-(1-((4-methyl-7-(methylamino)-6-(piperidin-1-carbonyl)phthalazin-1-yl)amino)ethyl)benzonitrile;
(*R*)-3-(1-((6-(azetidin-1-carbonyl)-4-methyl-7-(methylamino)phthalazin-1-yl)amino)ethyl)-2-methylbenzonitrile;
(*R*)-3-(1-((6-(3-fluoroazetidin-1-carbonyl)-4-methyl-7-(methylamino)phthalazin-1-yl)amino)ethyl)-2-methylbenzonitrile;
(*R*)-2-methyl-3-(1-((4-methyl-7-(methylamino)-6-(5-azaspiro[2.4]heptane-5-carbonyl)phthalazin-1-yl)amino)ethyl)benzonitrile;
3-((1R)-1-((6-(2-oxa-5-azabicyclo[2.2.1]heptane-5-carbonyl)-4-methyl-7-(methylamino)phthalazin-1-yl)amino)ethyl)-2-methylbenzonitrile;
(*R*)-3-(1-((6-(4-cyclohexylpiperazin-1-carbonyl)-4-methyl-7-(methylamino)phthalazin-1-yl)amino)ethyl)-2-methylbenzonitrile;
(*R*)-3-(1-((6-(4-isopropylpiperazin-1-carbonyl)-4-methyl-7-(methylamino)phthalazin-1-yl)amino)ethyl)-2-methylbenzonitrile;
(*R*)-2-methyl-3-(1-((4-methyl-7-(methylamino)-6-(4-morpholinopiperidin-1-carbonyl)phthalazin-1-yl)amino)ethyl)benzonitrile;
(*R*)-1-((1-(3-cyano-2-methylphenyl)ethyl)amino)-4-methyl-7-(methylamino)-N-(2-morpholinoethyl)phthalazin-6-carboxamide;
(*R*)-2-methyl-3-(1-((4-methyl-7-(methylamino)-6-(1,4-oxazepane-4-carbonyl)phthalazin-1-yl)amino)ethyl)benzonitrile;
(*R*)-(1-((1-(3-amino-5-(trifluoromethyl)phenyl)ethyl)amino)-4-methyl-7-(methylamino)phthalazin-6-yl)(morpholino)methanone;
(*R*)-(1-((1-(5-amino-3-(difluoromethyl)-2-fluorophenyl)ethyl)amino)-4-methyl-7-(methylamino)phthalazin-6-yl)(morpholino)methanone;
(*R*)-(1-((1-(3-(1,1-difluoro-2-hydroxyethyl)phenyl)ethyl)amino)-4-methyl-7-(methylamino)phthalazin-6-yl)(morpholino)methanone;
(*R*)-3-(1-((7-methoxy-4-methyl-6-(morpholinomethyl)phthalazin-1-yl)amino)ethyl)-2-methylbenzonitrile;
(*R*)-2-methyl-3-(1-((4-methyl-7-(methylamino)-6-(morpholinomethyl)phthalazin-1-yl)amino)ethyl)benzonitrile;
(*R*)-3-(1-((7-(cyclopentylamino)-4-methyl-6-(morpholinomethyl)phthalazin-1-yl)amino)ethyl)-2-methylbenzonitrile;
(*R*)-3-(1-((7-(cyclopentylamino)-6-((4-cyclopropylpiperazin-1-yl)methyl)-4-methylphthalazin-1-yl)amino)ethyl)-2-methylbenzonitrile;
(*R*)-3-(1-((7-methoxy-4-methyl-6-(morpholine-4-carbonyl)phthalazin-1-yl)amino)ethyl)-2-methylbenzonitrile;
(*R*)-3-(1-((7-(cyclopentylamino)-4-methyl-6-(morpholine-4-carbonyl)phthalazin-1-yl)amino)ethyl)-2-methylbenzonitrile.

In the present invention, a method for preparing the compound of Chemical Formula 1 is not particularly limited. For example, the compound of Chemical Formula 1 may be synthesized by the preparation method of Reaction Formula 1, Reaction Formula 2, Reaction Formula 3, Formula 4, Reaction Formula 5, Reaction Formula 6, or Reaction Formula 7 below:

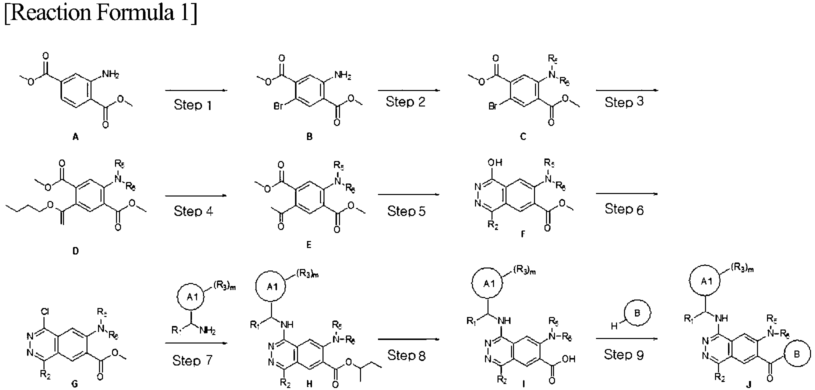

In Reaction Formula 1, 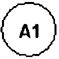 , R₁, R₂, R₃, R₅, R₆, m, and 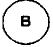 are the same as defined in Chemical Formula 1 but are not limited thereto.

Dimethyl aminoterephthalate (1.0 equivalent) was slowly added dropwise to chloroform at 20 to 25°C and refluxed for 4 to 6 minutes. N-bromosuccinimide (1.1 equivalents) was slowly added dropwise to the reaction mixture at 20 to 25°C. After completion of the addition, the mixture was refluxed at 20 to 25°C for 18 to 24 hours. Upon completion of the reaction, water was slowly added dropwise to the reaction mixture. The mixture was extracted with dichloromethane, dried over anhydrous sodium sulfate, and concentrated under reduced pressure. The resulting solution was concentrated under reduced pressure to obtain the title compound.

### [Step-2]

B (1.0 equivalent) prepared in [Step-1], methyl iodide (2.0 equivalents), and potassium carbonate (4.0 equivalents) were dissolved in DMF, and the mixture was refluxed with stirring at 100 to 120°C for 15 to 18 hours. Upon completion of the reaction, the solution was cooled to room temperature, and water was slowly added dropwise to the reaction mixture. The mixture was extracted with ethyl acetate and dried over anhydrous sodium sulfate. The dried organic layer was filtered and concentrated under reduced pressure. The resulting residue was purified by MPLC to obtain the title compound.

### [Step-3]

C (1.0 equivalent) obtained in [Step-2], butyl vinyl ether (5.0 equivalents), palladium acetate (0.1 equivalent), triphenylphosphine (0.2 equivalent), and triethylamine (1.2 equivalents) were dissolved in acetonitrile, and the mixture was refluxed with stirring at 90 to 110°C for 15 to 20 hours. Upon completion of the reaction, the reaction solution was cooled to room temperature, filtered through a Celite-packed filter, and washed with dichloromethane. The filtered organic layer was concentrated under reduced pressure to obtain the title compound.

### [Step-4]

D (1.0 equivalent) obtained in [Step-3] was dissolved in tetrahydrofuran, and 6 N hydrochloric acid was slowly added dropwise at 20 to 25°C. After completion of the addition, the mixture was refluxed at 20 to 25°C for 0.5 to 2 hours. Upon completion of the reaction, the mixture was concentrated under reduced pressure. Water was slowly added dropwise to the residue, and the mixture was extracted with ethyl acetate. The obtained organic layer was washed with a saturated aqueous sodium bicarbonate solution, dried over anhydrous sodium sulfate, filtered, and concentrated under reduced pressure. The resulting residue was purified by column chromatography to obtain the title compound.

### [Step-5]

E (1.0 equivalent) obtained in [Step-4] and hydrazine hydrate (1.0 equivalent) were dissolved in ethanol, and the mixture was refluxed with stirring at 85 to 105°C for 0.5 to 2 hours. Upon completion of the reaction, the reaction solution was cooled to room temperature, filtered, and washed with ethanol to obtain the title compound.

### [Step-6]

F (1.0 equivalent) prepared in [Step-5] was dissolved in phosphoryl chloride, and the mixture was refluxed at 120 to 140°C for 3 to 4 hours. Upon completion of the reaction, the mixture was cooled to room temperature and concentrated. The residue thus obtained was neutralized by dropwise addition of an aqueous sodium bicarbonate solution. The mixture was extracted with dichloromethane, dried over anhydrous sodium sulfate, and concentrated under reduced pressure. The resulting residue was purified by MPLC to obtain the title compound.

### [Step-7]

G (1.0 equivalent) prepared in [Step-6], aniline (1.3 equivalents), magnesium chloride (3.0 equivalents), and DIPEA (6.0 equivalents) were dissolved in 2-butanol, and the mixture was refluxed with stirring at 110 to 120°C for 47 to 50 hours. Upon completion of the reaction, the mixture was cooled to room temperature, water was added, and the mixture was extracted with ethyl acetate, dried over anhydrous sodium sulfate, and concentrated under reduced pressure. The residue was purified by column chromatography to obtain the title compound.

### [Step-8]

H (1.0 equivalent) obtained in [Step-7] and sodium hydroxide (10.0 equivalents) were dissolved in a mixture of tetrahydrofuran:methanol:water, and the mixture was refluxed at 20 to 30°C for 2 to 4 hours. Upon completion of the reaction, the mixture was concentrated under reduced pressure, and the resulting residue was acidified at low temperature with a 6 N hydrochloric acid solution until pH 2. The obtained solid was collected by filtration under reduced pressure and washed with water. The filtered solid was dried to obtain the title compound.

### [Step-9]

I (1.0 equivalent) prepared in [Step-8], an amine (8.0 equivalents), HATU (3.0 equivalents), and DIPEA (8.0 equivalents) were dissolved in DMF, and the mixture was stirred at room temperature for 3 to 5 hours. Upon completion of the reaction, water was added, and the mixture was extracted with ethyl acetate, dried over anhydrous sodium sulfate, and concentrated under reduced pressure. The resulting residue was purified by column chromatography to obtain the title compound.

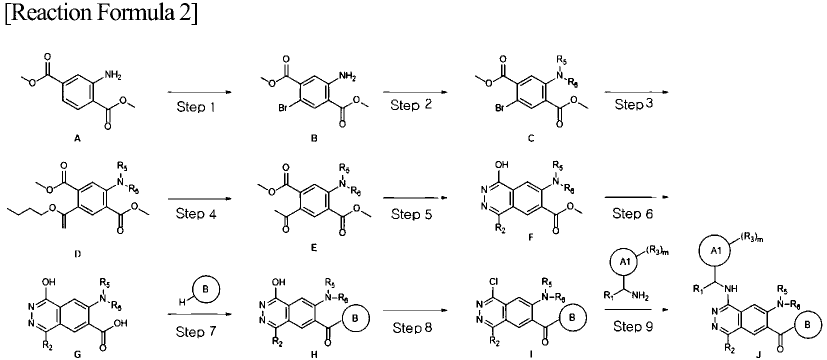

In Reaction Formula 2, 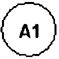 R₁, R₂, R₃, R5, R₆, m, and 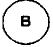 are the same as defined in Chemical Formula 1 but are not limited thereto.

### [Step-1]

Dimethyl aminoterephthalate (1.0 equivalent) was slowly added dropwise to chloroform at 20 to 25°C and refluxed for 4 to 6 minutes. N-bromosuccinimide (1.1 equivalents) was slowly added dropwise to the reaction mixture at 20 to 25°C. After completion of the addition, the mixture was refluxed at 20 to 25°C for 18 to 24 hours. Upon completion of the reaction, water was slowly added dropwise to the reaction mixture. The mixture was extracted with dichloromethane, dried over anhydrous sodium sulfate, and concentrated under reduced pressure to obtain the title compound.

### [Step-2]

B (1.0 equivalent) prepared in [Step-1], methyl iodide (2.0 equivalents), and potassium carbonate (4.0 equivalents) were dissolved in DMF, and the mixture was refluxed with stirring at 100 to 120°C for 15 to 18 hours. Upon completion of the reaction, the solution was cooled to room temperature, and water was slowly added dropwise to the reaction mixture. The mixture was extracted with ethyl acetate and dried over anhydrous sodium sulfate. The dried organic layer was concentrated under reduced pressure. The resulting residue was purified by MPLC to obtain the title compound.

### [Step-3]

C (1.0 equivalent) obtained in [Step-2], butyl vinyl ether (5.0 equivalents), palladium acetate (0.1 equivalent), triphenylphosphine (0.2 equivalent), and triethylamine (1.2 equivalents) were dissolved in acetonitrile, and the mixture was refluxed with stirring at 90 to 110°C for 15 to 20 hours. Upon completion of the reaction, the reaction solution was cooled to room temperature, filtered through a Celite-packed filter, and washed with dichloromethane. The filtered organic layer was concentrated under reduced pressure to obtain the title compound.

### [Step-4]

D (1.0 equivalent) obtained in [Step-3] was dissolved in tetrahydrofuran, and 6 N hydrochloric acid was slowly added dropwise at 20 to 25°C. After completion of the addition, the mixture was refluxed with stirring at 20 to 25°C for 0.5 to 2 hours. Upon completion of the reaction, the mixture was concentrated under reduced pressure. Water was slowly added dropwise to the residue, and the mixture was extracted with ethyl acetate. The obtained organic layer was washed with a saturated aqueous sodium bicarbonate solution, dried over anhydrous sodium sulfate, and concentrated under reduced pressure. The resulting residue was purified by MPLC to obtain the title compound.

### [Step-5]

E (1.0 equivalent) obtained in [Step-4] and hydrazine hydrate (1.0 equivalent) were dissolved in ethanol, and the mixture was refluxed with stirring at 85 to 105°C for 0.5 to 2 hours. Upon completion of the reaction, the reaction solution was cooled to room temperature, filtered, and washed with ethanol to obtain the title compound.

### [Step-6]

F (1.0 equivalent) prepared in [Step-5] and sodium hydroxide (10.0 equivalents) were dissolved in a mixture of tetrahydrofuran:methanol:water, and the mixture was refluxed at 20 to 30°C for 1.5 to 3.5 hours. Upon completion of the reaction, the mixture was concentrated under reduced pressure, and the resulting residue was acidified at low temperature with a 6 N hydrochloric acid solution until pH 2. The obtained solid was collected by filtration under reduced pressure and washed with water. The filtered solid was dried to obtain the title compound.

### [Step-7]

G (1.0 equivalent) prepared in [Step-6], an amine (2.0 equivalents), HATU (1.3 equivalents), and DIPEA (5.0 equivalents) were dissolved in DMF, and the mixture was stirred at room temperature for 2 to 3 hours. Upon completion of the reaction, water was added, and the mixture was extracted with ethyl acetate, dried over anhydrous sodium sulfate, and concentrated under reduced pressure. The residue was purified by column chromatography to obtain the title compound.

### [Step-8]

H (1.0 equivalent) obtained in [Step-7] was dissolved in phosphoryl chloride, and the mixture was refluxed at 105 to 115°C for 0.5 to 2 hours. Upon completion of the reaction, the mixture was cooled to room temperature and concentrated. The residue thus obtained was neutralized by dropwise addition of an aqueous sodium bicarbonate solution. The mixture was extracted with dichloromethane, dried over anhydrous sodium sulfate, and concentrated under reduced pressure. The resulting residue was purified by MPLC to obtain the title compound.

### [Step-9]

I (1.0 equivalent) prepared in [Step-8], aniline (2.4 equivalents), cesium fluoride (3.0 equivalents), and DIPEA (5.0 equivalents) were dissolved in DMSO, and the mixture was refluxed with stirring at 120 to 140°C for 15 to 17 hours. Upon completion of the reaction, the mixture was cooled to room temperature, water was added, and the mixture was extracted with ethyl acetate, dried over anhydrous sodium sulfate, and concentrated under reduced pressure. The resulting residue was purified by column chromatography to obtain the title compound.

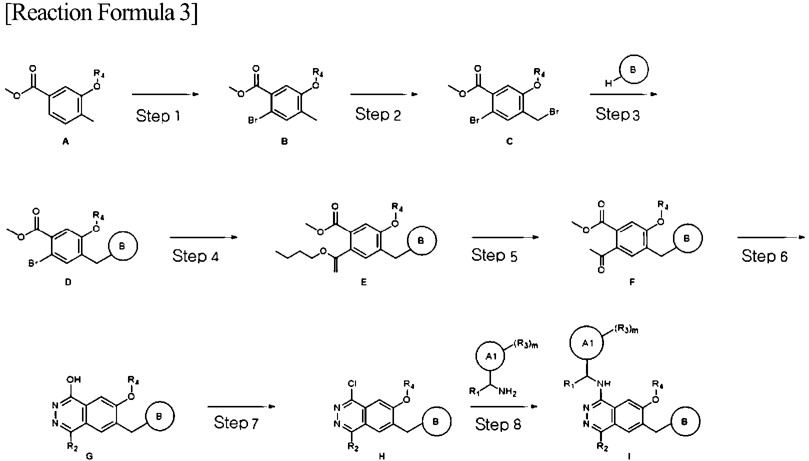

In Reaction Formula 3, 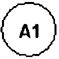 R₁, R₂, R₃, R₄, m, and 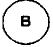 are the same as defined in Chemical Formula 1 but are not limited thereto.

### [Step-1]

Methyl 3-methoxy-4-methylbenzoate (1.0 equivalent) was mixed with acetic acid and water, and bromine (1.0 equivalent) was added dropwise thereto. After completion of the addition, the mixture was refluxed with stirring at 50 to 60°C for 1 to 2 hours. Upon completion of the reaction, the mixture was cooled to room temperature to obtain a solid product. The obtained solid was collected by filtration under reduced pressure and washed with water. The filtered solid was dried to obtain the title compound.

### [Step-2]

B (1.0 equivalent) obtained in [Step-1], N-bromosuccinimide (1.2 equivalents), and azobisisobutyronitrile (AIBN) (1.0 equivalent) were dissolved in chloroform, and the mixture was refluxed with stirring at 65 to 70°C for 2 to 3 hours. Upon completion of the reaction, the mixture was cooled to room temperature, and a saturated aqueous sodium bicarbonate solution was added. The mixture was extracted with dichloromethane, and the obtained organic layer was dried over anhydrous sodium sulfate. The dried organic layer was filtered and concentrated under reduced pressure. The resulting residue was purified by column chromatography to obtain the title compound.

### [Step-3]

C (1.0 equivalent) obtained in [Step-2], an amine (1.1 equivalents), and potassium carbonate (2.0 equivalents) were dissolved in acetonitrile, and the mixture was stirred at 20 to 30°C for 17 to 20 hours. Upon completion of the reaction, the mixture was cooled to room temperature, and a saturated aqueous sodium bicarbonate solution was added. The mixture was extracted with ethyl acetate, and the obtained organic layer was dried over anhydrous sodium sulfate. The dried organic layer was filtered and concentrated under reduced pressure. The resulting residue was purified by column chromatography to obtain the title compound.

### [Step-4]

D (1.0 equivalent) obtained in [Step-3], butyl vinyl ether (5.0 equivalents), palladium acetate (0.1 equivalent), triphenylphosphine (0.2 equivalent), and triethylamine (1.2 equivalents) were dissolved in acetonitrile, and the mixture was refluxed with stirring at 90 to 110°C for 15 to 20 hours. Upon completion of the reaction, the reaction solution was cooled to room temperature, filtered through a Celite-packed filter, and washed with dichloromethane. The filtered organic layer was concentrated under reduced pressure, and the next reaction was performed without further purification.

### [Step-5]

E (1.0 equivalent) obtained in [Step-4] was dissolved in tetrahydrofuran, and 6 N hydrochloric acid was slowly added dropwise at 25 to 30°C. After completion of the addition, the mixture was refluxed at 25 to 30°C for 0.5 to 2 hours. Upon completion of the reaction, the mixture was concentrated under reduced pressure. Water was slowly added dropwise to the residue, and the mixture was extracted with ethyl acetate. The obtained organic layer was washed with a saturated aqueous sodium bicarbonate solution, dried over anhydrous sodium sulfate, and concentrated under reduced pressure. The resulting residue was purified by column chromatography to obtain the title compound.

### [Step-6]

F (1.0 equivalent) obtained in [Step-5] and hydrazine monohydrate (1.5 equivalents) were dissolved in ethanol, and the mixture was refluxed with stirring at 85 to 105°C for 0.5 to 2 hours. Upon completion of the reaction, the reaction solution was cooled to room temperature, filtered, and washed with ethanol. The filtered solid was dried to obtain the title compound.

### [Step-7]

G (1.0 equivalent) obtained in [Step-6] and phosphoryl chloride (3.0 equivalents) were dissolved in acetonitrile, and the mixture was refluxed with stirring at 95 to 115°C for 0.5 to 2 hours. Upon completion of the reaction, the mixture was cooled to room temperature and concentrated. The residue thus obtained was dissolved in dichloromethane, and neutralized at -5 to 5°C by dropwise addition to a saturated aqueous sodium bicarbonate solution. The mixture was extracted with dichloromethane, dried over anhydrous sodium sulfate, and concentrated under reduced pressure. The residue was purified by column chromatography to obtain the title compound.

### [Step-8]

H (1.0 equivalent) prepared in [Step-7], aniline (1.5 equivalents), magnesium chloride (3.0 equivalents), and N,N-diisopropylethylamine (DIPEA) (6.0 equivalents) were dissolved in 2-butanol, and the mixture was refluxed with stirring at 120 to 140°C for 46 to 50 hours. Upon completion of the reaction, the mixture was cooled to room temperature, water was added, and the mixture was extracted with ethyl acetate, dried over anhydrous sodium sulfate, and concentrated under reduced pressure. The resulting residue was purified by column chromatography to obtain the title compound.

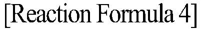

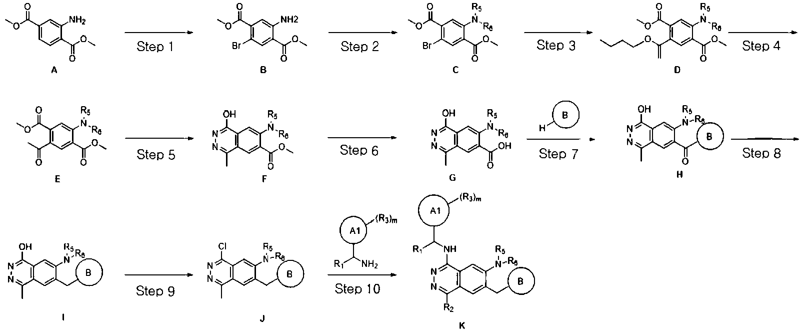

In Reaction Formula 4, 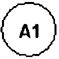 R₁, R₂, R₃, R₅, R₆, m, and 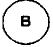 are the same as defined in Chemical Formula 1 but are not limited thereto.

### [Step-1]

Dimethyl 2-aminoterephthalate (1.0 equivalent) and N-bromosuccinimide (1.1 equivalents) were slowly added dropwise to chloroform at 20 to 30°C. After completion of the addition, the mixture was refluxed with stirring at 20 to 30°C for 15 to 18 hours. Upon completion of the reaction, water was slowly added dropwise, and the mixture was extracted with ethyl acetate. The obtained organic layer was dried over anhydrous sodium sulfate and concentrated under reduced pressure. The resulting residue was purified by column chromatography to obtain the title compound.

### [Step-2]

B (1.0 equivalent) prepared in [Step-1], methyl iodide (2.0 equivalents), and potassium carbonate (1.0 equivalent) were dissolved in DMF, and the mixture was refluxed with stirring at 100 to 120°C for 2.5 to 4.5 hours. Upon completion of the reaction, the mixture was cooled to room temperature, and water was slowly added dropwise. The mixture was extracted with ethyl acetate, and the obtained organic layer was dried over anhydrous sodium sulfate and concentrated under reduced pressure. The resulting residue was purified by column chromatography to obtain the title compound.

### [Step-3]

C (1.0 equivalent) obtained in [Step-2], butyl vinyl ether (5.0 equivalents), palladium acetate (0.1 equivalent), triphenylphosphine (0.2 equivalent), and triethylamine (1.2 equivalents) were dissolved in acetonitrile, and the mixture was refluxed with stirring at 90 to 110°C for 15 to 20 hours. Upon completion of the reaction, the reaction solution was cooled to room temperature, filtered through a Celite-packed filter, and washed with dichloromethane. The filtered organic layer was concentrated under reduced pressure to obtain the title compound.

### [Step-4]

D (1.0 equivalent) obtained in [Step-3] was dissolved in tetrahydrofuran, and 6 N hydrochloric acid was slowly added dropwise at 20 to 25°C. After completion of the addition, the mixture was refluxed with stirring at 20 to 25°C for 0.5 to 2 hours. Upon completion of the reaction, the mixture was concentrated under reduced pressure. Water was slowly added dropwise to the residue, and the mixture was extracted with ethyl acetate. The obtained organic layer was washed with a saturated aqueous sodium bicarbonate solution, dried over anhydrous sodium sulfate, and concentrated under reduced pressure. The resulting residue was purified by column chromatography to obtain the title compound.

### [Step-5]

E (1.0 equivalent) obtained in [Step-4] and hydrazine hydrate (1.3 equivalents) were dissolved in ethanol, and the mixture was refluxed with stirring at 85 to 105°C for 0.5 to 2 hours. Upon completion of the reaction, the reaction solution was cooled to room temperature, filtered, and washed with ethanol to obtain the title compound.

### [Step-6]

F (1.0 equivalent) prepared in [Step-5] and sodium hydroxide (12.0 equivalents) were dissolved in a mixture of tetrahydrofuran:methanol:water, and the mixture was refluxed with stirring at 20 to 30°C for 1.5 to 3.5 hours. Upon completion of the reaction, the mixture was concentrated under reduced pressure, and the resulting residue was acidified at low temperature with a 6 N hydrochloric acid solution until pH 2. The obtained solid was collected by filtration under reduced pressure and washed with water. The filtered solid was dried to obtain the title compound.

### [Step-7]

G (1.0 equivalent) prepared in [Step-6], an amine (8.0 equivalents), HATU (3.0 equivalents), and N,N-diisopropylethylamine (DIPEA) (8.0 equivalents) were dissolved in DMF, and the mixture was stirred at room temperature for 15 to 18 hours. Upon completion of the reaction, water was added, and the mixture was extracted with ethyl acetate, dried over anhydrous sodium sulfate, and concentrated under reduced pressure. The resulting residue was purified by column chromatography to obtain the title compound.

### [Step-8]

H (1.0 equivalent) obtained in [Step-7] was dissolved in tetrahydrofuran, and borane dimethyl sulfide was slowly added dropwise thereto. After completion of the addition, the mixture was refluxed with stirring at 20 to 30°C for 15 to 17 hours. Upon completion of the reaction, water was added, and the mixture was extracted with a mixed solvent of dichloromethane and methanol. The obtained organic layer was dried over anhydrous sodium sulfate and concentrated under reduced pressure. The resulting residue was purified by column chromatography to obtain the title compound.

### [Step-9]

I (1.0 equivalent) obtained in [Step-8] was dissolved in phosphoryl chloride, and the mixture was refluxed with stirring at 105 to 115°C for 1 to 3 hours. Upon completion of the reaction, the mixture was cooled to room temperature and concentrated. The residue thus obtained was neutralized by dropwise addition of a saturated aqueous sodium bicarbonate solution. The mixture was extracted with a mixed solvent of dichloromethane and acetone, dried over anhydrous sodium sulfate, and concentrated under reduced pressure. The resulting residue was purified by column chromatography to obtain the title compound.

### [Step-10]

J (1.0 equivalent) prepared in [Step-9], aniline (1.5 equivalents), magnesium chloride (3.0 equivalents), and N,N-diisopropylethylamine (DIPEA) (6.0 equivalents) were dissolved in 2-butanol, and the mixture was refluxed with stirring at 120 to 140°C for 45 to 50 hours. Upon completion of the reaction, the mixture was cooled to room temperature, water was added, and the mixture was extracted with ethyl acetate, dried over anhydrous sodium sulfate, and concentrated under reduced pressure. The resulting residue was purified by column chromatography to obtain the title compound.

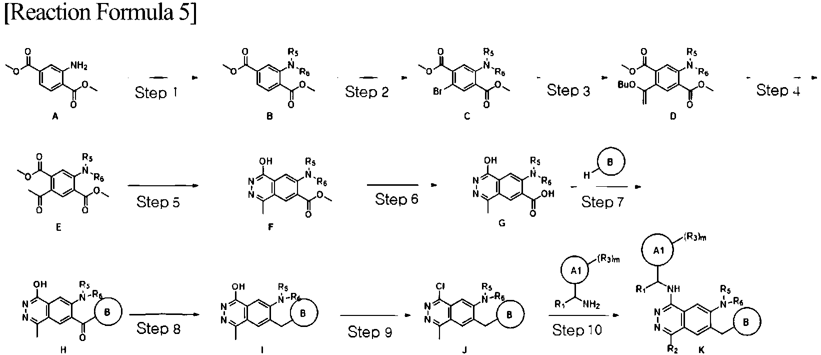

In Reaction Formula 5, 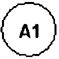 R₁, R₂, R₃, R₅, R₆, m, and 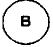 are the same as defined in Chemical Formula 1 but are not limited thereto.

### [Step-1]

Dimethyl 2-aminoterephthalate (1.0 equivalent), a ketone (2.2 equivalents), sodium triacetoxyborohydride (2.0 equivalents), and acetic acid (8.5 equivalents) were slowly added dropwise to dichloromethane at 25 to 30°C, and the mixture was refluxed with stirring for 45 to 50 hours. Upon completion of the reaction, water was slowly added dropwise to the reaction mixture. The mixture was extracted with dichloromethane. The obtained organic layer was washed with a saturated aqueous sodium bicarbonate solution, dried over anhydrous sodium sulfate, and concentrated under reduced pressure. The resulting residue was purified by column chromatography to obtain the title compound.

### [Step-2]

B (1.0 equivalent) prepared in [Step-1] and N-bromosuccinimide (1.05 equivalents) were slowly added dropwise to acetonitrile at 25 to 30°C, and the mixture was refluxed with stirring for 15 to 17 hours. Upon completion of the reaction, water was slowly added dropwise to the reaction mixture. The mixture was extracted with dichloromethane. The obtained organic layer was dried over anhydrous sodium sulfate and concentrated under reduced pressure. The resulting residue was purified by column chromatography to obtain the title compound.

### [Step-3]

C (1.0 equivalent) obtained in [Step-2], butyl vinyl ether (5.0 equivalents), palladium acetate (0.1 equivalent), triphenylphosphine (0.2 equivalent), and triethylamine (1.2 equivalents) were dissolved in acetonitrile, and the mixture was refluxed with stirring at 90 to 110°C for 15 to 20 hours. Upon completion of the reaction, the reaction solution was cooled to room temperature, filtered through a Celite-packed filter, and washed with dichloromethane. The filtered organic layer was concentrated under reduced pressure to obtain the title compound.

### [Step-4]

D (1.0 equivalent) obtained in [Step-3] was dissolved in tetrahydrofuran, and 6 N hydrochloric acid was slowly added dropwise thereto. After completion of the addition, the mixture was refluxed with stirring at 20 to 30°C for 1.5 to 3 hours. Upon completion of the reaction, the mixture was concentrated under reduced pressure, and the resulting residue was slowly added dropwise to water and extracted with ethyl acetate. The obtained organic layer was washed with a saturated aqueous sodium bicarbonate solution, dried over anhydrous sodium sulfate, and concentrated under reduced pressure. The resulting residue was purified by column chromatography to obtain the title compound.

### [Step-5]

D (1.0 equivalent) obtained in [Step-4] and hydrazine hydrate (1.3 equivalents) were dissolved in ethanol, and the mixture was refluxed with stirring at 85 to 105°C for 0.5 to 2 hours. Upon completion of the reaction, the reaction solution was cooled to room temperature, filtered, and washed with ethanol to obtain the title compound.

### [Step-6]

E (1.0 equivalent) prepared in [Step-5] and sodium hydroxide (12.0 equivalents) were dissolved in a mixture of tetrahydrofuran:methanol:water, and the mixture was refluxed with stirring at 20 to 30°C for 1.5 to 3.5 hours. Upon completion of the reaction, the mixture was concentrated under reduced pressure, and the resulting residue was acidified at low temperature with a 6 N hydrochloric acid solution until pH 2. The obtained solid was collected by filtration under reduced pressure and washed with water. The filtered solid was dried to obtain the title compound.

### [Step-7]

F (1.0 equivalent) prepared in [Step-6], an amine (8.0 equivalents), HATU (3.0 equivalents), and N,N-diisopropylethylamine (DIPEA) (8.0 equivalents) were dissolved in DMF, and the mixture was stirred at room temperature for 15 to 18 hours. Upon completion of the reaction, water was added, and the mixture was extracted with ethyl acetate, dried over anhydrous sodium sulfate, and concentrated under reduced pressure. The resulting residue was purified by column chromatography to obtain the title compound.

### [Step-8]

G (1.0 equivalent) obtained in [Step-7] was dissolved in tetrahydrofuran, and borane dimethyl sulfide was slowly added dropwise thereto. After completion of the addition, the mixture was refluxed with stirring at 20 to 30°C for 15 to 17 hours. Upon completion of the reaction, water was added, and the mixture was extracted with a mixed solvent of dichloromethane and methanol. The obtained organic layer was dried over anhydrous sodium sulfate and concentrated under reduced pressure. The resulting residue was purified by column chromatography to obtain the title compound.

### [Step-9]

H (1.0 equivalent) obtained in [Step-8] was dissolved in phosphoryl chloride, and the mixture was refluxed with stirring at 105 to 115°C for 1 to 3 hours. Upon completion of the reaction, the mixture was cooled to room temperature and concentrated. The residue thus obtained was neutralized by dropwise addition of a saturated aqueous sodium bicarbonate solution. The mixture was extracted with a mixed solvent of dichloromethane and acetone, dried over anhydrous sodium sulfate, and concentrated under reduced pressure. The resulting residue was purified by column chromatography to obtain the title compound.

### [Step-10]

I (1.0 equivalent) prepared in [Step-9], aniline (1.5 equivalents), magnesium chloride (3.0 equivalents), and N,N-diisopropylethylamine were dissolved in 2-butanol, and the mixture was refluxed with stirring at 120 to 140°C for 45 to 50 hours. Upon completion of the reaction, the mixture was cooled to room temperature, water was added, and the mixture was extracted with ethyl acetate, dried over anhydrous sodium sulfate, and concentrated under reduced pressure. The resulting residue was purified by column chromatography to obtain the title compound.

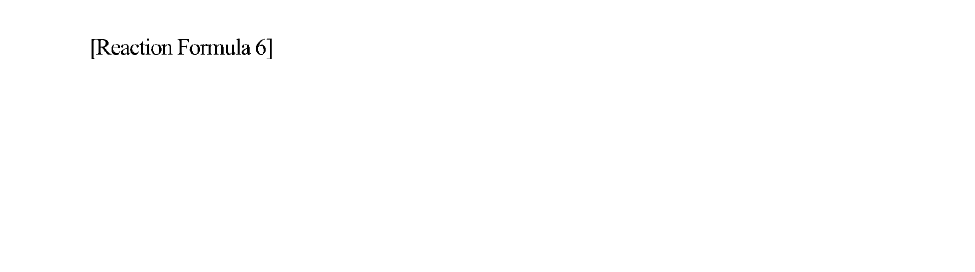

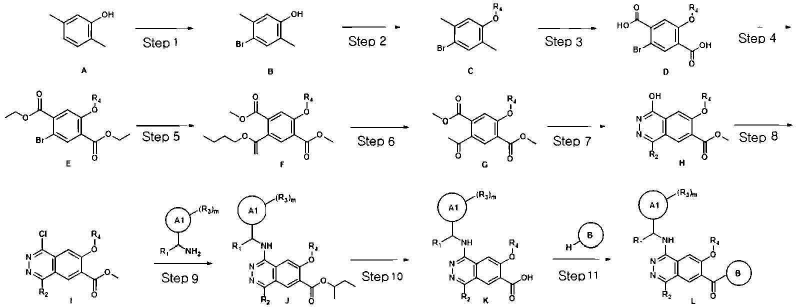

In Reaction Formula 6, 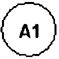 R₁, R₂, R₃, R₄, m, and 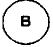 are the same as defined in Chemical Formula 1 but are not limited thereto.

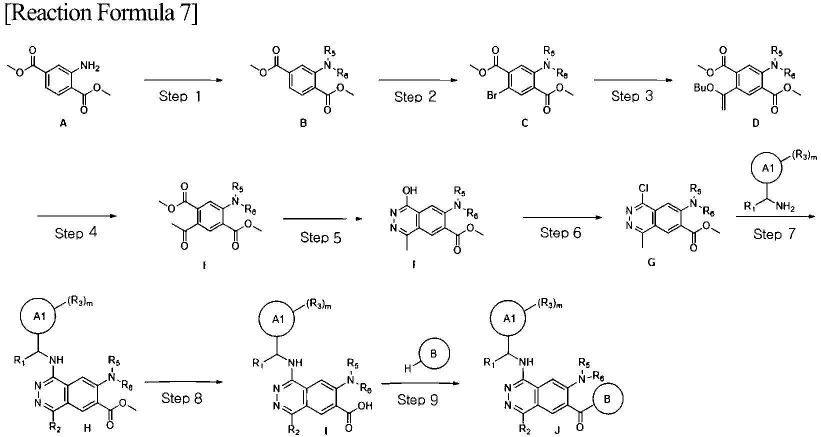

In Reaction Formula 7, 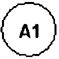 R₁, R₂, R₃, R₅, R₆, m, and 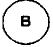 are the same as defined in Chemical Formula 1 but are not limited thereto.

### [Step-1]

Dimethyl 2-aminoterephthalate (1.0 equivalent), a ketone (2.2 equivalents), sodium triacetoxyborohydride (2.0 equivalents), and acetic acid (8.5 equivalents) were slowly added dropwise to dichloromethane at 25 to 30°C, and the mixture was refluxed with stirring for 45 to 50 hours. Upon completion of the reaction, water was slowly added dropwise to the reaction mixture. The mixture was extracted with dichloromethane. The obtained organic layer was washed with a saturated aqueous sodium bicarbonate solution, dried over anhydrous sodium sulfate, and concentrated under reduced pressure. The resulting residue was purified by column chromatography to obtain the title compound.

### [Step-2]

B (1.0 equivalent) prepared in [Step-1] and N-bromosuccinimide (1.05 equivalents) were slowly added dropwise to acetonitrile at 25 to 30°C, and the mixture was refluxed with stirring for 15 to 17 hours. Upon completion of the reaction, water was slowly added dropwise to the reaction mixture. The mixture was extracted with dichloromethane. The obtained organic layer was dried over anhydrous sodium sulfate and concentrated under reduced pressure. The resulting residue was purified by column chromatography to obtain the title compound.

### [Step-3]

C (1.0 equivalent) obtained in [Step-2], butyl vinyl ether (5.0 equivalents), palladium acetate (0.1 equivalent), triphenylphosphine (0.2 equivalent), and triethylamine (1.2 equivalents) were dissolved in acetonitrile, and the mixture was refluxed with stirring at 90 to 110°C for 15 to 20 hours. Upon completion of the reaction, the reaction solution was cooled to room temperature, filtered through a Celite-packed filter, and washed with dichloromethane. The filtered organic layer was concentrated under reduced pressure to obtain the title compound.

### [Step-4]

D (1.0 equivalent) obtained in [Step-3] was dissolved in tetrahydrofuran, and 6 N hydrochloric acid was slowly added dropwise thereto. After completion of the addition, the mixture was refluxed with stirring at 20 to 30°C for 1.5 to 3 hours. Upon completion of the reaction, the mixture was concentrated under reduced pressure, and the resulting residue was slowly added dropwise to water. The mixture was extracted with ethyl acetate. The obtained organic layer was washed with a saturated aqueous sodium bicarbonate solution, dried over anhydrous sodium sulfate, and concentrated under reduced pressure. The resulting residue was purified by column chromatography to obtain the title compound.

### [Step-5]

D (1.0 equivalent) obtained in [Step-4] and hydrazine hydrate (1.3 equivalents) were dissolved in ethanol, and the mixture was refluxed with stirring at 85 to 105°C for 0.5 to 2 hours. Upon completion of the reaction, the reaction solution was cooled to room temperature, filtered, and washed with ethanol to obtain the title compound.

### [Step-6]

F (1.0 equivalent) prepared in [Step-5] was dissolved in phosphoryl chloride, and the mixture was refluxed at 100 to 120°C for 3 to 4 hours. Upon completion of the reaction, the mixture was cooled to room temperature and concentrated. The residue thus obtained was neutralized by dropwise addition of an aqueous sodium bicarbonate solution. The mixture was extracted with dichloromethane, dried over anhydrous sodium sulfate, and concentrated under reduced pressure. The resulting residue was purified by MPLC to obtain the title compound.

### [Step-7]

G (1.0 equivalent) prepared in [Step-6], aniline (1.6 equivalents), magnesium chloride (3.0 equivalents), and DIPEA (6.0 equivalents) were dissolved in 2-butanol, and the mixture was refluxed with stirring at 110 to 130°C for 16 to 18 hours. Upon completion of the reaction, the mixture was cooled to room temperature, water was added, and the mixture was extracted with ethyl acetate, dried over anhydrous sodium sulfate, and concentrated under reduced pressure. The resulting residue was purified by column chromatography to obtain the title compound.

### [Step-8]

H (1.0 equivalent) obtained in [Step-7] and sodium hydroxide (10.0 equivalents) were dissolved in a mixture of tetrahydrofuran:methanol:water, and the mixture was refluxed at 20 to 30°C for 2 to 4 hours. Upon completion of the reaction, the mixture was concentrated under reduced pressure, and the resulting residue was acidified at low temperature with a 6 N hydrochloric acid solution until pH 2. The obtained solid was collected by filtration under reduced pressure and washed with water. The filtered solid was dried to obtain the title compound.

### [Step-9]

I (1.0 equivalent) prepared in [Step-8], an amine (1.5 equivalents), HATU (1.3 equivalents), and DIPEA (3.0 equivalents) were dissolved in DMF, and the mixture was stirred at room temperature for 3 to 5 hours. Upon completion of the reaction, water was added, and the mixture was extracted with ethyl acetate, dried over anhydrous sodium sulfate, and concentrated under reduced pressure. The resulting residue was purified by column chromatography to obtain the title compound.

Although the method for preparing the compound of Formula 1 has been described with reference to specific examples, specific reaction conditions, for example, reaction solvents, bases, and amounts of reactants, are not limited only to those described in the present specification, and should not be construed in any way as limiting the scope of the present invention.

The pharmaceutical composition of the present invention is also useful for preventing or treating various diseases associated therewith, by inhibiting SOS1 binding to RAS family proteins and/or RAC1 by the compound of Formula 1 contained therein.

According to another embodiment of the present invention, there is provided a pharmaceutical composition for preventing or treating a disease associated with inhibition of SOS1 binding to RAS family proteins and/or RAC1, comprising, as an active ingredient, a compound selected from the group consisting of the compound of Formula 1, optical isomers thereof, diastereomers thereof, hydrates thereof, solvates thereof, and pharmaceutically acceptable salts thereof.

According to another embodiment of the present invention, there is provided a pharmaceutical preparation comprising the pharmaceutical composition described above.

The pharmaceutical preparation of the present invention may be in various dosage forms for oral administration, such as tablets, pills, powders, capsules, syrups, or emulsions, or in dosage forms for parenteral administration, such as injections for intramuscular, intravenous, or subcutaneous administration.

When the pharmaceutical preparation of the present invention is in the form of an injection, examples of carriers therefor may include, but are not limited to, water, saline, aqueous glucose solution, aqueous sugar solution, alcohols, glycols, ethers, oils, fatty acids, fatty acid esters, and glycerides.

To use the compound according to the present invention as a medicament, it is formulated in the form of a pharmaceutical preparation, which contains suitable pharmaceutically acceptable organic or inorganic inert carrier materials in addition to the active ingredient for oral or parenteral administration, for example, water, gelatin, gum arabic, lactose, starch, vegetable oils, polyalkylene glycols, and the like. The pharmaceutical preparation may be present in solid form, for example, as tablets, sugar-coated tablets, suppositories, or capsules, or in liquid form, for example, as solutions, suspensions, or emulsions. In addition, the pharmaceutical preparation may optionally contain adjuvants, for example, preservatives, stabilizers, wetting agents, or emulsifiers; and salts for altering osmotic pressure or buffers.

As a carrier system, it is also possible to use a surface-active auxiliary agent, for example, bile salt(s) or animal or plant phospholipids, as well as mixtures thereof, and liposomes or components thereof.

In addition, the dosage of the compound of Formula 1 according to the present invention for humans is generally preferably in the range of 0.1 mg/day to 2,000 mg/day, based on an adult patient weighing 70 kg. The compound according to the present invention may be administered once daily or divided into several doses per day. However, the above dosage may vary depending on the health condition, age, body weight, and sex of the patient, as well as the dosage form and severity of the disease, and thus the scope of the present invention is not limited to the dosage described above.

According to another embodiment of the present invention, there is provided a use of the compound of Formula 1 of the present invention, optical isomers thereof, diastereomers thereof, hydrates thereof, solvates thereof, and pharmaceutically acceptable salts thereof, or a pharmacologically acceptable salt thereof, for preventing or treating cancer or tumor.

According to another embodiment of the present invention, there is provided a method of preventing or treating cancer or tumor, comprising administering to a subject a compound selected from the group consisting of the compound of Formula 1 of the present invention, optical isomers thereof, diastereomers thereof, hydrates thereof, solvates thereof, and pharmaceutically acceptable salts thereof. Preferably, the subject refers to an individual or a patient, but is not limited thereto.

According to another embodiment of the present invention, there is provided a method of preventing or treating cancer or tumor in a subject in need of administration of an inhibitor of the compound and a standard-of-care formulation, comprising administering a therapeutically effective amount of a standard-of-care formulation comprising a compound selected from the group consisting of the compound of Formula 1 of the present invention, optical isomers thereof, diastereomers thereof, hydrates thereof, solvates thereof, and pharmaceutically acceptable salts thereof.

According to another embodiment of the present invention, there is provided a method of preventing or treating cancer or tumor in a subject in need of administration of the composition, comprising administering a therapeutically effective amount of a composition comprising a compound selected from the group consisting of the compound of Formula 1 of the present invention, optical isomers thereof, diastereomers thereof, hydrates thereof, solvates thereof, and pharmaceutically acceptable salts thereof.

According to another embodiment of the present invention, there is provided a method of inhibiting SOS1 binding to RAS family proteins and/or RAC1 in a subject or a cell, comprising administering to the subject a compound selected from the group consisting of the compound of Formula 1 of the present invention, optical isomers thereof, diastereomers thereof, hydrates thereof, solvates thereof, and pharmaceutically acceptable salts thereof.

According to another embodiment of the present invention, there is provided a method of inhibiting tyrosine kinase in a subject or a cell, comprising administering to the subject a compound selected from the group consisting of the compound of Formula 1 of the present invention, optical isomers thereof, diastereomers thereof, hydrates thereof, solvates thereof, and pharmaceutically acceptable salts thereof.

According to another embodiment of the present invention, there is provided a method of preventing or treating cancer or tumor in a subject, comprising administering to the subject a compound selected from the group consisting of the compound of Formula 1 of the present invention, optical isomers thereof, diastereomers thereof, hydrates thereof, solvates thereof, and pharmaceutically acceptable salts thereof.

According to another embodiment of the present invention, there is provided a method of preventing or treating cancer or tumor treatable by inhibiting SOS1 protein binding to RAS family proteins and/or RAC1 in a subject or a cell, comprising administering to the subject a compound selected from the group consisting of the compound of Formula 1 of the present invention, optical isomers thereof, diastereomers thereof, hydrates thereof, solvates thereof, and pharmaceutically acceptable salts thereof.

Hereinafter, the present invention will be described in more detail with reference to the following Examples and Experimental Examples. However, these Examples and Experimental Examples are provided only for illustration to facilitate understanding of the present invention, and the scope of the present invention is not limited thereto in any manner.

### Example 1: (R)-2-methyl-3-(1-((4-methyl-7-(methylamino)-6-(morpholine-4-carbonyl)phthalazin-1-yl)amino)ethyl)benzonitrile

### [Step-1] Preparation of dimethyl 2-amino-5-bromoterephthalate

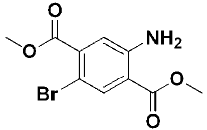

Dimethyl aminoterephthalate (20.0 g, 95.60 mmol) and N-bromosuccinimide (18.7 g, 105.16 mmol) were dissolved in chloroform (1000 mL), and the mixture was refluxed with stirring at room temperature for 16 hours. Upon completion of the reaction, water was added, and the mixture was extracted with dichloromethane. The organic layer was dried over anhydrous sodium sulfate and concentrated under reduced pressure. The residue was purified by column chromatography (hexane:ethyl acetate = 3:1 (v/v)) to obtain the title compound (15.0 g) in a yield of 54%.

¹H-NMR (300 MHz, CDCl₃): *δ* 8.11 (s, 1H), 7.07 (s, 1H), 5.84 (s, 2H), 3.94 (s, 3H), 3.91 (s, 3H).

### [Step-2] Preparation of dimethyl 2-bromo-5-(methylamino)terephthalate

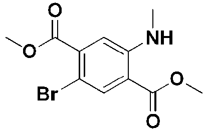

Dimethyl 2-amino-5-bromoterephthalate (15.0 g, 52.06 mmol) obtained in [Step-1], methyl iodide (9.7 mL, 156.18 mmol), and potassium carbonate (28.8 g, 208.26 mmol) were dissolved in DMF (150 mL), and the mixture was refluxed with stirring at 110°C for 16 hours. Upon completion of the reaction, the mixture was cooled to room temperature, water was added, and the mixture was acidified with 2 N hydrochloric acid to pH 3. The mixture was extracted with ethyl acetate, and the obtained organic layer was dried over anhydrous sodium sulfate and concentrated under reduced pressure. The resulting residue was purified by column chromatography (hexane:ethyl acetate = 9:1 (v/v)) to obtain the title compound (7.4 g) in a yield of 47%.

¹H-NMR (300 MHz, CDCl₃): *δ* 8.13 (s, 1H), 7.68 (s, 1H), 7.01 (s, 1H), 3.96 (s, 3H), 3.89 (s, 3H), 2.94 (d, 3H).

### [Step-3] Preparation of dimethyl 2-(1-butoxyvinyl)-5-(methylamino)terephthalate

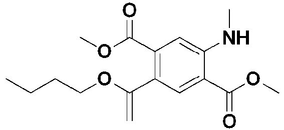

Dimethyl 2-bromo-5-(methylamino)terephthalate (7.4 g, 24.49 mmol) obtained in [Step-2], butyl vinyl ether (15.7 mL, 122.47 mmol), Pd(OAc)2 (550 mg, 2.45 mmol), triphenylphosphine (1.3 g, 4.90 mmol), and triethylamine (4.1 mL, 29.39 mmol) were dissolved in acetonitrile (80 mL), and the mixture was refluxed with stirring at 100°C for 16 hours. Upon completion of the reaction, the mixture was cooled to room temperature, filtered through a Celite-packed filter, and washed with dichloromethane. The filtrate was concentrated under reduced pressure, and the next reaction was performed without further purification.

¹H-NMR (300 MHz, DMSO-*d₆*): *δ* 8.43 (s, 1H), 6.85 (s, 1H), 3.83 (s, 1H).

### [Step-4] Preparation of dimethyl 2-acetyl-5-(methylamino)terephthalate

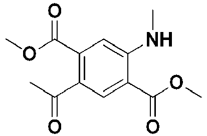

Dimethyl 2-(1-butoxyvinyl)-5-(methylamino)terephthalate (7.87 g, 24.49 mmol) obtained in [Step-3] and 6 N hydrochloric acid (33 mL, 195.91 mmol) were dissolved in tetrahydrofuran (80 mL), and the mixture was refluxed with stirring at room temperature for 1 hour. Upon completion of the reaction, water was added, and the mixture was extracted with ethyl acetate. The organic layer was washed with an aqueous sodium bicarbonate solution and an aqueous sodium chloride solution, dried over anhydrous sodium sulfate, and concentrated under reduced pressure. The resulting residue was purified by column chromatography (hexane:ethyl acetate = 1:1 (v/v)) to obtain the title compound (5.5 g) in a yield of 84%.

¹H-NMR (300 MHz, CDCl₃): *δ* 8.42 (s, 1H), 8.33 (s, 1H), 6.72 (s, 1H), 3.95 (s, 3H), 3.93 (s, 3H), 3.00 (d, 3H), 2.54 (s, 3H).

### [Step-5] Preparation of methyl 1-hydroxy-4-methyl-7-(methylamino)phthalazine-6-carboxylate

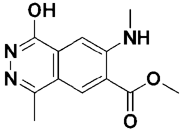

Dimethyl 2-acetyl-5-(methylamino)terephthalate (5.5 g, 20.73 mmol) obtained in [Step-4] and hydrazine monohydrate (1.1 g, 20.73 mmol) were dissolved in ethanol (70 mL), and the mixture was refluxed with stirring at 95°C for 1 hour. Upon completion of the reaction, the reaction solution was cooled to room temperature to obtain a solid product. The solid was collected by filtration under reduced pressure and washed with ethanol to obtain the title compound (4.8 g) in a yield of 93%.

¹H-NMR (300 MHz, DMSO-*d*₆): *δ* 12.16 (s, 1H), 8.26 (s, 1H), 7.92 (d, 1H), 7.29 (s, 1H), 3.91 (s, 3H), 2.96 (d, 3H), 2.42 (s, 3H).

### [Step-6] Preparation of methyl 1-chloro-4-methyl-7-(methylamino)phthalazine-6-carboxylate

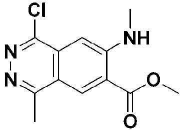

Methyl 1-hydroxy-4-methyl-7-(methylamino)phthalazine-6-carboxylate (1.0 g, 4.04 mmol) obtained in [Step-5] was dissolved in phosphoryl chloride (20 mL), and the mixture was refluxed with stirring at 130°C for 4 hours. Upon completion of the reaction, the mixture was cooled to room temperature and concentrated. The resulting residue was neutralized by dropwise addition of a saturated aqueous sodium bicarbonate solution, extracted with dichloromethane, dried over anhydrous sodium sulfate, and concentrated under reduced pressure. The residue was purified by column chromatography (hexane:ethyl acetate = 1:1 (v/v)) to obtain the title compound (730 mg) in a yield of 68%.

¹H-NMR (300 MHz, DMSO-*d*₆): *6* 8.61 (s, 1H), 8.09 (s, 1H), 6.98 (s, 1H), 3.96 (s, 3H), 3.93 (s, 3H), 3.01 (d, 3H), 2.79 (s, 3H).

### [Step-7] Preparation of 3-bromo-N-methoxy-N,2-dimethylbenzamide

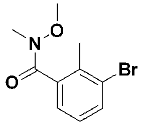

3-Bromo-2-methylbenzoic acid (100 g, 465.01 mmol), HATU (194.49 g, 511.51 mmol), N,O-dimethylhydroxylamine hydrochloride (50 g, 511.51 mmol), and N,N-diisopropylethylamine (245.44 mL, 1395.0 mmol) were dissolved in DMF (800 mL), and the mixture was stirred at room temperature for 16 hours. Upon completion of the reaction, water was added, and the mixture was extracted with ethyl acetate. The organic layer was dried over anhydrous sodium sulfate and concentrated under reduced pressure, and the next reaction was performed without further purification.

¹H-NMR (300 MHz, CDCl₃): 6 7.60 (d, 1H), 7.21 (d, 1H), 7.09 (t, 1H), 3.42-3.31 (m, 3H), 2.38 (s, 3H), 1.48 (m, 3H).

### [Step-8] Preparation of 1-(3-bromo-2-methylphenyl)ethan-1-one

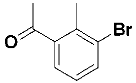

3-Bromo-N-methoxy-N,2-dimethylbenzamide (120 g, 464.92 mmol) obtained in [Step-7] was dissolved in tetrahydrofuran (700 mL) at room temperature and cooled to 0°C. 3 M methylmagnesium bromide (310 mL, 929.84 mmol) was slowly added dropwise to the reaction mixture. The mixture was refluxed with stirring at 45°C for 1.5 hours. Upon completion of the reaction, the mixture was cooled to 0°C, and 6 N hydrochloric acid (500 mL) was slowly added dropwise. The mixture was refluxed with stirring at 45°C for 1 hour. After cooling to room temperature, a saturated aqueous ammonium chloride solution was added, and the mixture was extracted with ethyl acetate. The organic layer was dried over anhydrous sodium sulfate and concentrated under reduced pressure, and the next reaction was performed without further purification.

¹H-NMR (300 MHz, CD₃OD): *δ* 7.73 (d, 1H), 7.64 (d, 1H), 7.23 (m, 1H), 2.57 (s, 3H), 2.35 (s, 3H).

### [Step-9] Preparation of (S,E)-N-(1-(3-bromo-2-methylphenyl)ethylidene)-2-methylpropane-2-sulfinamide

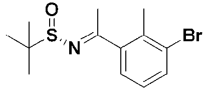

1-(3-Bromo-2-methylphenyl)ethan-1-one (200 g, 938.66 mmol) obtained in [Step-8], (S)-(-)-2-methyl-2-propanesulfinamide (170.6 g, 1407.98 mmol), and titanium ethoxide (725 mL, 3285.4 mmol) were dissolved in tetrahydrofuran (1600 mL) at room temperature, and the mixture was refluxed with stirring at 85°C for 12 hours. Upon completion of the reaction, the mixture was cooled to room temperature, filtered through a Celite-packed filter, and washed with ethyl acetate. The filtrate was concentrated under reduced pressure, and the residue was purified by column chromatography (hexane:ethyl acetate = 10:1 to 3:1 (v/v)) to obtain the title compound (213 g) in a yield of 72%.

¹H-NMR (300 MHz, CD₃OD): *δ* 7.65 (m, 1H), 7.31-7.05 (m, 2H), 2.69 (m, 3H), 2.51-2.31 (m, 3H), 1.28 (m, 9H).

### [Step-10] Preparation of (S)-N-((R)-1-(3-bromo-2-methylphenyl)ethyl)-2-methylpropane-2-sulfinamide

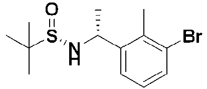

(S,E)-N-(1-(3-Bromo-2-methylphenyl)ethylidene)-2-methylpropane-2-sulfinamide (213 g, 675.08 mmol) obtained in [Step-9] was dissolved in tetrahydrofuran (1100 mL) and cooled to -78°C. 1 M L-selectride (1352 mL, 1352 mmol) was slowly added dropwise at -78°C. The mixture was stirred at -78°C for 0.5 hour and then stirred at 0°C for 2.5 hours. Upon completion of the reaction, a saturated aqueous ammonium chloride solution was added at 0°C, and the mixture was stirred at room temperature for 30 minutes. The mixture was extracted with ethyl acetate, dried over anhydrous sodium sulfate, and concentrated under reduced pressure. The residue was purified by column chromatography (hexane:ethyl acetate = 3:1 (v/v)) to obtain the title compound (187.5 g) in a yield of 87%.

¹H-NMR (300 MHz, CDCl₃): *δ* 7.49 (d, 1H), 7.35 (d, 1H), 7.05 (m, 1H), 4.87 (m, 1H), 3.26 (m, 1H), 2.47 (s, 3H), 1.55-1.50 (m, 3H), 1.20 (m, 9H).

### [Step-11] Preparation of (R)-1-(3-bromo-2-methylphenyl)ethan-1-amine

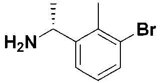

(S)-N-((R)-1-(3-Bromo-2-methylphenyl)ethyl)-2-methylpropane-2-sulfinamide (187.5 g, 589.12 mmol) obtained in [Step-10] was dissolved in 1,4-dioxane (900 mL) and methanol (450 mL), and a solution of hydrochloric acid in dioxane (290 mL, 4 M) was slowly added dropwise. The mixture was refluxed with stirring for 2 hours. Upon completion of the reaction, the mixture was concentrated under reduced pressure, and the next reaction was performed without further purification.

### [Step-12] Preparation of tert-butyl (R)-1-(3-bromo-2-methylphenyl)ethylcarbamate

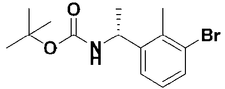

(R)-1-(3-Bromo-2-methylphenyl)ethan-1-amine (121.5 g, 484.91 mmol) obtained in [Step-11], di-tert-butyl dicarbonate (157.54 mL, 678.88 mmol), and N,N-diisopropylethylamine (204.75 mL, 1163.8 mmol) were dissolved in dichloromethane (1220 mL), and the mixture was stirred at room temperature for 1 hour. Upon completion of the reaction, the mixture was concentrated under reduced pressure, and the next reaction was performed without further purification.

¹H-NMR (300 MHz, CD₃OD): *δ* 7.46 (d, 1H), 7.34 (d, 1H), 7.10 (m, 1H), 4.96 (m, 1H), 2.47 (s, 3H), 1.53-1.25 (m, 12H).

### [Step-13] Preparation of tert-butyl (R)-1-(3-cyano-2-methylphenyl)ethylcarbamate

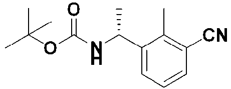

tert-Butyl (R)-1-(3-bromo-2-methylphenyl)ethylcarbamate (150 g, 477.37 mmol) obtained in [Step-12], Pd(PPh3)4 (55.17 g, 47.737 mmol), and zinc cyanide (67.28 g, 572.85 mmol) were dissolved in DMF (1500 mL), and the mixture was refluxed with stirring at 130°C for 2 hours. Upon completion of the reaction, the mixture was cooled to room temperature, filtered through a Celite-packed filter, and washed with ethyl acetate. The filtrate was concentrated under reduced pressure, and the residue was purified by column chromatography (hexane:ethyl acetate = 5:1 to 1:1 (v/v)) to obtain the title compound (105 g) in a yield of 84%.

¹H-NMR (300 MHz, CDCl₃): *δ* 7.54 (m, 1H), 7.30 (m, 2H), 5.01-4.84 (m, 2H), 2.61 (s, 3H), 1.42 (m, 12H).

### [Step-14] Preparation of (R)-3-(1-aminoethyl)-2-methylbenzonitrile 2,2,2-trifluoroacetate

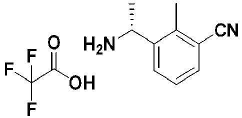

tert-Butyl (R)-1-(3-cyano-2-methylphenyl)ethylcarbamate (105 g, 401.78 mmol) obtained in [Step-13] was dissolved in dichloromethane (1000 mL), and trifluoroacetic acid (153.73 mL, 20008.9 mmol) was slowly added dropwise at room temperature. The mixture was refluxed with stirring for 3 hours. Upon completion of the reaction, the mixture was concentrated under reduced pressure to obtain the title compound (105.5 g) in a yield of 96%.

¹H-NMR (300 MHz, DMSO-*d*₆): *δ* 8.38 (bs, 3H), 7.83 (m, 2H), 7.55 (m, 1H), 4.70 (m, 1H), 2.49 (s, 3H), 2.95 (d, 3H).

### [Step-15] Preparation of sec-butyl 1-(((R)-1-(3-cyano-2-methylphenyl)ethyl)amino)-4-methyl-7-(methylamino)phthalazine-6-carboxylate

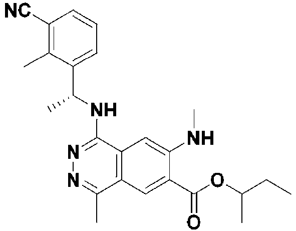

Methyl 1-chloro-4-methyl-7-(methylamino)phthalazine-6-carboxylate (150 mg, 0.56 mmol) obtained in [Step-6], (R)-3-(1-aminoethyl)-2-methylbenzonitrile 2,2,2-trifluoroacetate (201 mg, 0.73 mmol) obtained in [Step-14], magnesium chloride (161 mg, 1.69 mmol), and N,N-diisopropylethylamine (0.59 mL, 3.39 mmol) were dissolved in 2-butanol (1.5 mL), and the mixture was refluxed with stirring at 120°C for 48 hours. Upon completion of the reaction, the mixture was cooled to room temperature, water was added, and the mixture was extracted with ethyl acetate. The organic layer was dried over anhydrous sodium sulfate and concentrated under reduced pressure. The residue was purified by column chromatography (hexane:ethyl acetate = 3:1 to 1:1 (v/v)) to obtain the title compound (85 mg) in a yield of 35%.

¹H-NMR (300 MHz, DMSO-*d*₆): *δ* 8.36 (s, 1H), 7.88 (m, 1H), 7.86 (d, 1H), 7.76 (d, 1H), 7.61 (m, 1H), 7.58-7.29 (m, 2H), 5.58 (m, 1H), 3.04 (d, 3H), 2.51 (s, 3H), 1.72 (m, 2H), 1.69 (m, 3H), 1.35 (m, 3H), 0.98 (m, 3H).

### [Step-16] Preparation of (R)-1-((1-(3-cyano-2-methylphenyl)ethyl)amino)-4-methyl-7-(methylamino)phthalazine-6-carboxylic acid

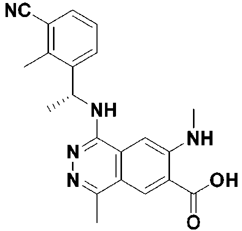

sec-Butyl 1-(((R)-1-(3-cyano-2-methylphenyl)ethyl)amino)-4-methyl-7-(methylamino)phthalazine-6-carboxylate (85 mg, 0.20 mmol) obtained in [Step-15] and sodium hydroxide (78 mg, 1.97 mmol) were dissolved in a solution (2 mL) of tetrahydrofuran:methanol:water = 2:1:1, and the mixture was refluxed with stirring at room temperature for 3 hours. Upon completion of the reaction, the mixture was concentrated under reduced pressure, and the resulting residue was acidified at low temperature with a 6 N hydrochloric acid solution until pH 2. The obtained solid was collected by filtration under reduced pressure and washed with water. The solid was dried in an oven at 55°C to obtain the title compound (50 mg) in a yield of 68%.

¹H-NMR (300 MHz, DMSO-*d*₆): *δ* 8.58 (s, 1H), 8.37 (m, 1H), 7.77 (d, 1H), 7.66 (d, 1H), 7.48 (s, 1H), 7.36 (t, 1H), 5.36 (m 1H), 3.12 (s, 3H), 2.71-2.67 (m, 6H), 1.62 (d, 3H).

### [Step-17] Preparation of (R)-2-methyl-3-(1-((4-methyl-7-(methylamino)-6-(morpholine-4-carbonyl)phthalazin-1-yl)amino)ethyl)benzonitrile

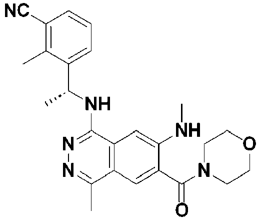

(R)-1-((1-(3-cyano-2-methylphenyl)ethyl)amino)-4-methyl-7-(methylamino)phthalazine-6-carboxylic acid (50 mg, 0.13 mmol) obtained in [Step-16], morpholine (0.1 mL, 1.07 mmol), HATU (152 mg, 0.40 mmol), and N,N-diisopropylethylamine (0.2 mL, 1.07 mmol) were dissolved in DMF (1 mL), and the mixture was stirred at room temperature for 4 hours. Upon completion of the reaction, the mixture was cooled to room temperature, water was added, and the mixture was extracted with ethyl acetate. The organic layer was dried over anhydrous sodium sulfate and concentrated under reduced pressure. The residue was purified by column chromatography (dichloromethane:ethyl acetate:methanol = 10:10:1 (v/v/v)) to obtain the title compound (10 mg) in a yield of 17%.
¹H-NMR (300 MHz, DMSO-*d*₆): *δ* 7.76 (d, 1H), 7.60 (m, 2H), 7.32 (m, 2H), 7.23 (s, 1H), 6.07 (d, 1H), 5.59 (m, 1H), 3.64 (m, 6H), 3.16 (m, 2H), 2.94 (d, 3H), 2.67 (s, 3H), 2.49 (s, 3H), 1.55 (d, 3H).
MS (ESI+, m/z): 445.2 [M+H]⁺

### Example 2: (R)-2-methyl-3-(1-((4-methyl-7-(methylamino)-6-(thiomorpholine-4-carbonyl)phthalazin-1-yl)amino)ethyl)benzonitrile

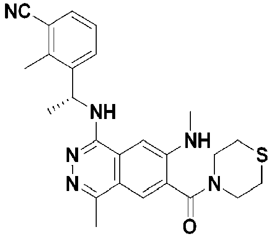

The procedure of Example 1 was repeated, except that thiomorpholine (82 mg, 0.79 mmol) was used instead of morpholine in [Step-17] of Example 1, to obtain the title compound (80 mg) in a yield of 32%.
¹H-NMR (300 MHz, DMSO-*d*₆): δ 7.78 (m, 1H), 7.60 (m, 2H), 7.35 (m, 2H), 7.21 (s, 1H), 6.02 (m, 1H), 5.61 (m, 1H), 3.92 (m, 2H), 3.45 (m, 2H), 2.94 (m, 3H), 2.77 (m, 2H), 2.67 (s, 3H), 2.61 (m, 2H), 2.49 (s, 3H), 1.56 (m, 3H).
MS (ESI+, m/z): 461.2 [M+H]⁺

### Example 3: (R)-2-methyl-3-(1-((4-methyl-7-(methylamino)-6-(thiazolidine-3-carbonyl)phthalazin-1-yl)amino)ethyl)benzonitrile

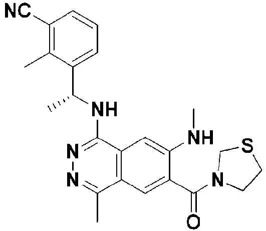

The procedure of Example 1 was repeated, except that thiazolidine (72 mg, 0.79 mmol) was used instead of morpholine in [Step-17] of Example 1, to obtain the title compound (56 mg) in a yield of 23%.
¹H-NMR (300 MHz, DMSO-*d*₆): δ 7.78 (m, 1H), 7.69 (s, 1H), 7.61 (m, 1H), 7.37 (m, 2H), 7.24 (s, 1H ), 6.22 (m, 1H), 5.62 (m, 1H), 4.66 (m, 2H), 3.87 (m, 2H), 3.05 (m, 2H), 2.95 (m, 3H), 2.67 (s, 3H), 2.49 (s, 3H), 1.56 (m, 3H).
MS (ESI+, m/z): 447.2 [M+H]+

### Example 4: (R)-3-(1-((6-(1,1-dioxothiomorpholine-4-carbonyl)-4-methyl-7-(methylamino)phthalazin-1-yl)amino)ethyl)-2-methylbenzonitriₗe

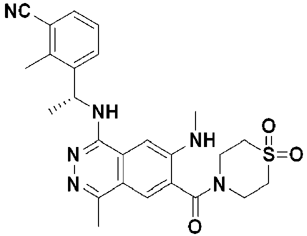

The procedure of Example 1 was repeated, except that thiomorpholine 1,1-dioxide (108 mg, 0.79 mmol) was used instead of morpholine in [Step-17] of Example 1, to obtain the title compound (80 mg) in a yield of 30%.
¹H-NMR (300 MHz, DMSO-*d₆*): δ 7.77 (m, 2H), 7.61 (m, 1H), 7.37 (m, 2H), 7.23 (s, 1H), 6.10 (m, 1H), 5.62 (m, 1H), 4.06 (m, 2H), 3.59 (m, 2H), 3.43 (m, 2H), 3.26 (m, 2H), 2.95 (m, 3H), 2.67 (s, 3H), 2.49 (s, 3H), 1.56 (m, 3H).
MS (ESI+, m/z): 493.2 [M+H]⁺

### Example 5: (R)-1-((1-(3-cyano-2-methylphenyl)ethyl)amino)-4-methyl-7-(methylamino)-N-(tetrahydro-2H-pyran-4-yl)phthalazine-6-carboxamide

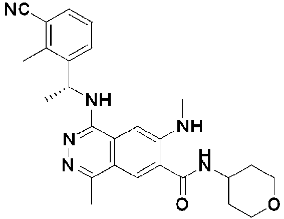

The procedure of Example 1 was repeated, except that tetrahydro-2H-pyran-4-amine hydrochloride (113 mg, 0.79 mmol) was used instead of morpholine in [Step-17] of Example 1, to obtain the title compound (14 mg) in a yield of 6%.
¹H-NMR (300 MHz, DMSO-*d*₆): δ 8.66-8.64 (m, 1H), 7.95 (s, 1H), 7.77-7.75 (m, 1H), 7.60-7.53 (m, 2H), 7.36-7.28 (m, 2H), 7.20 (s, 1H), 5.59-5.50 (m, 1H), 4.06-3.99 (m, 1H), 3.97-3.87 (m, 2H), 3.44-3.35 (m, 2H), 2.98-2.96 (m, 3H), 2.67 (s, 3H), 2.55 (s, 3H), 1.84-1.79 (m, 2H), 1.66-1.53 (m, 5H).
MS (ESI+, m/z): 459.2 [M+H]⁺

### Example 6: (R)-1-((1-(3-cyano-2-methylphenyl)ethyl)amino)-N,4-dimethyl-7-(methylamino)-N-(tetrahydro-2H-pyran-4-yl)phthalazine-6-carboxamide

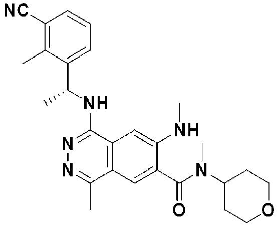

The procedure of Example 1 was repeated, except that N-methyltetrahydro-2H-pyran-4-amine (0.11 mL, 0.79 mmol) was used instead of morpholine in [Step-17] of Example 1, to obtain the title compound (25 mg) in a yield of 10%.
¹H-NMR (300 MHz, DMSO-*d*₆): δ 7.79-7.76 (m, 1H), 7.61-7.58 (m, 1H), 7.53 (s, 1H), 7.35-7.31 (m, 2H), 7.28-7.21 (m, 1H), 6.05-5.84 (m, 1H), 5.61- 5.52 (m, 1H), 4.04-3.82 (m, 3H), 3.79-3.37 (m, 2H), 2.93-2.91 (m, 3H), 2.67 (s, 3H), 2.47 (s, 6H), 1.85-1.80 (m, 3H), 1.78-1.74 (m, 4H).
MS (ESI+, m/z): 473.3 [M+H]⁺

### Example 7: 3-((1R)-1-((6-(hexahydro-1H-furo[3,4-c]pyrrole-5-carbonyl)-4-methyl-7-(methylamino)phthalazin-1-yl)amino)ethyl)-2-methylbenzonitrile

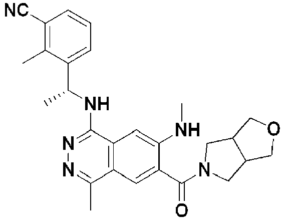

The procedure of Example 1 was repeated, except that hexahydro-1H-furo[3,4-c]pyrrole (90 mg, 0.79 mmol) was used instead of morpholine in [Step-17] of Example 1, to obtain the title compound (20 mg) in a yield of 7%.
¹H-NMR (300 MHz, DMSO-*d₆*): δ 7.78 (m, 1H), 7.64 (m, 2H), 7.35 (m, 2H), 7.22 (s, 1H), 6.16 (m, 1H), 5.62 (m, 1H), 3.61 (m, 8H), 3.14 (m, 1H), 2.95 (m, 4H), 2.68 (s, 3H), 2.51 (s, 3H), 1.57 (m, 3H).
MS (ESI+, m/z): 471.3 [M+H]+

### Example 8: (R)-2-methyl-3-(1-((4-methyl-7-(methylamino)-6-(4-methylpiperazine-1-carbonyl)phthalazin-1-yl)amino)ethyl)benzonitrile

The procedure of Example 1 was repeated, except that 1-methylpiperazine (0.09 mL, 0.79 mmol) was used instead of morpholine in [Step-17] of Example 1, to obtain the title compound (25 mg) in a yield of 10%.¹H-NMR (300 MHz, DMSO-*d*₆): δ 7.77-7.74 (m, 1H), 7.60-7.56 (m, 2H), 7.39-7.28 (m, 2H), 7.22 (s, 1H), 6.08-6.02 (m, 1H), 5.60-5.51 (m, 1H), 3.75-3.61 (m, 2H), 3.24-3.19 (m, 2H), 2.94-2.92 (m, 3H), 2.67 (s, 3H), 2.48 (s, 3H), 2.42-2.35 (m, 2H), 2.33-2.23 (m, 5H), 1.56-1.54 (m, 3H).
MS (ESI+, m/z): 458.3 [M+H]+

### Example 9: (R)-2-methyl-3-(1-((4-methyl-7-(methylamino)-6-(piperazine-1-carbonyl)phthalazin-1-yl)amino)ethyl)benzonitrile

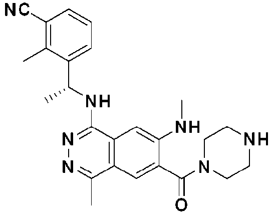

The procedure of Example 1 was repeated, except that piperazine (379 mg, 1.99 mmol) was used instead of morpholine in [Step-17] of Example 1, to obtain the title compound (50 mg) in a yield of 29%.
¹H-NMR (300 MHz, DMSO-*d*₆): δ 7.77-7.74 (m, 1H), 7.60-7.58 (m, 1H), 7.55 (s, 1H), 7.35-7.27 (m, 2H), 7.21 (s, 1H), 6.03-5.98 (m, 1H), 5.58-5.54 (m, 1H), 3.66-3.54 (m, 2H), 3.20-3.07 (m, 2H), 2.94-2.92 (m, 3H), 2.84-2.72 (m, 2H), 2.69-2.54 (m, 6H), 2.50 (s, 3H), 1.56-1.53 (m, 3H).
MS (ESI+, m/z): 444.3 [M+H]+

### Example 10: (R)-3-(1-((6-(4,4-difluoropiperidin-1-carbonyl)-4-methyl-7-(methylamino)phthalazin-1-yl)amino)ethyl)-2-methylbenzonitrile

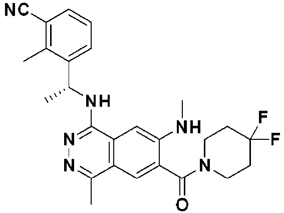

The procedure of Example 1 was repeated, except that 4,4-difluoropiperidine hydrochloride (161 mg, 0.99 mmol) was used instead of morpholine in [Step-17] of Example 1, to obtain the title compound (150 mg) in a yield of 47%.
¹H-NMR (300 MHz, DMSO-*d*₆): δ 8.33 (s, 1H), 8.03 (s, 1H), 7.74-7.71 (m, 1H), 7.66-7.64 (m, 1H), 7.46 (s, 1H), 7.38-7.33 (m, 1H), 7.13-7.06 (m, 1H), 5.41-5.32 (m, 1H), 3.81-3.71 (m, 2H), 3.66-3.58 (m, 2H), 3.29-3.10 (m, 4H), 3.03-3.01 (m, 3H), 2.67 (s, 6H), 1.62-1.59 (m, 3H).
MS (ESI+, m/z): 479.2 [M+H]+

### Example 11: (R)-2-methyl-3-(1-((4-methyl-7-(methylamino)-6-(2-oxa-6-azaspiro[3.3]heptane-6-carbonyl)phthalazin-1-yl)amino)ethyl)benzonitrile

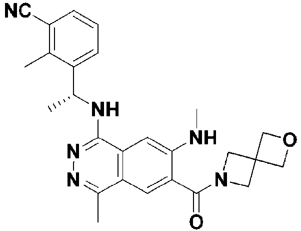

The procedure of Example 1 was repeated, except that 2-oxa-6-azaspiro[3.3]heptane (79 mg, 0.79 mmol) was used instead of morpholine in [Step-17] of Example 1, to obtain the title compound (60 mg) in a yield of 24%.
¹H-NMR (300 MHz, DMSO-*d₆*): δ 7.78 (m, 1H), 7.64 (m, 2H), 7.35 (m, 2H), 7.22 (s, 1H), 6.16 (m, 1H), 5.62 (m, 1H), 3.61 (m, 8H), 3.14 (m, 1H), 2.95 (m, 4H), 2.68 (s, 3H), 2.51 (s, 3H), 1.57 (m, 3H).
MS (ESI+, m/z): 457.2 [M+H]+

### Example 12: (R)-3-(1-((6-(4-ethylpiperazine-1-carbonyl)-4-methyl-7-(methylamino)phthalazin-1-yl)amino)ethyl)-2-methylbenzonitrile

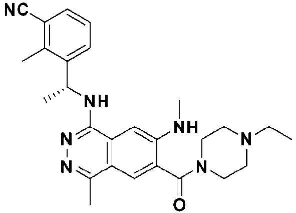

The procedure of Example 1 was repeated, except that 1-ethylpiperazine (0.10 mL, 0.79 mmol) was used instead of morpholine in [Step-17] of Example 1, to obtain the title compound (130 mg) in a yield of 52%.
¹H-NMR (300 MHz, DMSO-*d*₆): δ 8.28 (s, 1H), 7.94 (s, 1H), 7.74-7.71 (m, 1H), 7.66-7.64 (m, 1H), 7.44 (s, 1H), 7.38-7.33 (m, 1H), 7.06-6.98 (m, 1H), 5.43-5.33 (m, 1H), 3.84-3.55 (m, 2H), 3.27-3.13 (m, 2H), 3.02-3.01 (m, 3H), 2.90-2.85 (m, 2H), 2.79-7.72 (m, 6H), 2.69-2.54(m, 2H), 2.51 (s, 3H), 1.61-1.59 (m, 3H), 1.08-1.05 (m, 3H).
MS (ESI+, m/z): 472.3 [M+H]+

### Example 13: (R)-3-(1-((6-(4-cyclopropylpiperazine-1-carbonyl)-4-methyl-7-(methylamino)phthalazin-1-yl)amino)ethyl)-2-methylbenzonitrile

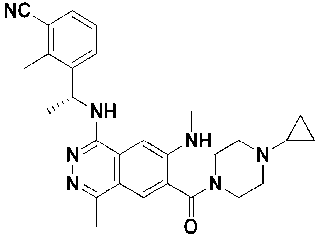

The procedure of Example 1 was repeated, except that 1-cyclopropylpiperazine (50 mg, 0.40 mmol) was used instead of morpholine in [Step-17] of Example 1, to obtain the title compound (40 mg) in a yield of 31%.
¹H-NMR (300 MHz, CDCl₃): δ 7.70(m, 1H), 7.64 (s, 1H), 7.51 (m, 1H), 7.24(m, 1H), 6.60 (s, 1H), 5.77 (m, 1H), 5.55 (m, 1H), 4.96 (m, 1H), 3.74 (m, 4H), 3.00 (m, 3H), 2.74 (m, 10H), 1.71 (m, 4H), 0.51 (m, 4H).
MS (ESI+, m/z): 484.3 [M+H]+

### Example 14: (R)-2-methyl-3-(1-((4-methyl-7-(methylamino)-6-(pyrrolidine-1-carbonyl)phthalazin-1-yl)amino)ethyl)benzonitrile

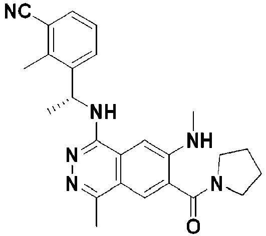

The procedure of Example 1 was repeated, except that pyrrolidine (113 mg, 1.59 mmol) was used instead of morpholine in [Step-17] of Example 1, to obtain the title compound (110 mg) in a yield of 24%.
¹H-NMR (300 MHz, CDCl₃): δ 7.78 (s, 1H), 7.70 (m, 1H), 7.52 (m, 1H), 7.25 (m, 1H), 6.57 (s, 1H), 6.14 (m, 1H), 5.77 (m, 1H), 4.93 (m, 1H), 3.74 (m, 2H), 3.51 (m, 2H), 2.99 (m, 3H), 2.75 (s, 3H), 2.69 (s, 3H), 2.00 (m, 4H), 1.66 (m, 3H).
MS (ESI+, m/z): 429.2 [M+H]⁺

### Example 15: (R)-2-methyl-3-(1-((4-methyl-7-(methylamino)-6-(piperidine-1-carbonyl)phthalazin-1-yl)amino)ethyl)benzonitrile

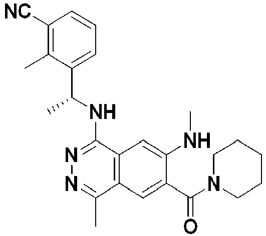

The procedure of Example 1 was repeated, except that piperidine (136 mg, 1.59 mmol) was used instead of morpholine in [Step-17] of Example 1, to obtain the title compound (170 mg) in a yield of 36%.
¹H-NMR (300 MHz, CDCl₃): δ 7.70 (m, 1H), 7.63 (s, 1H), 7.50 (m, 1H), 7.24 (m, 1H), 6.61 (s, 1H), 5.78 (m, 1H), 5.52 (m, 1H), 4.99 (m, 1H), 3.66 (m, 4H), 2.98 (m, 3H), 2.74 (s, 3H), 2.68 (s, 3H), 1.73 (m, 9H).
MS (ESI+, m/z): 443.3 [M+H]+

### Example 16: (R)-3-(1-((6-(azetidine-1-carbonyl)-4-methyl-7-(methylamino)phthalazin-1-yl)amino)ethyl)-2-methylbenzonitrile

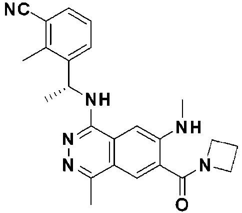

The procedure of Example 1 was repeated, except that azetidine hydrochloride (157 mg, 1.59 mmol) was used instead of morpholine in [Step-17] of Example 1, to obtain the title compound (110 mg) in a yield of 25%.
¹H-NMR (300 MHz, DMSO-*d*₆): δ 8.24 (s, 1H), 8.02 (s, 1H), 7.73-7.71 (m, 1H), 7.66-7.63 (m, 2H), 7.43 (s, 1H), 7.38-7.33 (m, 1H), 5.42-5.33 (m, 1H), 4.19-4.08 (m, 4H), 3.05-3.03 (m, 3H), 2.67-2.66 (m, 6H), 2.31-2.21 (m, 2H), 1.61-1.58 (m, 3H).
MS (ESI+, m/z): 415.2 [M+H]+

### Example 17: (R)-3-(1-((6-(3-fluoroazetidine-1-carbonyl)-4-methyl-7-(methylamino)phthalazin-1-yl)amino)ethyl)-2-methylbenzonitrile

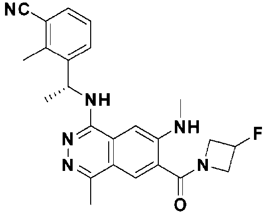

The procedure of Example 1 was repeated, except that 3-fluoroazetidine hydrochloride (182 mg, 1.59 mmol) was used instead of morpholine in [Step-17] of Example 1, to obtain the title compound (120 mg) in a yield of 26%.
¹H-NMR (300 MHz, DMSO-*d*₆): δ 7.73 (s, 1H), 7.61-7.58 (m, 1H),7.51-7.49 (m,1H), 7.48-7.45 (m, 1H), 7.33-7.26 (m, 2H), 7.03-6.96 (m, 1H), 5.57-5.31 (m, 2H), 4.52-4.08 (m, 4H), 2.98-2.97(m, 3H), 2.66 (s, 3H), 2.53 (s, 3H), 1.56-1.54 (m, 3H).
MS (ESI+, m/z): 433.2 [M+H]+

### Example 18: (R)-2-methyl-3-(1-((4-methyl-7-(methylamino)-6-(5-azaspiro[2.4]heptane-5-carbonyl)phthalazin-1-yl)amino)ethyl)benzonitrile

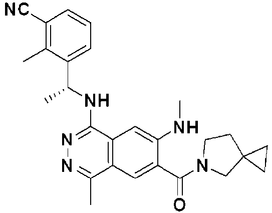

The procedure of Example 1 was repeated, except that 5-azaspiro[2.4]heptane (0.18 mL, 1.79 mmol) was used instead of morpholine in [Step-17] of Example 1, to obtain the title compound (200 mg) in a yield of 37%.
¹H-NMR (300 MHz, DMSO-*d*₆): δ 7.76-7.71 (m, 2H), 7.61-7.59 (m, 1H), 7.54-7.49 (m, 1H), 7.34-7.24 (m, 2H), 6.44-6.38 (m, 1H), 5.57-5.48 (m, 1H), 3.71-3.67 (m, 1H), 3.46-3.39 (m, 2H), 3.19-3.11 (m, 1H), 2.97-2.94(m, 3H), 2.67-2.66 (m, 3H), 2.54-2.52 (m, 3H), 1.87-73 (m, 2H), 1.57-1.54 (m, 3H), 0.68-0.60 (m, 4H).
MS (ESI+, m/z): 455.3 [M+H]+

### Example 19: 3-((1R)-1-((6-(2-oxa-5-azabicyclo[2.2.1]heptane-5-carbonyl)-4-methyl-7-(methylamino)phthalazin-1-yl)amino)ethyl)-2-methylbenzonitrile

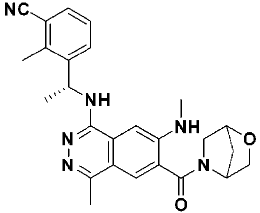

The procedure of Example 1 was repeated, except that 2-oxa-5-azabicyclo[2.2.1]heptane hydrochloride (248 mg, 1.79 mmol) was used instead of morpholine in [Step-17] of Example 1, to obtain the title compound (280 mg) in a yield of 51%.
¹H-NMR (300 MHz, DMSO-*d*₆): δ 7.77-7.72 (m, 2H), 7.61-7.51 (m, 2H), 7.34-7.26 (m, 2H), 6.40-6.37 (m, 1H), 5.55-5.51 (m, 1H), 4.90-4.4.14(m, 2H), 4.12-3.75 (m, 2H), 3.60-3.44 (m, 2H), 2.97-2.95 (m, 3H), 2.67 (s, 3H), 2.52 (s, 3H), 2.02-1.70 (m, 2H), 1.57-1.54 (m, 3H).
MS (ESI+, m/z): 457.2 [M+H]+

### Example 20: (R)-3-(1-((6-(4-cyclohexylpiperazine-1-carbonyl)-4-methyl-7-(methylamino)phthalazin-1-yl)amino)ethyl)-2-methylbenzonitrile

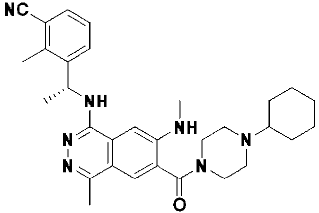

The procedure of Example 1 was repeated, except that 1-cyclohexylpiperazine (0.04 mL, 0.2 mmol) was used instead of morpholine in [Step-17] of Example 1, to obtain the title compound (33 mg) in a yield of 47%.
¹H-NMR (300 MHz, DMSO-*d*₆): δ 7.78 (d, 1H), 7.61 (d, 1H), 7.54 (s, 1H), 7.35-7.28 (m, 2H), 7.21 (s, 1H), 6.00 (m, 1H), 5.59 (m, 1H), 3.65 (m, 1H), 2.94 (m, 2H), 2.89 (m, 3H), 2.73-2.40 (m, 10H), 2.25 (m, 4H), 1.74 (m, 4H), 1.17 (m, 4H).
MS (ESI+, m/z): 526.3 [M+H]+

### Example 21: (R)-3-(1-((6-(4-isopropylpiperazine-1-carbonyl)-4-methyl-7-(methylamino)phthalazin-1-yl)amino)ethyl)-2-methylbenzonitrile

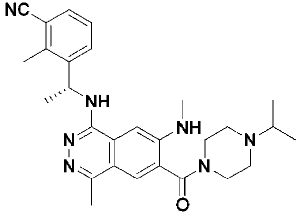

The procedure of Example 1 was repeated, except that 1-isopropylpiperazine (0.02 mL, 0.16 mmol) was used instead of morpholine in [Step-17] of Example 1, to obtain the title compound (30 mg) in a yield of 58%.
¹H-NMR (300 MHz, DMSO-*d*₆): δ 7.78 (d, 1H), 7.61 (d, 1H), 7.55 (s, 1H), 7.35-7.28 (m, 2H), 7.21 (s, 1H), 6.02 (m, 1H), 5.57 (m, 1H), 3.66 (m, 1H), 3.16 (m, 2H), 2.94 (m, 3H), 2.71 (m, 4H), 2.52 (m, 4H), 2.27 (m, 2H), 1.56 (m, 3H), 0.95 (m, 6H).
MS (ESI+, m/z): 486.3 [M+H]+

### Example 22: (R)-2-methyl-3-(1-((4-methyl-7-(methylamino)-6-(4-morpholinopiperidin-1-carbonyl)phthalazin-1-yl)amino)ethyl)benzonitrile

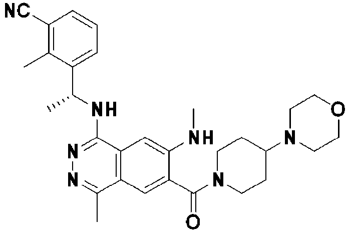

The procedure of Example 1 was repeated, except that 4-(piperidin-4-yl)morpholine (306 mg, 1.79 mmol) was used instead of morpholine in [Step-17] of Example 1, to obtain the title compound (35 mg) in a yield of 5%.
¹H-NMR (300 MHz, CDCl₃): δ 7.70 (m, 1H), 7.62 (s, 1H), 7.51 (m, 1H), 7.22 (m, 1H), 6.61 (s, 1H), 5.79 (m, 1H), 5.53 (m, 1H), 3.77 (m, 4H), 2.99 (m, 5H), 2.75 (s, 3H), 2.68 (s, 3H), 2.59 (m, 7H), 1.98 (m, 2H), 1.67 (m, 3H), 1.48 (m, 2H).
MS (ESI+, m/z): 528.3 [M+H]+

### Example 23: (R)-1-((1-(3-cyano-2-methylphenyl)ethyl)amino)-4-methyl-7-(methylamino)-N-(2-morpholinoethyl)phthalazine-6-carboxamide

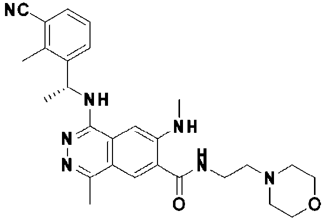

The procedure of Example 1 was repeated, except that 2-morpholinoethanamine (0.24 mL, 1.79 mmol) was used instead of morpholine in [Step-17] of Example 1, to obtain the title compound (500 mg) in a yield of 85%.
¹H-NMR (300 MHz, MeOD-*d*₄): δ 8.32 (s, 1H), 7.74-7.72 (m, 1H),7.55-7.52 (m, 1H), 7.46 (s, 1H), 7.31-7.26 (m, 1H), 5.49-5.42 (m, 1H), 3.73-3.70 (m, 4H), 3.61-3.56 (m, 2H), 3.13 (s, 3H), 2.75 (s, 6H), 2.67-2.63 (m, 2H), 2.59-2.56 (m, 4H), 1.67-1.65 (m, 3H).
MS (ESI+, m/z): 488.3 [M+H]+

### Example 24: (R)-2-methyl-3-(1-((4-methyl-7-(methylamino)-6-(1,4-oxazepane-4-carbonyl)phthalazin-1-yl)amino)ethyl)benzonitrile

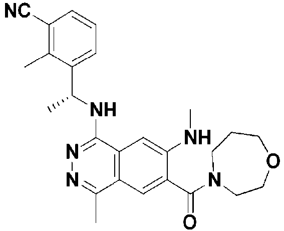

The procedure of Example 1 was repeated, except that 1,4-oxazepane (161 mg, 1.59 mmol) was used instead of morpholine in [Step-17] of Example 1, to obtain the title compound (170 mg) in a yield of 34%.
¹H-NMR (300 MHz, CDCl₃): δ 7.70 (m, 2H), 7.51 (m, 1H), 7.25 (m, 1H), 6.62 (s, 1H), 5.80 (m, 1H), 5.42 (m, 1H), 5.00 (m, 1H), 3.93 (m, 5H), 3.54 (m, 3H), 3.00 (m, 3H), 2.74 (s, 3H), 2.69 (s, 3H), 2.09 (m, 1H), 1.84 (m, 1H), 1.67 (m, 3H).
MS (ESI+, m/z): 459.3 [M+H]+

### Example 25: (R)-(1-((1-(3-amino-5-(trifluoromethyl)phenyl)ethyl)amino)-4-methyl-7-(methylamino)phthalazin-6-yl)(morpholino)methanone

### [Step-1] Preparation of dimethyl 2-amino-5-bromoterephthalate

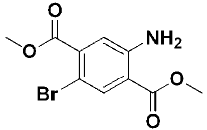

Dimethyl aminoterephthalate (20.0 g, 95.60 mmol) and N-bromosuccinimide (18.7 g, 105.16 mmol) were dissolved in chloroform (1000 mL), and the mixture was refluxed with stirring at room temperature for 16 hours. Upon completion of the reaction, water was added, and the mixture was extracted with dichloromethane. The organic layer was dried over anhydrous sodium sulfate and concentrated under reduced pressure. The resulting residue was purified by column chromatography (hexane:ethyl acetate = 3:1 (v/v)) to obtain the title compound (15.0 g) in a yield of 54%.1H-NMR (300 MHz, CDCl3): δ 8.11 (s, 1H), 7.07 (s, 1H), 5.84 (s, 2H), 3.94 (s, 3H), 3.91 (s, 3H).

¹H-NMR (300 MHz, CDCl₃): δ 8.11 (s, 1H), 7.07 (s, 1H), 5.84 (s, 2H), 3.94 (s, 3H), 3.91 (s, 3H).

### [Step-2] Preparation of dimethyl 2-bromo-5-(methylamino)terephthalate

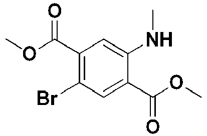

Dimethyl 2-amino-5-bromoterephthalate (15.0 g, 52.06 mmol) obtained in [Step-1], methyl iodide (9.7 mL, 156.18 mmol), and potassium carbonate (28.8 g, 208.26 mmol) were dissolved in DMF (150 mL), and the mixture was refluxed with stirring at 110°C for 16 hours. Upon completion of the reaction, the mixture was cooled to room temperature, water was added, and the mixture was acidified with 2 N hydrochloric acid to pH 3. The mixture was extracted with ethyl acetate, and the obtained organic layer was dried over anhydrous sodium sulfate and concentrated under reduced pressure. The resulting residue was purified by column chromatography (hexane:ethyl acetate = 9:1 (v/v)) to obtain the title compound (7.4 g) in a yield of 47%.

¹H-NMR (300 MHz, CDCl₃): δ 8.13 (s, 1H), 7.68 (s, 1H), 7.01 (s, 1H), 3.96 (s, 3H), 3.89 (s, 3H), 2.94 (d, 3H).

### [Step-3] Preparation of dimethyl 2-(1-butoxyvinyl)-5-(methylamino)terephthalate

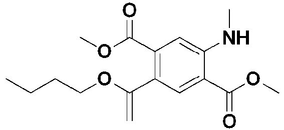

Dimethyl 2-bromo-5-(methylamino)terephthalate (7.4 g, 24.49 mmol) obtained in [Step-2], butyl vinyl ether (15.7 mL, 122.47 mmol), Pd(OAc)2 (550 mg, 2.45 mmol), triphenylphosphine (1.3 g, 4.90 mmol), and triethylamine (4.1 mL, 29.39 mmol) were dissolved in acetonitrile (80 mL), and the mixture was refluxed with stirring at 100°C for 16 hours. Upon completion of the reaction, the mixture was cooled to room temperature, filtered through a Celite-packed filter, and washed with dichloromethane. The filtrate was concentrated under reduced pressure, and the next reaction was performed without further purification.

### [Step-4] Preparation of dimethyl 2-acetyl-5-(methylamino)terephthalate

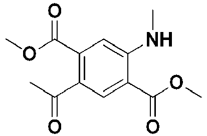

Dimethyl 2-(1-butoxyvinyl)-5-(methylamino)terephthalate (7.87 g, 24.49 mmol) obtained in [Step-3] and 6 N hydrochloric acid (33 mL, 195.91 mmol) were dissolved in tetrahydrofuran (80 mL), and the mixture was refluxed with stirring at room temperature for 1 hour. Upon completion of the reaction, water was added, and the mixture was extracted with ethyl acetate. The organic layer was washed with an aqueous sodium bicarbonate solution and an aqueous sodium chloride solution, dried over anhydrous sodium sulfate, and concentrated under reduced pressure. The resulting residue was purified by column chromatography (hexane:ethyl acetate = 1:1 (v/v)) to obtain the title compound (5.5 g) in a yield of 84%.

¹H-NMR (300 MHz, CDCl₃): δ 8.42 (s, 1H), 8.33 (s, 1H), 6.72 (s, 1H), 3.95 (s, 3H), 3.93 (s, 3H), 3.00 (d, 3H), 2.54 (s, 3H).

### [Step-5] Preparation of methyl 1-hydroxy-4-methyl-7-(methylamino)phthalazine-6-carboxylate

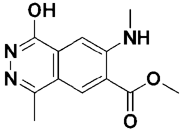

Dimethyl 2-acetyl-5-(methylamino)terephthalate (5.5 g, 20.73 mmol) obtained in [Step-4] and hydrazine monohydrate (1.1 g, 20.73 mmol) were dissolved in ethanol (70 mL), and the mixture was refluxed with stirring at 95°C for 1 hour. Upon completion of the reaction, the reaction solution was cooled to room temperature to obtain a solid product. The solid was collected by filtration under reduced pressure and washed with ethanol to obtain the title compound (4.8 g) in a yield of 93%.

¹H-NMR (300 MHz, DMSO-*d*₆): δ 12.16 (s, 1H), 8.26 (s, 1H), 7.92 (d, 1H), 7.29 (s, 1H), 3.91 (s, 3H), 2.96 (d, 3H), 2.42 (s, 3H).

### [Step-6] Preparation of 1-hydroxy-4-methyl-7-(methylamino)phthalazine-6-carboxylic acid

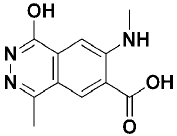

Methyl 1-hydroxy-4-methyl-7-(methylamino)phthalazine-6-carboxylate (4.8 g, 19.41 mmol) obtained in [Step-5] and sodium hydroxide (7.8 g, 194.14 mmol) were dissolved in a solution (160 mL) of tetrahydrofuran:methanol:water = 2:1:1, and the mixture was refluxed with stirring at room temperature for 2 hours. Upon completion of the reaction, the mixture was concentrated under reduced pressure, and the resulting residue was acidified at low temperature with a 6 N hydrochloric acid solution until pH 2. The obtained solid was collected by filtration under reduced pressure and washed with water. The solid was dried in an oven at 55°C to obtain the title compound (4.8 g) in a yield of 91%.

¹H-NMR (300 MHz, DMSO-*d*₆): δ 12.14 (s, 1H), 8.30 (s, 1H), 7.27 (s, 1H), 2.96 (d, 3H), 2.43 (s, 3H).

### [Step-7] Preparation of (1-hydroxy-4-methyl-7-(methylamino)phthalazin-6-yl)(morpholino)methanone

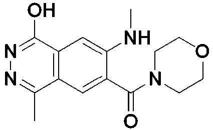

1-Hydroxy-4-methyl-7-(methylamino)phthalazine-6-carboxylic acid (4.8 g, 17.80 mmol) obtained in [Step-6], morpholine (3.1 mL, 35.60 mmol), HATU (8.8 g, 23.14 mmol), and DIPEA (15.5 mL, 88.99 mmol) were dissolved in DMF (100 mL), and the mixture was stirred at room temperature for 3 days. Upon completion of the reaction, water was added, and the mixture was extracted with ethyl acetate, dried over anhydrous sodium sulfate, and concentrated under reduced pressure. The residue was purified by column chromatography (dichloromethane:methanol = 20:1 (v/v)) to obtain the title compound (510 mg) in a yield of 9%.

¹H-NMR (300 MHz, DMSO-*d*₆): δ 12.12 (s, 1H), 7.56 (s, 1H), 7.18 (s, 1H), 6.20 (d,1H), 3.64 (m, 6H), 3.16 (m, 2H), 2.84 (d, 3H), 2.41 (s, 3H).

### [Step-8] Preparation of (1-chloro-4-methyl-7-(methylamino)phthalazin-6-yl)(morpholino)methanone

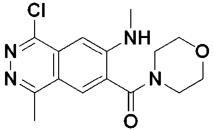

1-Hydroxy-4-methyl-7-(methylamino)phthalazin-6-yl)(morpholino)methanone (510 mg, 1.69 mmol) obtained in [Step-7] was dissolved in phosphoryl chloride (10 mL), and the mixture was refluxed with stirring at 110°C for 1 hour. Upon completion of the reaction, the reaction solution was cooled to room temperature and concentrated under reduced pressure. The residue was dissolved in dichloromethane and neutralized at 0°C by dropwise addition to an aqueous sodium bicarbonate solution. The mixture was extracted with dichloromethane, dried over anhydrous sodium sulfate, and concentrated under reduced pressure. The residue was purified by column chromatography (dichloromethane:methanol = 20:1 (v/v)) to obtain the title compound (290 mg) in a yield of 53%.

¹H-NMR (300 MHz, DMSO-*d*₆): δ 7.94 (s, 1H), 6.87 (s, 1H), 6.67 (d, 1H), 3.64 (m, 6H), 3.16 (m, 2H), 2.90 (d, 3H), 2.77 (s, 3H).

### [Step-9] Preparation of 1-(3-nitro-5-(trifluoromethyl)phenyl)ethan-1-one

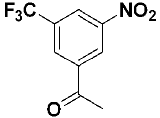

1-Bromo-3-nitro-5-trifluoromethylbenzene (100 g, 362.96 mmol), tributyl(1-ethylvinyl)tin (159.4 mL, 471.85 mmol), Pd(PPh3)2Cl2 (10.2 g, 14.52 mmol), and triethylamine (101 mL, 725.93 mmol) were dissolved in 1,4-dioxane (500 mL), and the mixture was refluxed with stirring at 120°C for 3 hours. Upon completion of the reaction, the reaction solution was cooled to room temperature, and tetrahydrofuran (250 mL) and 6 N hydrochloric acid (250 mL) were added, followed by reflux with stirring at room temperature for 2 hours. Then, 1 N aqueous potassium fluoride solution (900 mL) was added, followed by reflux with stirring at room temperature for 12 hours. Upon completion of the reaction, the mixture was filtered through a Celite-packed filter and washed with ethyl acetate. The filtrate was washed with water, dried over anhydrous sodium sulfate, and concentrated under reduced pressure. The residue was purified by column chromatography (hexane:ethyl acetate = 15:1 to 10:1 (v/v)) to obtain the title compound (66 g) in a yield of 78%.

¹H-NMR (300 MHz, CDCl₃): δ 8.95 (s, 1H), 8.68 (s, 1H), 8.54 (s, 1H), 2.76 (s, 3H).

### [Step-10] Preparation of (R,E)-2-methyl-N-(1-(3-nitro-5-(trifluoromethyl)phenyl)ethylidene)propane-2-sulfinamide

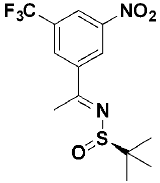

1-(3-Nitro-5-(trifluoromethyl)phenyl)ethan-1-one (66 g, 283.09 mmol) obtained in [Step-9], (R)-(+)-2-methyl-2-propanesulfinamide (42.4 g, 339.71 mmol), and titanium ethoxide (71.3 mL, 339.71 mmol) were dissolved in tetrahydrofuran (660 mL), and the mixture was refluxed with stirring at 85°C for 3 hours. Upon completion of the reaction, water was added, and the mixture was extracted with ethyl acetate. The organic layer was filtered through a Celite-packed filter and washed with ethyl acetate. The filtrate was washed with water, dried over anhydrous sodium sulfate, and concentrated under reduced pressure, and the next reaction was performed without further purification.

¹H-NMR (300 MHz, CDCl₃): δ 8.86 (s, 1H), 8.60 (s, 1H), 8.43 (s, 1H), 2.89 (s, 3H), 1.37 (s, 9H).

### [Step-11] Preparation of (R)-2-methyl-N-((R)-1-(3-nitro-5-(trifluoromethyl)phenyl)ethyl)propane-2-sulfinamide

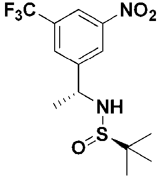

(R,E)-2-Methyl-N-(1-(3-nitro-5-(trifluoromethyl)phenyl)ethylidene)propane-2-sulfinamide (94 g, 279.5 mmol) obtained in [Step-10] was dissolved in tetrahydrofuran (660 mL) and water (14 mL), and the mixture was cooled to -30°C. Sodium borohydride (19 g, 503.1 mmol) was slowly added dropwise, and the mixture was stirred for 2 hours. Upon completion of the reaction, water was slowly added dropwise, followed by stirring for 1.5 hours. The mixture was extracted with ethyl acetate, and the organic layer was washed with water, dried over anhydrous sodium sulfate, and concentrated under reduced pressure. The residue was purified by column chromatography (hexane:ethyl acetate = 5:1 (v/v)) to obtain the title compound (35 g) in a yield of 37%.

¹H-NMR (300 MHz, DMSO-*d*₆): δ 8.63 (s, 1H), 8.38 (s, 1H), 8.30 (s, 1H), 6.08 (d, 1H), 4.74-4.64 (m, 1H), 1.47 (d, 3H), 1.14 (s, 9H).

### [Step-12] Preparation of (R)-1-(3-nitro-5-(trifluoromethyl)phenyl)ethan-1-amine hydrochloride

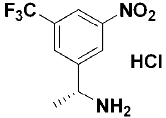

(R)-2-Methyl-N-((R)-1-(3-nitro-5-(trifluoromethyl)phenyl)ethyl)propane-2-sulfinamide (35 g, 103.5 mmol) obtained in [Step-11] was dissolved in 1,4-dioxane (355 mL), and 4 N hydrochloric acid in 1,4-dioxane (105 mL) was slowly added dropwise. The mixture was stirred at room temperature for 2.5 hours. Upon completion of the reaction, the mixture was concentrated under reduced pressure, and ether was added to the residue to form a solid. The solid was filtered to obtain the title compound (27 g) in a yield of 96%.

¹H-NMR (300 MHz, DMSO-*d*₆): δ 8.76 (s, 1H), 8.63 (bs, 2H), 8.52 (s, 1H), 8.44 (s, 1H), 4.76 (m, 1H), 1.59 (d, 3H).

### [Step-13] Preparation of (R)-3-(1-aminoethyl)-5-(trifluoromethyl)aniline hydrochloride

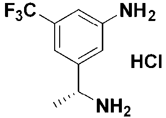

(R)-1-(3-Nitro-5-(trifluoromethyl)phenyl)ethan-1-amine hydrochloride (27 g, 99.76 mmol) obtained in [Step-12] was dissolved in methanol (405 mL), Pd/C (2.7 g) was slowly added, and the mixture was stirred under hydrogen gas for 2 hours. Upon completion of the reaction, the mixture was filtered through a Celite-packed filter, and the filtrate was concentrated under reduced pressure to obtain the title compound (23 g) in a yield of 96%.

¹H-NMR (300 MHz, DMSO-*d*₆): δ 8.46 (bs, 2H), 6.97 (s, 1H), 6.85 (s, 2H), 5.75 (s, 2H), 4.33 (m, 1H), 1.49 (d, 3H).

### [Step-14] Preparation of (R)-(1-((1-(3-amino-5-(trifluoromethyl)phenyl)ethyl)amino)-4-methyl-7-(methylamino)phthalazin-6-yl)(morpholino)methanone

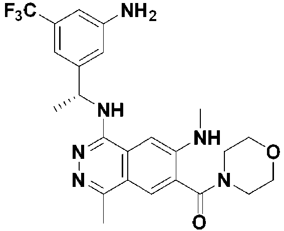

1-Chloro-4-methyl-7-(methylamino)phthalazin-6-yl)(morpholino)methanone (290 mg, 0.9 mmol) obtained in [Step-8], (R)-3-(1-aminoethyl)-5-(trifluoromethyl)aniline hydrochloride (261 mg, 1.08 mmol) obtained in [Step-13], cesium fluoride (412 mg, 2.71 mmol), and N,N-diisopropylethylamine (0.79 mL, 4.52 mmol) were dissolved in DMF (5 mL), and the mixture was stirred at room temperature for 5 hours. Upon completion of the reaction, the mixture was cooled to room temperature, water was added, and the mixture was extracted with ethyl acetate. The organic layer was dried over anhydrous sodium sulfate and concentrated under reduced pressure. The residue was purified by column chromatography (ethyl acetate:methanol = 10:1 (v/v)) to obtain the title compound (52 mg) in a yield of 12%.
¹H-NMR (300 MHz, DMSO-*d*₆): δ 7.61 (s, 1H), 7.20 (m, 2H), 6.85 (d, 2H), 6.67 (s, 1H), 6.04 (m, 1H), 5.40 (m, 3H), 3.64 (m, 6H), 3.16 (m, 2H), 2.93 (d, 3H), 2.49 (s, 3H), 1.55 (d, 3H).
MS (ESI+, m/z): 489.2 [M+H]⁺

### Example 26: (R)-(1-((1-(5-amino-3-(difluoromethyl)-2-fluorophenyl)ethyl)amino)-4-methyl-7-(methylamino)phthalazin-6-yl)(morpholino)methanone

### [Step-1] Preparation of 1-bromo-3-(difluoromethyl)-2-fluorobenzene

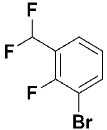

3-Bromo-2-fluorobenzaldehyde (150 g, 724.1 mmol) was dissolved in dichloromethane (1200 mL) and cooled to 0°C. Diethylaminosulfur trifluoride (150 mL, 868.92 mmol) was slowly added dropwise. The temperature was raised to room temperature, and the mixture was stirred for 4 hours. Upon completion of the reaction, the reaction mixture was slowly added dropwise to ice water. The mixture was extracted with dichloromethane, and the obtained organic layer was washed with a saturated aqueous sodium bicarbonate solution, dried over anhydrous sodium sulfate, and concentrated under reduced pressure to obtain the title compound (159 g) in a yield of 97.6%.

¹H-NMR (300 MHz, CDCl₃): δ 7.79-7.67 (m, 1H), 7.59-7.54 (m, 1H), 7.19 (m, 1H), 7.16-6.72 (m, 1H).

### [Step-2] Preparation of 1-bromo-3-(difluoromethyl)-2-fluoro-5-nitrobenzene

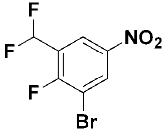

1-Bromo-3-(difluoromethyl)-2-fluorobenzene (159 g, 706.64 mmol) obtained in [Step-1] was dissolved in sulfuric acid (450 mL) and nitric acid (55 mL) at 0°C, the temperature was raised to room temperature, and the mixture was stirred for 2 hours. Upon completion of the reaction, the mixture was slowly added dropwise to ice water. The mixture was extracted with ethyl acetate. The organic layer was washed with a saturated aqueous sodium bicarbonate solution, dried over anhydrous sodium sulfate, and concentrated under reduced pressure, and the next reaction was performed without further purification.

### [Step-3] Preparation of 1-(3-(difluoromethyl)-2-fluoro-5-nitrophenyl)ethan-1-one

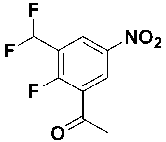

The procedure of [Step-9] of Example 25 was repeated, except that 1-bromo-3-(difluoromethyl)-2-fluoro-5-nitrobenzene (180 g, 666.67 mmol) obtained in [Step-2] was used instead of 1-bromo-3-nitro-5-trifluoromethylbenzene, to obtain the title compound (153 g) in a yield of 98% without further purification.

### [Step-4] Preparation of (R,E)-N-(1-(3-(difluoromethyl)-2-fluoro-5-nitrophenyl)ethylidene)-2-methylpropane-2-sulfinamide

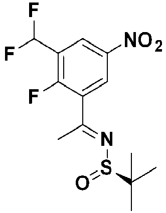

The procedure of [Step-10] of Example 25 was repeated, except that 1-(3-(difluoromethyl)-2-fluoro-5-nitrophenyl)ethan-1-one (153 g, 656.3 mmol) obtained in [Step-3] was used instead of 1-(3-nitro-5-(trifluoromethyl)phenyl)ethan-1-one, to obtain the title compound (193 g) in a yield of 87% without further purification.

### [Step-5] Preparation of (R)-N-((R)-1-(3-(difluoromethyl)-2-fluoro-5-nitrophenyl)ethyl)-2-methylpropane-2-sulfinamide

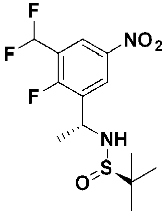

The procedure of [Step-11] of Example 25 was repeated, except that (R,E)-N-(1-(3-(difluoromethyl)-2-fluoro-5-nitrophenyl)ethylidene)-2-methylpropane-2-sulfinamide (193 g, 573.8 mmol) obtained in [Step-4] was used instead of (R,E)-2-methyl-N-(1-(3-nitro-5-(trifluoromethyl)phenyl)ethylidene)propane-2-sulfinamide, to obtain the title compound (24.5 g) in a yield of 13%.

¹H-NMR (300 MHz, CDCl₃): δ 8.47 (m, 2H), 7.21-6.76 (m, 1H), 4.94-4.85 (m, 1H), 3.66 (m, 1H), 1.64-1.61 (m, 3H), 1.27 (m, 9H).

### [Step-6] Preparation of (R)-1-(3-(difluoromethyl)-2-fluoro-5-nitrophenyl)ethan-1-amine hydrochloride

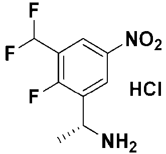

The procedure of [Step-12] of Example 25 was repeated, except that (R)-N-((R)-1-(3-(difluoromethyl)-2-fluoro-5-nitrophenyl)ethyl)-2-methylpropane-2-sulfinamide (35 g, 103.44 mmol) obtained in [Step-5] was used instead of (R)-2-methyl-N-((R)-1-(3-nitro-5-(trifluoromethyl)phenyl)ethyl)propane-2-sulfinamide, to obtain the title compound (28 g) in a yield of 99%.

¹H-NMR (300 MHz, DMSO-*d*₆): δ 8.93-8.87 (m, 3H), 8.53 (m, 1H), 7.55-7.19 (m, 1H), 4.79-4.73 (m, 1H), 1.61 (m, 3H).

### [Step-7] Preparation of (R)-3-(1-aminoethyl)-5-(difluoromethyl)-4-fluoroaniline hydrochloride

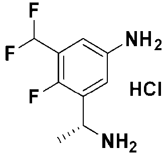

The procedure of [Step-13] of Example 25 was repeated, except that (R)-1-(3-(difluoromethyl)-2-fluoro-5-nitrophenyl)ethan-1-amine hydrochloride (1.5 g, 5.54 mmol) obtained in [Step-6] was used instead of (R)-1-(3-nitro-5-(trifluoromethyl)phenyl)ethan-1-amine hydrochloride, to obtain the title compound (1.46 g) in a yield of 99% without further purification.

### [Step-8] Preparation of (R)-(1-((1-(5-amino-3-(difluoromethyl)-2-fluorophenyl)ethyl)amino)-4-methyl-7-(methylamino)phthalazin-6-yl)(morpholino)methanone

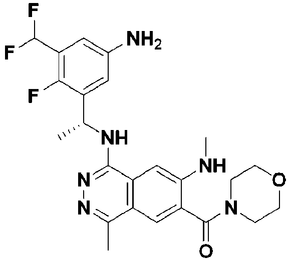

(1-Chloro-4-methyl-7-(methylamino)phthalazin-6-yl)(morpholino)methanone (60 mg, 0.19 mmol) obtained in [Step-8] of Example 25, (R)-3-(1-aminoethyl)-5-(difluoromethyl)-4-fluoroaniline hydrochloride (90 mg, 0.37 mmol) obtained in [Step-7], magnesium chloride (27 mg, 0.28 mmol), and N,N-diisopropylethylamine (0.13 mL, 0.75 mmol) were dissolved in 2-butanol (1.2 mL), and the mixture was refluxed with stirring at 110°C for 16 hours. Upon completion of the reaction, the mixture was cooled to room temperature, water was added, and the mixture was extracted with dichloromethane. The organic layer was dried over anhydrous sodium sulfate and concentrated under reduced pressure. The residue was purified by column chromatography (dichloromethane:methanol = 7:1 (v/v)) to obtain the title compound (15 mg) in a yield of 16%.
¹H-NMR (300 MHz, DMSO-*d*₆): δ 7.63 (s, 1H), 7.29-6.89 (m, 2H), 6.69 (m, 1H), 6.57 (m, 1H), 6.12 (m, 1H), 5.55 (m, 1H), 5.10 (m, 2H), 3.69-3.40 (m 6H), 3.31-3.18 (m, 2H), 2.96 (m, 3H), 1.58 (m, 3H).
MS (ESI+, m/z): 489.2 [M+H]⁺

### Example 27: (R)-(1-((1-(3-(1,1-difluoro-2-hydroxyethyl)phenyl)ethyl)amino)-4-methyl-7-(methylamino)phthalazin-6-yl)(morpholino)methanone

### [Step-1] Preparation of ethyl 2-(3-acetylphenyl)-2,2-difluoroacetate

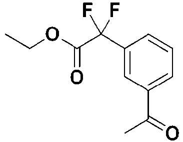

1-(3-Iodophenyl)ethanone (10 g, 40.64 mmol), ethyl 2-bromo-2,2-difluoroacetate (13 mL, 101.61 mmol), and copper (6.45 g, 101.61 mmol) were dissolved in DMSO (20 mL), and the mixture was refluxed with stirring at 80°C for 3 hours. Upon completion of the reaction, the mixture was cooled to room temperature, water was added, and the mixture was filtered through a Celite-packed filter. The filtrate was extracted with ethyl acetate. The organic layer was dried over anhydrous sodium sulfate and concentrated under reduced pressure to obtain the title compound (5.3 g) in a yield of 53.8%.

¹H-NMR (300 MHz, DMSO-*d*₆): δ 8.22 (m, 1H), 8.08 (s, 1H), 7.86 (m, 1H), 4.36 (m, 2H), 2.63 (s, 3H), 1.24 (m 3H).

### [Step-2] Preparation of ethyl (R,Z)-2-(3-(1-((tert-butylsulfinyl)imino)ethyl)phenyl)-2,2-difluoroacetate

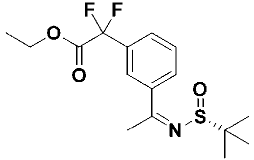

Ethyl 2-(3-acetylphenyl)-2,2-difluoroacetate (5.3 g, 21.88 mmol) obtained in [Step-1], (R)-2-methylpropane-2-sulfinamide (15.6 g, 54.7 mmol), and titanium ethoxide (14.3 mL, 54.7 mmol) were dissolved in tetrahydrofuran (55 mL), and the mixture was refluxed with stirring at 80°C for 16 hours. Upon completion of the reaction, water was added, and the mixture was extracted with ethyl acetate. The organic layer was filtered through a Celite-packed filter and washed with ethyl acetate. The filtrate was washed with water, dried over anhydrous sodium sulfate, and concentrated under reduced pressure. The residue was purified by column chromatography (hexane:ethyl acetate = 10:1 to 5:1 (v/v)) to obtain the title compound (5.1 g) in a yield of 67.5%.

¹H-NMR (300 MHz, CDCl₃): δ 8.20 (s, 1H), 8.11 (m, 1H), 7.74 (m, 1H), 7.55 (m, 1H), 4.36-4.29 (m, 2H), 2.81 (s, 3H), 1.35-1.27 (m, 12H).

### [Step-3] Preparation of (R)-N-((R)-1-(3-(1,1-difluoro-2-hydroxyethyl)phenyl)ethyl)-2-methylpropane-2-sulfinamide

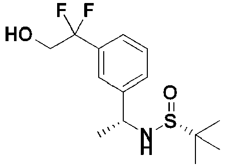

Ethyl (R,Z)-2-(3-(1-((tert-butylsulfinyl)imino)ethyl)phenyl)-2,2-difluoroacetate (5.1 g, 14.77 mmol) obtained in [Step-2] was dissolved in tetrahydrofuran (50 mL) and water (5 mL), and the mixture was cooled to -78°C. Sodium borohydride (1.23 g, 32.48 mmol) was slowly added dropwise, and the mixture was stirred at room temperature for 1 hour. Upon completion of the reaction, a saturated aqueous ammonium chloride solution was slowly added dropwise, the mixture was stirred for 0.5 hour, and the mixture was extracted with ethyl acetate. The organic layer was washed with water, dried over anhydrous sodium sulfate, and concentrated under reduced pressure. The residue was purified by column chromatography (hexane:ethyl acetate = 3:1 (v/v)) to obtain the title compound (1.65 g) in a yield of 37%.

¹H-NMR (300 MHz, CDCl₃): δ 7.55 (s, 1H), 7.48-7.43 (m, 3H), 4.58 (m, 1H), 3.97-3.91 (m, 2H), 3.47 (m, 1H), 2.93 (t, 1H), 1.57 (m, 3H), 1.28 (m, 9H).

### [Step-4] Preparation of (R)-2-(3-(1-aminoethyl)phenyl)-2,2-difluoroethan-1-ol hydrochloride

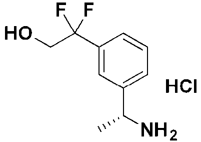

(R)-N-((R)-1-(3-(1,1-difluoro-2-hydroxyethyl)phenyl)ethyl)-2-methylpropane-2-sulfinamide (330 mg, 1.08 mmol) obtained in [Step-3] was dissolved in 1,4-dioxane (4 mL), and 4 N hydrochloric acid in 1,4-dioxane (4 mL) was slowly added dropwise. The mixture was stirred at room temperature for 16 hours. Upon completion of the reaction, the mixture was concentrated under reduced pressure to obtain the title compound (225 mg) in a yield of 87.6% without further purification.

### [Step-5] Preparation of (R)-(1-((1-(3-(1,1-difluoro-2-hydroxyethyl)phenyl)ethyl)amino)-4-methyl-7-(methylamino)phthalazin-6-yl)(morpholino)methanone

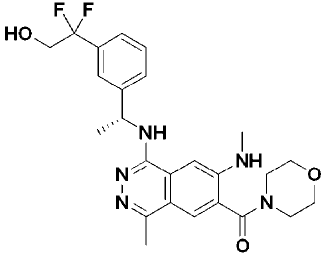

(1-Chloro-4-methyl-7-(methylamino)phthalazin-6-yl)(morpholino)methanone (130 mg, 0.41 mmol) obtained in [Step-8] of Example 25, (R)-2-(3-(1-aminoethyl)phenyl)-2,2-difluoroethan-1-ol hydrochloride (190 mg, 0.8 mmol) obtained in [Step-4], magnesium chloride (58 mg, 0.61 mmol), and N,N-diisopropylethylamine (0.28 mL, 1.62 mmol) were dissolved in 2-butanol (1.5 mL), and the mixture was refluxed with stirring at 110°C for 16 hours. Upon completion of the reaction, the mixture was cooled to room temperature, water was added, and the mixture was extracted with dichloromethane. The organic layer was dried over anhydrous sodium sulfate and concentrated under reduced pressure. The residue was purified by column chromatography (dichloromethane:methanol = 10:1 (v/v)) to obtain the title compound (15 mg) in a yield of 8%.
¹H-NMR (300 MHz, DMSO-*d*₆): δ 7.63-7.54 (m, 3H), 7.43-7.32 (m, 3H), 7.24 (s, 1H), 6.15 (m, 1H), 5.59-5.51 (m, 2H), 3.88-3.60 (m, 8H), 3.29-3.23 (m, 2H), 3.21 (m, 2H), 3.18 (m, 3H), 1.62 (d, 3H).
MS (ESI+, m/z): 486.2 [M+H]⁺

### Example 28: (R)-3-(1-((7-methoxy-4-methyl-6-(morpholinomethyl)phthalazin-1-yl)amino)ethyl)-2-methylbenzonitrile

### [Step-1] Preparation of methyl 2-bromo-5-methoxy-4-methylbenzoate

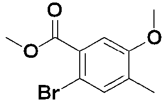

Methyl 3-methoxy-4-methylbenzoate (10 g, 55.49 mmol) was mixed with acetic acid (50 mL) and water (50 mL), and bromine (2.84 mL, 55.49 mmol) was added dropwise. After completion of the addition, the mixture was refluxed with stirring at 60°C for 2 hours. Upon completion of the reaction, the mixture was cooled to room temperature to obtain a solid product. The solid was collected by filtration under reduced pressure and washed with water. The filtered solid was dried to obtain the title compound (13.0 g) in a yield of 90%.

¹H-NMR (300 MHz, CDCl₃): δ 7.42 (s, 1H), 7.28 (s, 1H), 3.94 (s, 3H), 3.86 (s, 3H), 2.23 (s, 3H).

### [Step-2] Preparation of methyl 2-bromo-4-(bromomethyl)-5-methoxybenzoate

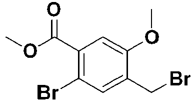

Methyl 2-bromo-5-methoxy-4-methylbenzoate (13.0 g, 50.17 mmol) obtained in [Step-1], N-bromosuccinimide (10.72 g, 60.21 mmol), and azobisisobutyronitrile (8.24 g, 50.17 mmol) were dissolved in chloroform (200 mL), and the mixture was refluxed with stirring at 70°C for 3 hours. Upon completion of the reaction, the mixture was cooled to room temperature and an aqueous sodium bicarbonate solution was added. The mixture was extracted with dichloromethane, and the obtained organic layer was dried over anhydrous sodium sulfate. The dried organic layer was filtered and concentrated under reduced pressure. The residue was purified by column chromatography (hexane:ethyl acetate = 9:1 (v/v)) to obtain the title compound (13.0 g) in a yield of 76%.

¹H-NMR (300 MHz, CDCl₃): δ 7.62 (s, 1H), 7. 32 (s, 1H), 4.47 (s, 2H), 3.96 (s, 3H), 3.94 (s, 3H).

### [Step-3] Preparation of methyl 2-bromo-5-methoxy-4-(morpholinomethyl)benzoate

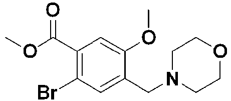

Methyl 2-bromo-4-(bromomethyl)-5-methoxybenzoate (13.0 g, 38.46 mmol) obtained in [Step-2], morpholine (3.65 mL, 42.31 mmol), and potassium carbonate (10.63 g, 76.93 mmol) were dissolved in acetonitrile (180 mL), and the mixture was stirred at room temperature for 18 hours. Upon completion of the reaction, an aqueous sodium bicarbonate solution was added. The mixture was extracted with ethyl acetate, and the obtained organic layer was dried over anhydrous sodium sulfate. The dried organic layer was filtered and concentrated under reduced pressure. The residue was purified by column chromatography (ethyl acetate:hexane = 1:5 (v/v) to 1:1 (v/v)) to obtain the title compound (11.0 g) in a yield of 83%.

¹H-NMR (300 MHz, CDCl₃): δ 7.68 (s, 1H), 7.28 (s, 1H), 3.93 (s, 3H), 3.84 (s, 3H), 3.73 (m, 4H), 3.51 (m, 2H), 2.49 (m, 4H).

### [Step-4] Preparation of methyl 2-(1-butoxyvinyl)-5-methoxy-4-(morpholinomethyl)benzoate

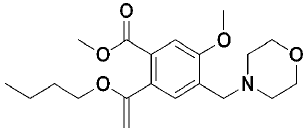

Methyl 2-bromo-5-methoxy-4-(morpholinomethyl)benzoate (11.0 g, 31.96 mmol) obtained in [Step-3], butyl vinyl ether (20.5 mL, 159.79 mmol), Pd(OAc)2 (718 mg, 3.20 mmol), triphenylphosphine (1.68 g, 6.39 mmol), and triethylamine (5.3 mL, 38.35 mmol) were dissolved in acetonitrile (110 mL), and the mixture was refluxed with stirring at 100°C for 16 hours. Upon completion of the reaction, the mixture was cooled to room temperature, filtered through a Celite-packed filter, and washed with dichloromethane. The filtrate was concentrated under reduced pressure, and the next reaction was performed without further purification.

### [Step-5] Preparation of methyl 2-acetyl-5-methoxy-4-(morpholinomethyl)benzoate

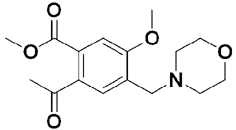

Methyl 2-(1-butoxyvinyl)-5-methoxy-4-(morpholinomethyl)benzoate (11.6 g, 31.92 mmol) obtained in [Step-4] and 6 N hydrochloric acid (42 mL, 255.33 mmol) were dissolved in tetrahydrofuran (120 mL), and the mixture was refluxed with stirring at room temperature for 1 hour. Upon completion of the reaction, the mixture was concentrated under reduced pressure, and water was slowly added dropwise. The mixture was extracted with ethyl acetate. The organic layer was washed with an aqueous sodium bicarbonate solution and an aqueous sodium chloride solution, dried over anhydrous sodium sulfate, and concentrated under reduced pressure. The residue was purified by column chromatography (hexane:ethyl acetate = 1:1 (v/v)) to obtain the title compound (4.7 g) in a yield of 47%.

¹H-NMR (300 MHz, CDCl₃): δ 7.68 (s, 1H), 7.17 (s, 1H), 3.92 (s, 3H), 3.91 (s, 3H), 3.75 (m, 4H), 3.37 (s, 2H), 2.54 (s, 3H), 2.52 (m, 4H).

### [Step-6] Preparation of 7-methoxy-4-methyl-6-(morpholinomethyl)phthalazin-1-ol

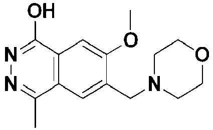

Methyl 2-acetyl-5-methoxy-4-(morpholinomethyl)benzoate (4.7 g, 15.29 mmol) obtained in [Step-5] and hydrazine monohydrate (1.17 g, 22.93 mmol) were dissolved in ethanol (50 mL), and the mixture was refluxed with stirring at 95°C for 1 hour. Upon completion of the reaction, the reaction solution was cooled to room temperature to obtain a solid product. The solid was collected by filtration under reduced pressure and washed with ethanol. The solid was dried to obtain the title compound (4.1 g) in a yield of 92%.

¹H-NMR (300 MHz, CDCl₃): δ 10.29 (s, 1H), 7.91 (s, 1H), 7.82 (s, 1H), 4.02 (s, 3H), 3.80 (m, 4H), 3.71 (s, 2H), 2.59 (m, 7H).

### [Step-7] Preparation of 4-((1-chloro-7-methoxy-4-methylphthalazin-6-yl)methyl)morpholine

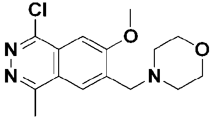

7-Methoxy-4-methyl-6-(morpholinomethyl)phthalazin-1-ol (1.0 g, 3.45 mmol) obtained in [Step-6] and phosphoryl chloride (0.96 mL, 10.36 mmol) were dissolved in acetonitrile (20 mL), and the mixture was refluxed with stirring at 100°C for 2 hours. Upon completion of the reaction, the reaction solution was cooled to room temperature and concentrated under reduced pressure. The residue was dissolved in dichloromethane and neutralized at 0°C by dropwise addition to an aqueous sodium bicarbonate solution. The mixture was extracted with dichloromethane, dried over anhydrous sodium sulfate, and concentrated under reduced pressure. The residue was purified by column chromatography (dichloromethane:methanol = 20:1 (v/v)) to obtain the title compound (850 mg) in a yield of 79%.

¹H-NMR (300 MHz, CDCl₃): δ 8.17 (s, 1H), 7.48 (s, 1H), 4.08 (s, 3H), 3.80 (m, 4H), 3.75 (s, 2H), 2.97 (s, 3H), 2.60 (m, 4H).

### [Step-8] Preparation of (R)-3-(1-((7-methoxy-4-methyl-6-(morpholinomethyl)phthalazin-1-yl)amino)ethyl)-2-methylbenzonitrile

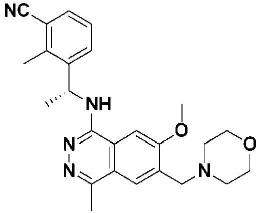

4-((1-Chloro-7-methoxy-4-methylphthalazin-6-yl)methyl)morpholine (350 mg, 1.13 mmol) obtained in [Step-7], (R)-3-(1-aminoethyl)-2-methylbenzonitrile 2,2,2-trifluoroacetate (467 mg, 1.70 mmol) obtained in [Step-14] of Example 1, magnesium chloride (324 mg, 3.41 mmol), and DIPEA (1.18 mL, 6.82 mmol) were dissolved in 2-butanol (7 mL), and the mixture was refluxed with stirring at 120°C for 48 hours. Upon completion of the reaction, the mixture was cooled to room temperature, water was added, and the mixture was extracted with ethyl acetate. The organic layer was dried over anhydrous sodium sulfate and concentrated under reduced pressure. The residue was purified by column chromatography (dichloromethane:methanol = 20:1 (v/v)) and (dichloromethane:ethyl acetate:methanol = 10:1:1 (v/v/v)) to obtain the title compound (105 mg) in a yield of 21%.
¹H-NMR (300 MHz, CDCl₃): δ 8.03 (s, 1H), 7.70 (m, 1H), 7.47 (m, 1H), 7.18 (m, 1H), 7.08 (s, 1H), 5.75 (m, 1H), 5.22 (s, 1H), 4.00 (s, 3H), 3.79 (m, 4H), 3.72 (s, 2H), 2.78 (s, 3H), 2.69 (s, 3H), 2.58 (m, 4H), 1.64 (m, 3H).
MS (ESI+, m/z): 432.2 [M+H]⁺

### Example 29: (R)-2-methyl-3-(1-((4-methyl-7-(methylamino)-6-(morpholinomethyl)phthalazin-1-yl)amino)ethyl)benzonitrile

### [Step-1] Preparation of dimethyl 2-amino-5-bromoterephthalate

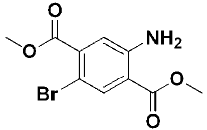

Dimethyl aminoterephthalate (135 g, 645.32 mmol) and N-bromosuccinimide (126 g, 709.85 mmol) were dissolved in chloroform (6750 mL), and the mixture was refluxed with stirring at room temperature for 16 hours. Upon completion of the reaction, water was added, and the mixture was extracted with dichloromethane. The organic layer was dried over anhydrous sodium sulfate and concentrated under reduced pressure. The residue was purified by column chromatography (hexane:ethyl acetate = 3:1 (v/v)) to obtain the title compound (102 g) in a yield of 55%.

¹H-NMR (300 MHz, CDCl₃): δ 8.11 (s, 1H), 7.07 (s, 1H), 5.84 (s, 2H), 3.94 (s, 3H), 3.91 (s, 3H).

### [Step-2] Preparation of dimethyl 2-bromo-5-(methylamino)terephthalate

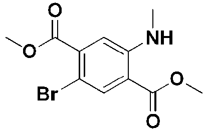

Dimethyl 2-amino-5-bromoterephthalate (96 g, 333.22 mmol) obtained in [Step-1], methyl iodide (41.5 mL, 666.44 mmol), and potassium carbonate (184.2 g, 1332.90 mmol) were dissolved in DMF (1200 mL), and the mixture was refluxed with stirring at 110°C for 16 hours. Upon completion of the reaction, the mixture was cooled to room temperature, water was added, and the mixture was acidified with 2 N hydrochloric acid to pH 3. The mixture was extracted with ethyl acetate, and the obtained organic layer was dried over anhydrous sodium sulfate and concentrated under reduced pressure. The residue was purified by column chromatography (hexane:ethyl acetate = 9:1 (v/v)) to obtain the title compound (50 g) in a yield of 50%.

¹H-NMR (300 MHz, CDCl₃): δ 8.13 (s, 1H), 7.68 (s, 1H), 7.01 (s, 1H), 3.96 (s, 3H), 3.89 (s, 3H), 2.94 (d, 3H).

### [Step-3] Preparation of dimethyl 2-(1-butoxyvinyl)-5-(methylamino)terephthalate

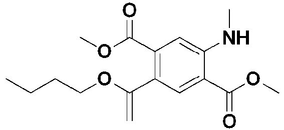

Dimethyl 2-amino-5-bromoterephthalate (96 g, 333.22 mmol) obtained in [Step-1], methyl iodide (41.5 mL, 666.44 mmol), and potassium carbonate (184.2 g, 1332.90 mmol) were dissolved in DMF (1200 mL), and the mixture was refluxed with stirring at 110°C for 16 hours. Upon completion of the reaction, the mixture was cooled to room temperature, water was added, and the mixture was acidified with 2 N hydrochloric acid to pH 3. The mixture was extracted with ethyl acetate, and the obtained organic layer was dried over anhydrous sodium sulfate and concentrated under reduced pressure. The residue was purified by column chromatography (hexane:ethyl acetate = 9:1 (v/v)) to obtain the title compound (50 g) in a yield of 50%.

### [Step-4] Preparation of dimethyl 2-acetyl-5-(methylamino)terephthalate

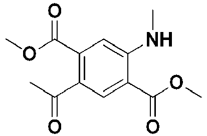

Dimethyl 2-(1-butoxyvinyl)-5-(methylamino)terephthalate (53 g, 164.92 mmol) obtained in [Step-3] and 6 N hydrochloric acid (155 mL) were dissolved in tetrahydrofuran (530 mL), and the mixture was refluxed with stirring at room temperature for 1 hour. Upon completion of the reaction, water was added, and the mixture was extracted with ethyl acetate. The organic layer was washed with an aqueous sodium bicarbonate solution and an aqueous sodium chloride solution, dried over anhydrous sodium sulfate, and concentrated under reduced pressure. The residue was purified by column chromatography (hexane:ethyl acetate = 1:1 (v/v)) to obtain the title compound (30 g) in a yield of 69%.

¹H-NMR (300 MHz, CDCl₃): δ 8.42 (s, 1H), 8.33 (s, 1H), 6.72 (s, 1H), 3.95 (s, 3H), 3.93 (s, 3H), 3.00 (d, 3H), 2.54 (s, 3H).

### [Step-5] Preparation of methyl 1-hydroxy-4-methyl-7-(methylamino)phthalazine-6-carboxylate

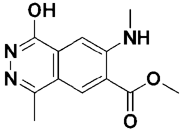

Dimethyl 2-acetyl-5-(methylamino)terephthalate (30 g, 113.10 mmol) obtained in [Step-4] and hydrazine monohydrate (7.3 mL, 147.03 mmol) were dissolved in ethanol (385 mL), and the mixture was refluxed with stirring at 95°C for 1 hour. Upon completion of the reaction, the reaction solution was cooled to room temperature to obtain a solid product. The solid was collected by filtration under reduced pressure and washed with ethanol to obtain the title compound (26 g) in a yield of 93%.

¹H-NMR (300 MHz, DMSO-*d*₆): δ 12.16 (s, 1H), 8.26 (s, 1H), 7.92 (d, 1H), 7.29 (s, 1H), 3.91 (s, 3H), 2.96 (d, 3H), 2.42 (s, 3H).

### [Step-6] Preparation of 1-hydroxy-4-methyl-7-(methylamino)phthalazine-6-carboxylic acid

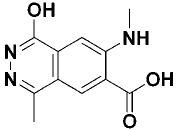

Methyl 1-hydroxy-4-methyl-7-(methylamino)phthalazine-6-carboxylate (10.0 g, 40.44 mmol) obtained in [Step-5] and sodium hydroxide (16.2 g, 404.45 mmol) were dissolved in a solution (120 mL) of tetrahydrofuran:methanol:water = 2:1:1, and the mixture was stirred at room temperature for 2 hours. Upon completion of the reaction, the mixture was concentrated under reduced pressure, and the residue was acidified at low temperature with a 6 N hydrochloric acid solution until pH 2. The obtained solid was collected by filtration under reduced pressure and washed with water. The solid was dried in an oven at 55°C to obtain the title compound (9.5 g) in a yield of 99%.

¹H-NMR (300 MHz, DMSO-*d*₆): δ 12.11 (s, 1H), 8.29 (s, 1H) 7.26 (s, 1H), 2.95 (s, 3H), 2.42 (s, 3H).

### [Step-7] Preparation of (1-hydroxy-4-methyl-7-(methylamino)phthalazin-6-yl)(morpholino)methanone

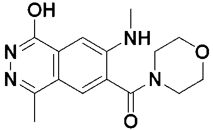

1-Hydroxy-4-methyl-7-(methylamino)phthalazine-6-carboxylic acid (9.5 g, 40.73 mmol) obtained in [Step-6], morpholine (28.4 mL, 325.86 mmol), HATU (46.9 g, 122.20 mmol), and N,N-diisopropylethylamine (57.3 mL, 325.86 mmol) were dissolved in DMF (100 mL), and the mixture was stirred at room temperature for 16 hours. Upon completion of the reaction, water was added, and the mixture was extracted with ethyl acetate. The organic layer was dried over anhydrous sodium sulfate and concentrated under reduced pressure. The residue was purified by column chromatography (dichloromethane:methanol = 20:1 (v/v)) to obtain the title compound (7.3 g) in a yield of 59%.

¹H-NMR (300 MHz, DMSO-*d*₆): δ 12.11 (s, 1H), 7.55 (s, 1H), 7.17 (s, 1H), 6.22-6.17 (m, 1H), 3.77-3.45 (m, 6H), 3.28-3.21 (m, 2H), 2.84-2.83 (m, 3H), 2.40 (s, 3H).

### [Step-8] Preparation of 4-methyl-7-(methylamino)-6-(morpholinomethyl)phthalazin-1-ol

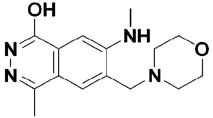

(1-Hydroxy-4-methyl-7-(methylamino)phthalazin-6-yl)(morpholino)methanone (4.0 g, 13.23 mmol) obtained in [Step-7] was dissolved in tetrahydrofuran at 0°C, and borane dimethyl sulfide (6.27 mL, 66.15 mmol) was slowly added dropwise. After completion of the addition, the mixture was stirred at room temperature for 16 hours. Upon completion of the reaction, water was slowly added dropwise at low temperature. The mixture was extracted with a mixed solvent of dichloromethane and methanol, dried over anhydrous sodium sulfate, and concentrated under reduced pressure. The residue was purified by column chromatography (dichloromethane:methanol = 25:1 to 10:1 (v/v)) to obtain the title compound (1.8 g) in a yield of 47%.

¹H-NMR (300 MHz, DMSO-*d*₆): δ 12.02 (s, 1H), 7.56 (s, 1H), 7.12 (s, 1H), 6.85-6.80 (m, 1H), 3.61-3.57 (m, 6H), 2.90-2.88 (m, 3H), 2.40-2.36 (m, 7H).

### [Step-9] Preparation of 4-chloro-N,1-dimethyl-7-(morpholinomethyl)phthalazin-6-amine

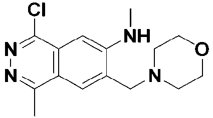

4-Methyl-7-(methylamino)-6-(morpholinomethyl)phthalazin-1-ol (1.2 g, 4.16 mmol) obtained in [Step-8] was dissolved in phosphoryl chloride (13 mL), and the mixture was refluxed with stirring at 110°C for 1.5 hours. Upon completion of the reaction, the reaction solution was cooled to room temperature and concentrated under reduced pressure. The residue was dissolved in dichloromethane and neutralized at 0°C by dropwise addition to an aqueous sodium bicarbonate solution. The mixture was extracted with a mixed solvent of dichloromethane and acetone, dried over anhydrous sodium sulfate, and concentrated under reduced pressure. The residue was purified by column chromatography (dichloromethane:methanol = 20:1 (v/v)) to obtain the title compound (509 mg) in a yield of 40%.

¹H-NMR (300 MHz, CDCl₃): δ 7.62 (s, 1H), 6.99 (s, 1H), 3.73-3.70 (m, 6H), 3.03-3.02 (m, 3H), 2.84 (s, 3H), 2.48-2.45 (m, 4H).

### [Step-10] Preparation of (R)-2-methyl-3-(1-((4-methyl-7-(methylamino)-6-(morpholinomethyl)phthalazin-1-yl)amino)ethyl)benzonitrile

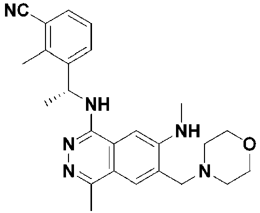

4-Chloro-N,1-dimethyl-7-(morpholinomethyl)phthalazin-6-amine (50 mg, 0.16 mmol) obtained in [Step-9], (R)-3-(1-aminoethyl)-2-methylbenzonitrile 2,2,2-trifluoroacetate (67 mg, 0.24 mmol) obtained in [Step-14] of Example 1, magnesium chloride (47 mg, 0.49 mmol), and N,N-diisopropylethylamine (0.17 mL, 0.98 mmol) were dissolved in 2-butanol (1 mL), and the mixture was refluxed with stirring at 130°C for 48 hours. Upon completion of the reaction, the mixture was cooled to room temperature, water was added, and the mixture was extracted with ethyl acetate. The organic layer was dried over anhydrous sodium sulfate and concentrated under reduced pressure. The residue was purified by column chromatography (dichloromethane:methanol = 20:1 (v/v)) to obtain the title compound (32 mg) in a yield of 33%.
¹H-NMR (300 MHz, DMSO-*d*₆): δ 7.78-7.75 (m, 1H), 7.59-7.57 (m, 2H), 7.31-7.23 (m, 2H), 7.16 (s, 1H), 6.83-6.78 (m, 1H), 5.58-5.53 (m, 1H), 3.63-3.57 (m, 6H), 3.00-2.98 (m, 3H), 2.67 (s, 3H), 2.48 (s, 3H), 2.38-2.35 (m, 4H), 1.55-1.53 (m, 3H).
MS (ESI+, m/z): 431.3 [M+H]⁺

### Example 30: (R)-3-(1-((7-(cyclopentylamino)-4-methyl-6-(morpholinomethyl)phthalazin-1-yl)amino)ethyl)-2-methylbenzonitrile

### [Step-1] Preparation of dimethyl 2-(cyclopentylamino)terephthalate

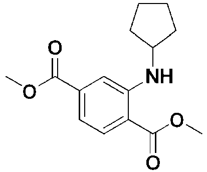

Dimethyl 2-aminoterephthalate (26 g, 124.28 mmol), cyclopentanone (27.5 mL, 310.71 mmol), sodium triacetoxyborohydride (60 g, 283.10 mmol), and acetic acid (71 mL, 1242.80 mmol) were dissolved in dichloromethane (390 mL) at 25°C, and the mixture was refluxed with stirring at room temperature for 48 hours. Upon completion of the reaction, water was added, and the mixture was extracted with dichloromethane. The organic layer was dried over anhydrous sodium sulfate and concentrated under reduced pressure. The residue was purified by column chromatography (hexane:ethyl acetate = 50:1 to 20:1 (v/v)) to obtain the title compound (9.6 g) in a yield of 28%.

¹H-NMR (300 MHz, DMSO-*d*₆): δ 7.92 (d, 1H), 7.77 (d, 1H), 7.31 (s, 1H), 7.13 (m, H), 3.94 (m, 1H), 3.86 (s, 3H), 3.82 (s, 3H), 2.03 (m, 2H), 1.70 (m, 4H) 1.64 (m, 2H).

### [Step-2] Preparation of dimethyl 2-bromo-5-(cyclopentylamino)terephthalate

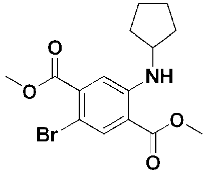

Dimethyl 2-(cyclopentylamino)terephthalate (9.6 g, 34.62 mmol) obtained in [Step-1] and N-bromosuccinimide (6.16 g, 34.62 mmol) were dissolved in acetonitrile (290 mL), and the mixture was refluxed with stirring at room temperature for 16 hours. Upon completion of the reaction, water was added, and the mixture was extracted with dichloromethane. The organic layer was dried over anhydrous sodium sulfate and concentrated under reduced pressure. The residue was purified by column chromatography (hexane:ethyl acetate = 50:1 to 25:1 (v/v)) to obtain the title compound (12 g) in a yield of 97%.

¹H-NMR (300 MHz, DMSO-*d*₆): δ 7.97 (s, 1H), 7.75 (d, 1H), 7.09 (s, 1H), 3.92 (m, 1H), 3.86 (s, 3H), 3.83 (s, 3H), 2.03 (m, 2H), 1.66 (m, 4H) 1.62 (m, 2H).

### [Step-3] Preparation of dimethyl 2-(1-butoxyvinyl)-5-(cyclopentylamino)terephthalate

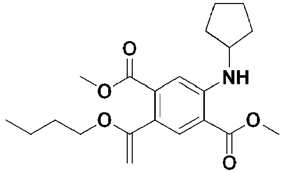

Dimethyl 2-bromo-5-(cyclopentylamino)terephthalate (12 g, 33.69 mmol) obtained in [Step-2], butyl vinyl ether (21.63 mL, 168.44 mmol), Pd(OAc)2 (756 mg, 3.37 mmol), triphenylphosphine (1.77 g, 6.73 mmol), and triethylamine (5.63 mL, 40.42 mmol) were dissolved in acetonitrile (120 mL), and the mixture was refluxed with stirring at 100°C for 16 hours. Upon completion of the reaction, the mixture was cooled to room temperature, filtered through a Celite-packed filter, and washed with dichloromethane. The filtrate was concentrated under reduced pressure, and the next reaction was performed without further purification.

### [Step-4] Preparation of dimethyl 2-acetyl-5-(cyclopentylamino)terephthalate

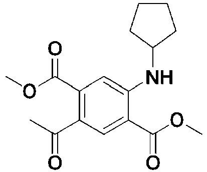

Dimethyl 2-(1-butoxyvinyl)-5-(cyclopentylamino)terephthalate (12.65 g, 33.69 mmol) obtained in [Step-3] and 6 N hydrochloric acid (170 mL, 269.49 mmol) were dissolved in tetrahydrofuran (130 mL), and the mixture was refluxed with stirring at room temperature for 2 hours. Upon completion of the reaction, the mixture was concentrated under reduced pressure, water was added to the residue, and the mixture was extracted with ethyl acetate. The organic layer was washed with a saturated aqueous sodium bicarbonate solution and an aqueous sodium chloride solution, dried over anhydrous sodium sulfate, and concentrated under reduced pressure. The residue was purified by column chromatography (hexane:ethyl acetate = 20:1 to 5:1 (v/v)) to obtain the title compound (10 g) in a yield of 93%.

¹H-NMR (300 MHz, DMSO-*d*₆): δ 8.37 (m, 2H), 6.86 (s, 1H), 4.04 (m, 1H), 3.87 (s, 3H), 3.77 (s, 3H), 2.49 (s, 3H), 2.05 (m, 2H), 1.67 (m, 4H) 1.62 (m, 2H).

### [Step-5] Preparation of methyl 7-(cyclopentylamino)-1-hydroxy-4-methylphthalazine-6-carboxylate

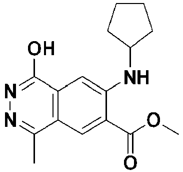

Dimethyl 2-acetyl-5-(cyclopentylamino)terephthalate (10 g, 31.31 mmol) obtained in [Step-4] and hydrazine monohydrate (1.53 mL, 31.31 mmol) were dissolved in ethanol (120 mL), and the mixture was refluxed with stirring at 100°C for 1.5 hours. Upon completion of the reaction, the reaction solution was cooled to room temperature to obtain a solid product. The solid was collected by filtration under reduced pressure and washed with ethanol to obtain the title compound (8.93 g) in a yield of 94.6%.

¹H-NMR (300 MHz, DMSO-*d*₆): δ 12.17 (s, 1H), 8.30 (s, 1H), 8.04 (d, 1H), 7.38 (s, 1H), 4.02 (m, 1H), 3.91 (s, 3H), 2.42 (s, 3H), 2.07 (m, 2H), 1.72 (m, 4H), 1.56 (m, 2H).

### [Step-6] Preparation of 7-(cyclopentylamino)-1-hydroxy-4-methylphthalazine-6-carboxylic acid

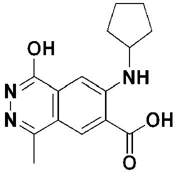

Methyl 7-(cyclopentylamino)-1-hydroxy-4-methylphthalazine-6-carboxylate (8.93 g, 29.63 mmol) obtained in [Step-5] and sodium hydroxide (14.5 g, 362.50 mmol) were dissolved in a solution (200 mL) of tetrahydrofuran:methanol:water = 2:1:1, and the mixture was refluxed with stirring at room temperature for 1 hour. Upon completion of the reaction, the mixture was concentrated under reduced pressure, and the residue was acidified at low temperature with a 6 N hydrochloric acid solution until pH 2. The obtained solid was collected by filtration under reduced pressure and washed with water. The solid was dried in an oven at 50°C to obtain the title compound (7 g) in a yield of 82%.

¹H-NMR (300 MHz, DMSO-*d*₆): δ 12.14 (s, 1H), 8.31 (s, 1H), 7.35 (s, 1H), 4.00 (m, 1H), 2.42 (s, 3H), 2.07 (m, 2H), 1.72 (m, 4H), 1.55 (m, 2H).

### [Step-7] Preparation of (7-(cyclopentylamino)-1-hydroxy-4-methylphthalazin-6-yl)(morpholino)methanone

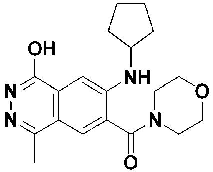

7-(Cyclopentylamino)-1-hydroxy-4-methylphthalazine-6-carboxylic acid (3.5 g, 12.18 mmol) obtained in [Step-6], morpholine (8.5 mL, 97.45 mmol), HATU (14 g, 36.55 mmol), and N,N-diisopropylethylamine (17.15 mL, 97.45 mmol) were dissolved in DMF (13 mL), and the mixture was stirred at room temperature for 5 hours. Upon completion of the reaction, water was added, and the mixture was extracted with ethyl acetate. The organic layer was dried over anhydrous sodium sulfate and concentrated under reduced pressure. The residue was purified by column chromatography (hexane:ethyl acetate = 1:1 (v/v)) to obtain the title compound (3.36 g) in a yield of 77%.

¹H-NMR (300 MHz, DMSO-*d*₆): δ 12.13 (s, 1H), 7.58 (s, 1H), 7.31 (s, 1H), 5.86 (m, 1H), 3.65 (m, 1H), 3.63-3.16 (m, 8H), 2.40 (s, 3H), 2.01 (m, 2H), 1.69-1.63 (m, 6H).

### [Step-8] Preparation of 7-(cyclopentylamino)-4-methyl-6-(morpholinomethyl)phthalazin-1-ol

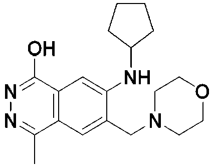

(7-(Cyclopentylamino)-1-hydroxy-4-methylphthalazin-6-yl)(morpholino)methanone (3.26 g, 9.15 mmol) obtained in [Step-7] was dissolved in tetrahydrofuran (65 mL), and borane dimethyl sulfide (1.38 mL, 13.72 mmol) was slowly added dropwise. After completion of the addition, the mixture was refluxed at room temperature for 2 hours. Upon completion of the reaction, water was slowly added dropwise, and the mixture was extracted with a mixed solvent of dichloromethane and methanol. The organic layer was dried over anhydrous sodium sulfate and concentrated under reduced pressure. The residue was purified by column chromatography (hexane:ethyl acetate = 1:1 to 9:1 (v/v)) to obtain the title compound (1 g) in a yield of 32%.

¹H-NMR (300 MHz, DMSO-*d*₆): δ 12.03 (s, 1H), 7.55 (s, 1H), 7.29 (m, 1H), 7.21 (s, 1H), 3.94 (m, 1H), 3.67 (s, 2H), 3.41 (m, 4H), 2.40 (s, 3H), 2.38 (m, 4H), 2.09 (m, 2H), 1.71 (m, 4H), 1.67 (m, 2H).

### [Step-9] Preparation of 4-chloro-N-cyclopentyl-1-methyl-7-(morpholinomethyl)phthalazin-6-amine

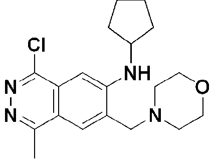

7-(Cyclopentylamino)-4-methyl-6-(morpholinomethyl)phthalazin-1-ol (810 mg, 2.81 mmol) obtained in [Step-8] was dissolved in phosphoryl chloride (8.5 mL), and the mixture was refluxed with stirring at 110°C for 1 hour. Upon completion of the reaction, the reaction solution was cooled to room temperature and concentrated under reduced pressure. The residue was dissolved in dichloromethane/acetone and neutralized at 0°C by dropwise addition to an aqueous sodium bicarbonate solution. The mixture was extracted with dichloromethane, dried over anhydrous sodium sulfate, and concentrated under reduced pressure. The residue was purified by column chromatography (100% ethyl acetate) to obtain the title compound (850 mg) in a yield of 98.6%.

¹H-NMR (300 MHz, DMSO-*d*₆): δ 7.91 (s, 1H), 7.81 (m, 1H), 6.90 (s, 1H), 4.02 (m, 1H), 3.77 (s, 2H), 3.59 (m, 4H), 2.75 (s, 3H), 2.40 (m, 4H), 2.09 (m, 2H), 1.73 (m, 4H), 1.61 (m, 2H).

### [Step-10] Preparation of (R)-3-(1-((7-(cyclopentylamino)-4-methyl-6-(morpholinomethyl)phthalazin-1-yl)amino)ethyl)-2-methylbenzonitrile

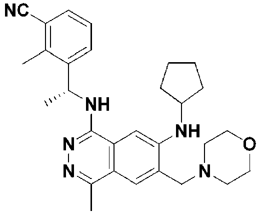

4-Chloro-N-cyclopentyl-1-methyl-7-(morpholinomethyl)phthalazin-6-amine (60 mg, 0.17 mmol) obtained in [Step-9], (R)-3-(1-aminoethyl)-2-methylbenzonitrile 2,2,2-trifluoroacetate (91 mg, 0.33 mmol) obtained in [Step-14] of Example 1, magnesium chloride (47 mg, 0.50 mmol), and N,N-diisopropylethylamine (0.17 mL, 1 mmol) were dissolved in 2-butanol (1 mL), and the mixture was refluxed with stirring at 120°C for 48 hours. Upon completion of the reaction, the mixture was cooled to room temperature, water was added, and the mixture was extracted with ethyl acetate. The organic layer was dried over anhydrous sodium sulfate and concentrated under reduced pressure. The residue was purified by column chromatography (dichloromethane:methanol = 30:1 to 10:1 (v/v)) to obtain the title compound (18 mg) in a yield of 22%.
¹H-NMR (300 MHz, DMSO-*d*₆): δ 7.77 (d, 1H), 7.60 (m, 2H), 7.32-7.20 (m, 4H), 5.56 (m, 1H), 4.21 (m, 1H), 3.66 (s, 2H), 3.42 (m, 4H), 2.67 (s, 3H), 2.48 (m, 3H), 2.37 (m, 4H), 2.13 (m, 2H), 1.77 (m, 4H), 1.56 (m, 5H).
MS (ESI+, m/z): 485.3 [M+H]+

### Example 31: (R)-3-(1-((7-(cyclopentylamino)-6-((4-cyclopropylpiperazin-1-yl)methyl)-4-methylphthalazin-1-yl)amino)ethyl)-2-methylbenzonitrile

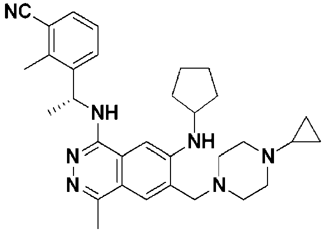

The procedure of Example 30 was repeated, except that 1-cyclopropylpiperazine (8.9 g, 69.60 mmol) was used instead of morpholine in [Step-7] of Example 30, to obtain the title compound (13 mg) in a yield of 3%.
¹H-NMR (300 MHz, DMSO-*d₆*): δ 7.76 (m, 1H), 7.62 (m, 3H), 7.43 (m, 1H), 7.34 (m, 2H), 5.56 (m, 1H), 4.27 (m, 1H), 3.66 (s, 2H), 2.67 (s, 3H), 2.54 (m, 5H), 2.34 (m, 3H), 2.18 (m, 2H), 1.74 (m, 10H), 0.40 (m, 4H).
MS (ESI+, m/z): 524.4 [M+H]⁺

### Example 32: (R)-3-(1-((7-methoxy-4-methyl-6-(morpholine-4-carbonyl)phthalazin-1-yl)amino)ethyl)-2-methylbenzonitrile

### [Step-1] Preparation of 4-bromo-2,5-dimethylphenol

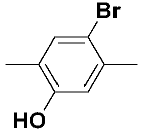

2,5-Dimethylphenol (15 g, 122.78 mmol) and tetrabutylammonium tribromide (59 g, 122.78 mmol) were dissolved in chloroform (450 mL)/water (450 mL) at 25°C, and the mixture was refluxed with stirring at room temperature for 3 hours. Upon completion of the reaction, water was added, and the mixture was extracted with chloroform. The organic layer was dried over anhydrous sodium sulfate and concentrated under reduced pressure. The resulting residue was purified by column chromatography (hexane:ethyl acetate = 9:1 (v/v)) to obtain the title compound (16.2 g) in a yield of 66%.

¹H-NMR (300 MHz, DMSO-*d*₆): δ 9.44 (s, 1H), 7.26 (s, 1H), 6.73 (s, 1H), 2.06 (s, 3H), 1.99 (s, 3H).

### [Step-2] Preparation of 1-bromo-4-methoxy-2,5-dimethylbenzene

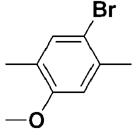

4-Bromo-2,5-dimethylphenol (16.2 g, 80.57 mmol) obtained in [Step-1], methyl iodide (10 mL, 161.15 mmol), and potassium hydroxide (18 g, 322.29 mmol) were dissolved in DMSO (120 mL), and the mixture was refluxed with stirring at room temperature for 1 hour. Upon completion of the reaction, water was added, and the mixture was extracted with ethyl acetate. The organic layer was dried over anhydrous sodium sulfate and concentrated under reduced pressure, and the next reaction was performed without further purification.

### [Step-3] Preparation of 2-bromo-5-methoxyterephthalic acid

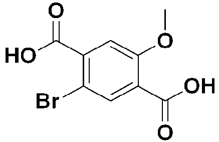

1-Bromo-4-methoxy-2,5-dimethylbenzene (17.33 g, 80.57 mmol) obtained in [Step-2] was dissolved in water (255 mL). Potassium permanganate (56 g, 354.52 mmol) was slowly added dropwise at room temperature, and the mixture was refluxed with stirring at 120°C for 16 hours. Upon completion of the reaction, the mixture was cooled to room temperature and filtered. To the filtrate, 2 N hydrochloric acid (200 mL) was added, and the mixture was stirred at room temperature for 30 minutes, followed by filtration. The obtained solid was washed with water, and the next reaction was performed without further purification.

### [Step-4] Preparation of diethyl 2-bromo-5-methoxyterephthalate

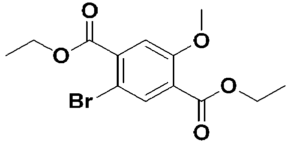

2-Bromo-5-methoxyterephthalic acid (5 g, 18.18 mmol) obtained in [Step-3] and sulfuric acid (3.2 mL, 18.18 mmol) were dissolved in ethanol (160 mL), and the mixture was refluxed with stirring at 100°C for 6 hours. Upon completion of the reaction, water was added, and the mixture was extracted with dichloromethane. The organic layer was dried over anhydrous sodium sulfate and concentrated under reduced pressure. The residue was purified by column chromatography (hexane:ethyl acetate = 20:1 (v/v)) to obtain the title compound (3.2 g) in a yield of 53%.

¹H-NMR (300 MHz, DMSO-*d*₆): δ 7.87 (s, 1H), 7.44 (s, 1H), 4.37-4.27 (m, 4H), 3.86 (s, 3H), 1.37-1.27 (m, 6H).

### [Step-5] Preparation of diethyl 2-(1-butoxyvinyl)-5-methoxyterephthalate

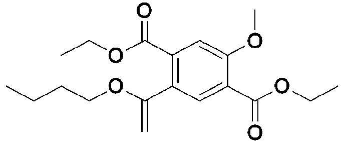

The procedure of [Step-3] of Example 1 was repeated, except that diethyl 2-bromo-5-methoxyterephthalate (3.2 g, 9.66 mmol) obtained in [Step-4] was used instead of dimethyl 2-bromo-5-(methylamino)terephthalate obtained in [Step-2] of Example 1, and the next reaction was performed without further purification.

### [Step-6] Preparation of diethyl 2-acetyl-5-methoxyterephthalate

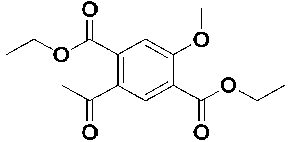

The procedure of [Step-4] of Example 1 was repeated, except that diethyl 2-(1-butoxyvinyl)-5-methoxyterephthalate (3.4 g, 9.66 mmol) obtained in [Step-5] was used instead of dimethyl 2-(1-butoxyvinyl)-5-(methylamino)terephthalate obtained in [Step-3] of Example 1, to obtain the title compound (2.4 g) in a yield of 84%.

¹H-NMR (300 MHz, DMSO-*d*₆): δ 8.08 (s, 1H), 7.32 (s, 1H), 4.32-4.27 (m, 4H), 3.93 (s, 3H), 2.53 (s, 3H), 1.33-1.26 (m, 6H).

### [Step-7] Preparation of ethyl 1-hydroxy-7-methoxy-4-methylphthalazine-6-carboxylate

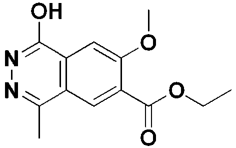

The procedure of [Step-5] of Example 1 was repeated, except that diethyl 2-acetyl-5-methoxyterephthalate (2.2 g, 7.48 mmol) obtained in [Step-6] was used instead of dimethyl 2-acetyl-5-(methylamino)terephthalate obtained in [Step-4] of Example 1, to obtain the title compound (1.8 g) in a yield of 92%.

¹H-NMR (300 MHz, DMSO-*d*₆): δ 12.48 (s, 1H), 8.09 (s, 1H), 7.77 (s, 1H), 4.39-4.32 (m, 2H), 4.00 (s, 3H), 2.52 (s, 3H), 1.36-1.31 (m, 3H).

### [Step-8] Preparation of ethyl 1-chloro-7-methoxy-4-methylphthalazine-6-carboxylate

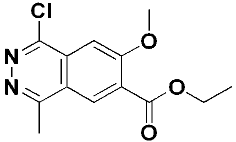

The procedure of [Step-6] of Example 1 was repeated, except that ethyl 1-hydroxy-7-methoxy-4-methylphthalazine-6-carboxylate (290 mg, 1.11 mmol) obtained in [Step-7] was used instead of methyl 1-hydroxy-4-methyl-7-(methylamino)phthalazine-6-carboxylate obtained in [Step-5] of Example 1, to obtain the title compound (200 mg) in a yield of 64%.

¹H-NMR (300 MHz, DMSO-*d₆*): δ 8.45 (s, 1H), 7.59 (s, 1H), 4.42-4.35 (m, 2H), 4.08 (s, 3H), 2.89 (s, 3H), 1.37-1.33 (m, 3H).

### [Step-9] Preparation of ethyl (R)-1-((1-(3-cyano-2-methylphenyl)ethyl)amino)-7-methoxy-4-methylphthalazine-6-carboxylate

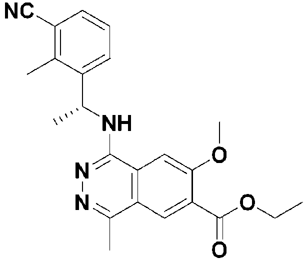

The procedure of [Step-15] of Example 1 was repeated, except that ethyl 1-chloro-7-methoxy-4-methylphthalazine-6-carboxylate (180 mg, 0.64 mmol) obtained in [Step-8] was used instead of methyl 1-chloro-4-methyl-7-(methylamino)phthalazine-6-carboxylate obtained in [Step-6] of Example 1, to obtain the title compound (104 mg) in a yield of 40%.

¹H-NMR (300 MHz, DMSO-*d₆*): δ 8.14 (s, 1H), 7.94 (s, 1H), 7.77 (d, 1H), 7.67-7.60 (m, 3H), 7.34-7.29 (m, 1H), 5.61-5.56 (m, 1H), 4.38-4.31 (m, 2H), 4.06 (s, 3H), 2.68 (s, 3H), 2.60 (s, 3H), 1.59 (d, 3H), 1.36 (m, 3H).

### [Step-10] Preparation of (R)-1-((1-(3-cyano-2-methylphenyl)ethyl)amino)-7-methoxy-4-methylphthalazine-6-carboxylic acid

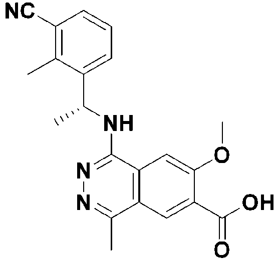

The procedure of [Step-16] of Example 1 was repeated, except that ethyl (R)-1-((1-(3-cyano-2-methylphenyl)ethyl)amino)-7-methoxy-4-methylphthalazine-6-carboxylate (104 mg, 0.26 mmol) obtained in [Step-9] was used instead of sec-butyl 1(((R)-1-(3-cyano-2-methylphenyl)ethyl)amino-4-methyl-7-(methylamino)phthalazine-6-carboxylate obtained in [Step-15] of Example 1, to obtain the title compound (88 mg) in a yield of 91%.

¹H-NMR (300 MHz, DMSO-*d₆*): δ 9.22 (s, 1H), 8.50-8.45 (m, 2H), 7.93 (m, 1H), 7.66-7.64 (m, 1H), 7.38-7.33 (m, 1H), 5.45 (m, 1H), 3.99 (s, 3H), 2.81 (s, 3H), 2.69 (s, 3H), 1.68 (m, 3H).

### [Step-11] Preparation of (R)-3-(1-((7-methoxy-4-methyl-6-(morpholine-4-carbonyl)phthalazin-1-yl)amino)ethyl)-2-methylbenzonitrile

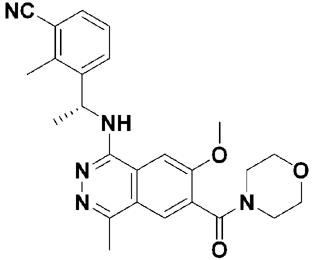

The procedure of [Step-17] of Example 1 was repeated, except that (R)-1-((1-(3-cyano-2-methylphenyl)ethyl)amino)-7-methoxy-4-methylphthalazine-6-carboxylic acid (88 mg, 0.23 mmol) obtained in [Step-10] was used instead of (R)-1-((1-(3-cyano-2-methylphenyl)ethyl)amino)-4-methyl-7-(methylamino)phthalazine-6-carboxylic acid obtained in [Step-16] of Example 1, to obtain the title compound (80 mg) in a yield of 77%.
¹H-NMR (300 MHz, DMSO-*d*₆): δ 7.93 (s, 1H), 7.83 (s, 1H), 7.78 (m, 1H), 7.76 (m, 2H), 7.34-7.29 (m, 1H), 5.60 (m, 1H), 4.07 (s, 3H), 3.72-3.67 (m, 4H), 3.65-3.62 (m, 2H), 3.12 (m, 2H), 2.68 (s, 3H), 2.58 (s, 3H), 1.59 (m, 3H).
MS (ESI+, m/z): 446.2 [M+H]⁺

### Example 33: (R)-3-(1-((7-cyclopentylamino)-4-methyl-6-(morpholine-4-carbonyl)phthalazin-1-yl)amino)ethyl)-2-methylbenzonitrile

### [Step-1] Preparation of dimethyl 2-(cyclopentylamino)terephthalate

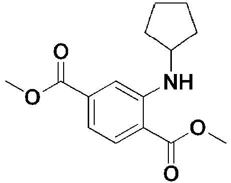

Dimethyl aminoterephthalate (2.8 g, 13.38 mmol), cyclopentanone (2.6 mL, 29.45 mmol), sodium triacetoxyborohydride (5.67 g, 26.77 mmol), and acetic acid (6.5 mL, 114.24 mmol) were dissolved in dichloromethane (40 mL), and the mixture was refluxed with stirring at room temperature for 48 hours. Upon completion of the reaction, a saturated aqueous sodium bicarbonate solution was added, and the mixture was extracted with dichloromethane. The organic layer was dried over anhydrous sodium sulfate and concentrated under reduced pressure. The residue was purified by column chromatography (hexane:ethyl acetate = 50:1 (v/v)) to obtain the title compound (1 g) in a yield of 27%.

¹H-NMR (300 MHz, DMSO-*d₆*): δ 7.92 (d, 1H), 7.77 (m, 1H), 7.31 (s, 1H), 7.13-7.09 (m, 1H), 3.94-3.92 (m, 1H), 3.86-3.83 (m, 6H), 2.04-1.99 (m, 2H), 1.70-1.64 (m, 4H), 1.51-1.48 (m, 2H).

### [Step-2] Preparation of dimethyl 2-bromo-5-(cyclopentylamino)terephthalate

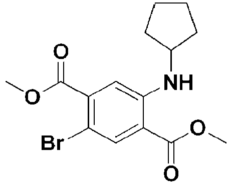

Dimethyl 2-(cyclopentylamino)terephthalate (1 g, 3.61 mmol) obtained in [Step-1] and N-bromosuccinimide (642 mg, 3.61 mmol) were dissolved in acetonitrile (30 mL), and the mixture was refluxed with stirring at room temperature for 16 hours. Upon completion of the reaction, water was added, and the mixture was extracted with dichloromethane. The organic layer was dried over anhydrous sodium sulfate and concentrated under reduced pressure. The residue was purified by column chromatography (hexane:ethyl acetate = 50:1 (v/v)) to obtain the title compound (1.13 g) in a yield of 88%.

¹H-NMR (300 MHz, DMSO-*d₆*): δ 7.97 (s, 1H), 7.75 (m, 1H), 7.09 (s, 1H), 3.92-3.90 (m, 1H), 3.86 (s, 3H), 3.83 (s, 3H), 2.03-1.99 (m, 2H), 1.69-1.61 (m, 4H), 1.47-1.43 (m, 2H).

### [Step-3] Preparation of (R)-3-(1-((7-cyclopentylamino)-4-methyl-6-(morpholine-4-carbonyl)phthalazin-1-yl)amino)ethyl)-2-methylbenzonitrile

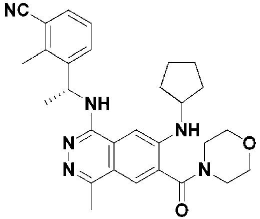

The procedure of Example 1 was repeated, except that dimethyl 2-bromo-5-(cyclopentylamino)terephthalate (1.13 g, 3.7 mmol) obtained in [Step-2] was used instead of dimethyl 2-bromo-5-(methylamino)terephthalate obtained in [Step-2] of Example 1, to obtain the title compound (132 mg) in a yield of 44%.
¹H-NMR (300 MHz, DMSO-*d*₆): δ 7.76 (m, 1H), 7.61-7.58 (m, 2H), 7.33-7.28 (m, 3H), 5.76-5.54 (m, 2H), 4.20 (m, 1H), 4.00-3.62 (m, 4H), 3.61-3.29 (m, 2H), 2.54 (s, 3H), 2.48 (m, 3H), 2.15-2.09 (m, 2H), 1.75-1.54 (m, 9H).
MS (ESI+, m/z): 499.3 [M+H]⁺

**[Table 1]**

| Compound Number | Structure | Name | MS[M+H]+ |
|---|---|---|---|
| | 1H-NMR spectrum (300 MHz) | | |
| 1 | 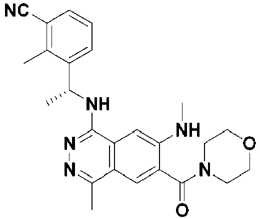 | (R)-2-methyl-3-(1-((4-methyl-7-(methylamino)-6-(morpholine-4-carbonyl)phthalazin-1-yl)amino)ethyl)benzonitrile | 445.2 |
| | 1H-NMR (300 MHz, DMSO-d6): δ 7.76 (d, 1H), 7.60 (m, 2H), 7.32 (m, 2H), 7.23 (s, 1H), 6.07 (d, 1H), 5.59 (m, 1H), 3.64 (m, 6H), 3.16 (m, 2H), 2.94 (d, 3H), 2.67 (s, 3H), 2.49 (s, 3H), 1.55 (d, 3H). | | |
| 2 | 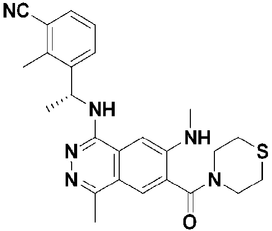 | (R)-2-methyl-3-(1-((4-methyl-7-(methylamino)-6-(thiomorpholine-4-carbonyl)phthalazin-1-yl)amino)ethyl)benzonitrile | 461.2 |
| | 1H-NMR (300 MHz, DMSO-d6): δ 7.78 (m, 1H), 7.60 (m, 2H), 7.35 (m, 2H), 7.21 (s, 1H), 6.02 (m, 1H), 5.61 (m, 1H), 3.92 (m, 2H), 3.45 (m, 2H), 2.94 (m, 3H), 2.77 (m, 2H), 2.67 (s, 3H), 2.61 (m, 2H), 2.49 (s, 3H), 1.56 (m, 3H). | | |
| 3 | 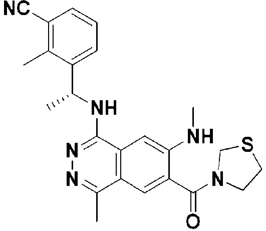 | (R)-2-methyl-3-(1-((4-methyl-7-(methylamino)-6-(thiazolidine-3-carbonyl)phthalazin-1-yl)amino)ethyl)benzonitrile | 447.2 |
| | 1H-NMR (300 MHz, DMSO-d6): δ 7.78 (m, 1H), 7.69 (s, 1H), 7.61 (m, 1H), 7.37 (m, 2H), 7.24 (s, 1H), 6.22 (m, 1H), 5.62 (m, 1H), 4.66 (m, 2H), 3.87 (m, 2H), 3.05 (m, 2H), 2.95 (m, 3H), 2.67 (s, 3H), 2.49 (s, 3H), 1.56 (m, 3H). | | |
| 4 | 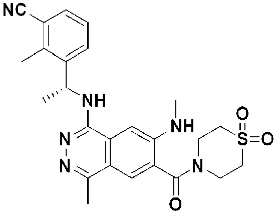 | (R)-3-(1-((6-(1,1-dioxothiomorpholine-4-carbonyl)-4-methyl-7-(methylamino)phthalazin-1-yl)amino)ethyl)-2-methylbenzonitrile | 493.2 |
| | 1H-NMR (300 MHz, DMSO-d6): δ 7.77 (m, 2H), 7.61 (m, 1H), 7.37 (m, 2H), 7.23 (s, 1H), 6.10 (m, 1H), 5.62 (m, 1H), 4.06 (m, 2H), 3.59 (m, 2H), 3.43 (m, 2H), 3.26 (m, 2H), 2.95 (m, 3H), 2.67 (s, 3H), 2.49 (s, 3H), 1.56 (m, 3H). | | |
| 5 | 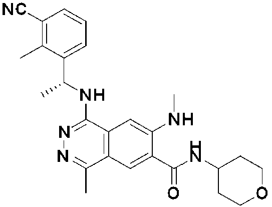 | (R)-1-((1-(3-cyano-2-methylphenyl)ethyl)amino)-4-methyl-7-(methylamino)-N-(tetrahydro-2H-pyran-4-yl)phthalazin-6-carboxamide | 459.2 |
| | 1H-NMR (300 MHz, DMSO-d6): δ 8.66-8.64 (m, 1H), 7.95 (s, 1H), 7.77-7.75 (m, 1H), 7.60-7.53 (m, 2H), 7.36-7.28 (m, 2H), 7.20 (s, 1H), 5.59-5.50 (m, 1H), 4.06-3.99 (m, 1H), 3.97-3.87 (m, 2H), 3.44-3.35 (m, 2H), 2.98-2.96 (m, 3H), 2.67 (s, 3H), 2.55 (s, 3H), 1.84-1.79 (m, 2H), 1.66-1.53 (m, 5H). | | |
| 6 | 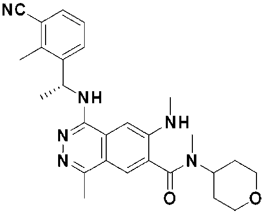 | (R)-1-((1-(3-cyano-2-methylphenyl)ethyl)amino)-N,4-dimethyl-7-(methylamino)-N-(tetrahydro-2H-pyran-4-yl)phthalazin-6-carboxamide | 473.3 |
| | 1H-NMR (300 MHz, DMSO-d6): δ 7.79-7.76 (m, 1H), 7.61-7.58 (m, 1H), 7.53 (s, 1H), 7.35-7.31 (m, 2H), 7.28-7.21 (m, 1H), 6.05-5.84 (m, 1H), 5.61- 5.52 (m, 1H), 4.04-3.82 (m, 3H), 3.79-3.37 (m, 2H), 2.93-2.91 (m, 3H), 2.67 (s, 3H), 2.47 (s, 6H), 1.85-1.80 (m, 3H), 1.78-1.74 (m, 4H). | | |
| 7 | 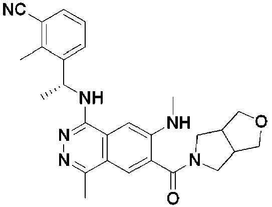 | 3-((1R)-1-((6-(hexahydro-1H-furo[3,4-c]pyrrole-5-carbonyl)-4-methyl-7-(methylamino)phthalazin-1-yl)amino)ethyl)-2-methylbenzonitrile | 471.3 |
| | 1H-NMR (300 MHz, DMSO-d6): δ 7.78 (m, 1H), 7.64 (m, 2H), 7.35 (m, 2H), 7.22 (s, 1H), 6.16 (m, 1H), 5.62 (m, 1H), 3.61 (m, 8H), 3.14 (m, 1H), 2.95 (m, 4H), 2.68 (s, 3H), 2.51 (s, 3H), 1.57 (m, 3H). | | |
| 8 | 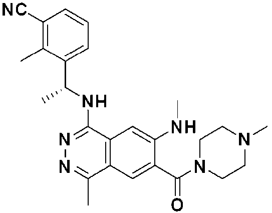 | (R)-2-methyl-3-(1-((4-methyl-7-(methylamino)-6-(4-methylpiperazin-1-carbonyl)phthalazin-1-yl)amino)ethyl)benzonitrile | 458.3 |
| | 1H-NMR (300 MHz, DMSO-d6): δ 7.77-7.74 (m, 1H), 7.60-7.56 (m, 2H), 7.39-7.28 (m, 2H), 7.22 (s, 1H), 6.08-6.02 (m, 1H), 5.60-5.51 (m, 1H), 3.75-3.61 (m, 2H), 3.24-3.19 (m, 2H), 2.94-2.92 (m, 3H), 2.67 (s, 3H), 2.48 (s, 3H), 2.42-2.35 (m, 2H), 2.33-2.23 (m, 5H), 1.56-1.54 (m, 3H). | | |
| 9 | 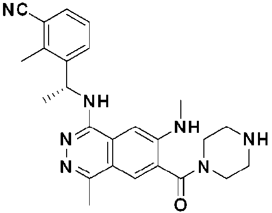 | (R)-2-methyl-3-(1-((4-methyl-7-(methylamino)-6-(piperazin-1-carbonyl)phthalazin-1-yl)amino)ethyl)benzonitrile | 444.3 |
| | 1H-NMR (300 MHz, DMSO-d6): δ 7.77-7.74 (m, 1H), 7.60-7.58 (m, 1H), 7.55 (s, 1H), 7.35-7.27 (m, 2H), 7.21 (s, 1H), 6.03-5.98 (m, 1H), 5.58-5.54 (m, 1H), 3.66-3.54 (m, 2H), 3.20-3.07 (m, 2H), 2.94-2.92 (m, 3H), 2.84-2.72 (m, 2H), 2.69-2.54 (m, 6H), 2.50 (s, 3H), 1.56-1.53 (m, 3H). | | |
| 10 | 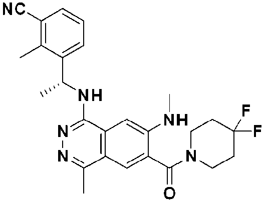 | (R)-3-(1-((6-(4,4-difluoropiperidin-1-carbonyl)-4-methyl-7-(methylamino)phthalazin-1-yl)amino)ethyl)-2-methylbenzonitrile | 479.2 |
| | 1H-NMR (300 MHz, DMSO-d6): δ 8.33 (s, 1H), 8.03 (s, 1H), 7.74-7.71 (m, 1H), 7.66-7.64 (m, 1H), 7.46 (s, 1H), 7.38-7.33 (m, 1H), 7.13-7.06 (m, 1H), 5.41-5.32 (m, 1H), 3.81-3.71 (m, 2H), 3.66-3.58 (m, 2H), 3.29-3.10 (m, 4H), 3.03-3.01 (m, 3H), 2.67 (s, 6H), 1.62-1.59 (m, 3H). | | |
| 11 | 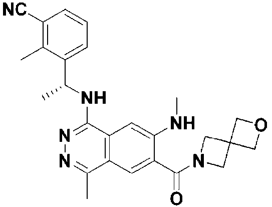 | (R)-2-methyl-3-(1-((4-methyl-7-(methylamino)-6-(2-oxa-6-azaspiro[3.3]heptane-6-carbonyl)phthalazin-1-yl)amino)ethyl)benzonitrile | 457.2 |
| | 1H-NMR (300 MHz, DMSO-d6): δ 7.78 (m, 1H), 7.64 (m, 2H), 7.35 (m, 2H), 7.22 (s, 1H), 6.16 (m, 1H), 5.62 (m, 1H), 3.61 (m, 8H), 3.14 (m, 1H), 2.95 (m, 4H), 2.68 (s, 3H), 2.51 (s, 3H), 1.57 (m, 3H). | | |
| 12 | 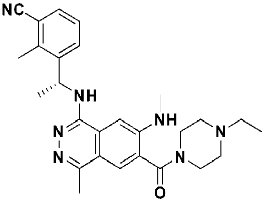 | (R)-3-(1-((6-(4-ethylpiperazin-1-carbonyl)-4-methyl-7-(methylamino)phthalazin-1-yl)amino)ethyl)-2-methylbenzonitrile | 472.3 |
| | 1H-NMR (300 MHz, DMSO-d6): δ 8.28 (s, 1H), 7.94 (s, 1H), 7.74-7.71 (m, 1H), 7.66-7.64 (m, 1H), 7.44 (s, 1H), 7.38-7.33 (m, 1H), 7.06-6.98 (m, 1H), 5.43-5.33 (m, 1H), 3.84-3.55 (m, 2H), 3.27-3.13 (m, 2H), 3.02-3.01 (m, 3H), 2.90-2.85 (m, 2H), 2.79-7.72 (m, 6H), 2.69-2.54(m, 2H), 2.51 (s, 3H), 1.61-1.59 (m, 3H), 1.08-1.05 (m, 3H). | | |
| 13 | 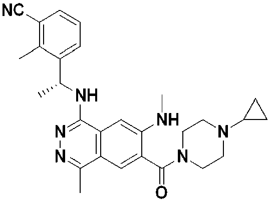 | (R)-3-(1-((6-(4-cyclopropylpiperazin-1-carbonyl)-4-methyl-7-(methylamino)phthalazin-1-yl)amino)ethyl)-2-methylbenzonitrile | 484.3 |
| | 1H-NMR (300 MHz, CDCl3): δ 7.70 (m, 1H), 7.64 (s, 1H), 7.51 (m, 1H), 7.24 (m, 1H), 6.60 (s, 1H), 5.77 (m, 1H), 5.55 (m, 1H), 4.96 (m, 1H), 3.74 (m, 4H), 3.00 (m, 3H), 2.74 (m, 10H), 1.71 (m, 4H), 0.51 (m, 4H). | | |
| 14 | 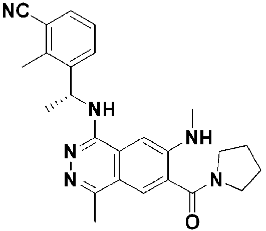 | (R)-2-methyl-3-(1-((4-methyl-7-(methylamino)-6-(pyrrolidin-1-carbonyl)phthalazin-1-yl)amino)ethyl)benzonitrile | 429.2 |
| | 1H-NMR (300 MHz, CDCl3): δ 7.78 (s, 1H), 7.70 (m, 1H), 7.52 (m, 1H), 7.25 (m, 1H), 6.57 (s, 1H), 6.14 (m, 1H), 5.77 (m, 1H), 4.93 (m, 1H), 3.74 (m, 2H), 3.51 (m, 2H), 2.99 (m, 3H), 2.75 (s, 3H), 2.69 (s, 3H), 2.00 (m, 4H), 1.66 (m, 3H). | | |
| 15 | 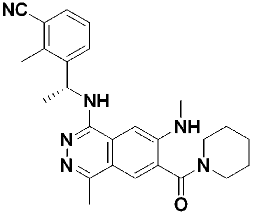 | (R)-2-methyl-3-(1-((4-methyl-7-(methylamino)-6-(piperidin-1-carbonyl)phthalazin-1-yl)amino)ethyl)benzonitrile | 443.3 |
| | 1H-NMR (300 MHz, CDCl3): δ 7.70 (m, 1H), 7.63 (s, 1H), 7.50 (m, 1H), 7.24 (m, 1H), 6.61 (s, 1H), 5.78 (m, 1H), 5.52 (m, 1H), 4.99 (m, 1H), 3.66 (m, 4H), 2.98 (m, 3H), 2.74 (s, 3H), 2.68 (s, 3H), 1.73 (m, 9H). | | |
| 16 | 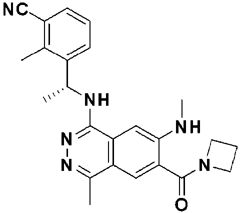 | (R)-3-(1-((6-(azetidin-1-carbonyl)-4-methyl-7-(methylamino)phthalazin-1-yl)amino)ethyl)-2-methylbenzonitrile | 415.2 |
| | 1H-NMR (300 MHz, DMSO-d6): δ 8.24 (s, 1H), 8.02 (s, 1H), 7.73-7.71 (m, 1H), 7.66-7.63 (m, 2H), 7.43 (s, 1H), 7.38-7.33 (m, 1H), 5.42-5.33 (m, 1H), 4.19-4.08 (m, 4H), 3.05-3.03 (m, 3H), 2.67-2.66 (m, 6H), 2.31-2.21 (m, 2H), 1.61-1.58 (m, 3H). | | |
| 17 | 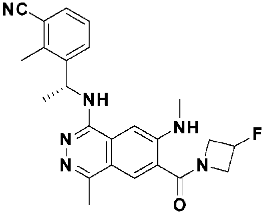 | (R)-3-(1-((6-(3-fluoroazetidin-1-carbonyl)-4-methyl-7-(methylamino)phthalazin-1-yl)amino)ethyl)-2-methylbenzonitrile | 433.2 |
| | 1H-NMR (300 MHz, DMSO-d6): δ 7.73 (s, 1H), 7.61-7.58 (m, 1H), 7.51-7.49 (m, 1H), 7.48-7.45 (m, 1H), 7.33-7.26 (m, 2H), 7.03-6.96 (m, 1H), 5.57-5.31 (m, 2H), 4.52-4.08 (m, 4H), 2.98-2.97(m, 3H), 2.66 (s, 3H), 2.53 (s, 3H), 1.56-1.54 (m, 3H). | | |
| 18 | 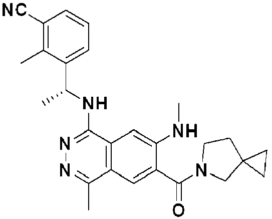 | (R)-2-methyl-3-(1-((4-methyl-7-(methylamino)-6-(5-azaspiro[2.4]heptane-5-carbonyl)phthalazin-1-yl)amino)ethyl)benzonitrile | 455.3 |
| | 1H-NMR (300 MHz, DMSO-d6): δ 7.76-7.71 (m, 2H), 7.61-7.59 (m, 1H), 7.54-7.49 (m, 1H), 7.34-7.24 (m, 2H), 6.44-6.38 (m, 1H), 5.57-5.48 (m, 1H), 3.71-3.67 (m, 1H), 3.46-3.39 (m, 2H), 3.19-3.11 (m, 1H), 2.97-2.94(m, 3H), 2.67-2.66 (m, 3H), 2.54-2.52 (m, 3H), 1.87-73 (m, 2H), 1.57-1.54 (m, 3H), 0.68-0.60 (m, 4H). | | |
| 19 | 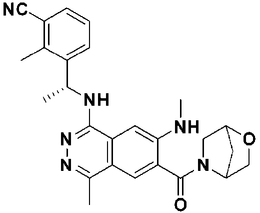 | 3-((1R)-1-((6-(2-oxa-5-azabicyclo[2.2.1]heptane-5-carbonyl)-4-methyl-7-(methylamino)phthalazin-1-yl)amino)ethyl)-2-methylbenzonitrile | 457.2 |
| | 1H-NMR (300 MHz, DMSO-d6): δ 7.77-7.72 (m, 2H), 7.61-7.51 (m, 2H), 7.34-7.26 (m, 2H), 6.40-6.37 (m, 1H), 5.55-5.51 (m, 1H), 4.90-4.4.14(m, 2H), 4.12-3.75 (m, 2H), 3.60-3.44 (m, 2H), 2.97-2.95 (m, 3H), 2.67 (s, 3H), 2.52 (s, 3H), 2.02-1.70 (m, 2H), 1.57-1.54 (m, 3H). | | |
| 20 | 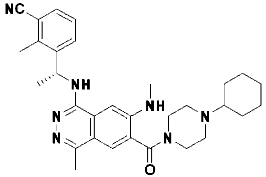 | (R)-3-(1-((6-(4-cyclohexylpiperazin-1-carbonyl)-4-methyl-7-(methylamino)phthalazin-1-yl)amino)ethyl)-2-methylbenzonitrile | 526.3 |
| | 1H-NMR (300 MHz, DMSO-d6): δ 7.78 (d, 1H), 7.61 (d, 1H), 7.54 (s, 1H), 7.35-7.28 (m, 2H), 7.21 (s, 1H), 6.00 (m, 1H), 5.59 (m, 1H), 3.65 (m, 1H), 2.94 (m, 2H), 2.89 (m, 3H), 2.73-2.40 (m, 10H), 2.25 (m, 4H), 1.74 (m, 4H), 1.17 (m, 4H). | | |
| 21 | 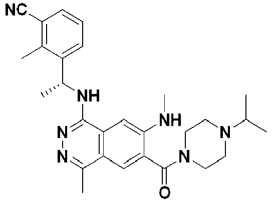 | (R)-3-(1-((6-(4-isopropylpiperazin-1-carbonyl)-4-methyl-7-(methylamino)phthalazin-1-yl)amino)ethyl)-2-methylbenzonitrile | 486.3 |
| | 1H-NMR (300 MHz, DMSO-d6): δ 7.78 (d, 1H), 7.61 (d, 1H), 7.55 (s, 1H), 7.35-7.28 (m, 2H), 7.21 (s, 1H), 6.02 (m, 1H), 5.57 (m, 1H), 3.66 (m, 1H), 3.16 (m, 2H), 2.94 (m, 3H), 2.71 (m, 4H), 2.52 (m, 4H), 2.27 (m, 2H), 1.56 (m, 3H), 0.95 (m, 6H). | | |
| 22 | 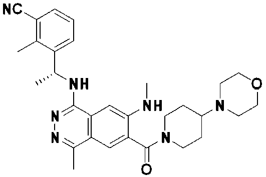 | (R)-2-methyl-3-(1-((4-methyl-7-(methylamino)-6-(4-morpholinopiperidin-1-carbonyl)phthalazin-1-yl)amino)ethyl)benzonitrile | 528.3 |
| | 1H-NMR (300 MHz, CDCl3): δ 7.70 (m, 1H), 7.62 (s, 1H), 7.51 (m, 1H), 7.22 (m, 1H), 6.61 (s, 1H), 5.79 (m, 1H), 5.53 (m, 1H), 3.77 (m, 4H), 2.99 (m, 5H), 2.75 (s, 3H), 2.68 (s, 3H), 2.59 (m, 7H), 1.98 (m, 2H), 1.67 (m, 3H), 1.48 (m, 2H). | | |
| 23 | 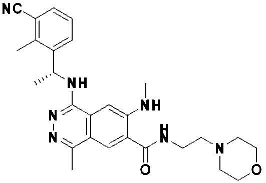 | (R)-1-((1-(3-cyano-2-methylphenyl)ethyl)amino)-4-methyl-7-(methylamino)-N-(2-morpholinoethyl)phthalazin-6-carboxamide | 488.3 |
| | 1H-NMR (300 MHz, MeOD-d4): δ 8.32 (s, 1H), 7.74-7.72 (m, 1H),7.55-7.52 (m, 1H), 7.46 (s, 1H), 7.31-7.26 (m, 1H), 5.49-5.42 (m, 1H), 3.73-3.70 (m, 4H), 3.61-3.56 (m, 2H), 3.13 (s, 3H), 2.75 (s, 6H), 2.67-2.63 (m, 2H), 2.59-2.56 (m, 4H), 1.67-1.65 (m, 3H). | | |
| 24 | 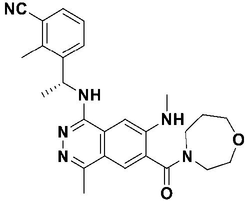 | (R)-2-methyl-3-(1-((4-methyl-7-(methylamino)-6-(1,4-oxazepane-4-carbonyl)phthalazin-1-yl)amino)ethyl)benzonitrile | 459.3 |
| | 1H-NMR (300 MHz, CDCl3): δ 7.70 (m, 2H), 7.51 (m, 1H), 7.25 (m, 1H), 6.62 (s, 1H), 5.80 (m, 1H), 5.42 (m, 1H), 5.00 (m, 1H), 3.93 (m, 5H), 3.54 (m, 3H), 3.00 (m, 3H), 2.74 (s, 3H), 2.69 (s, 3H), 2.09 (m, 1H), 1.84 (m, 1H), 1.67 (m, 3H). | | |
| 25 | 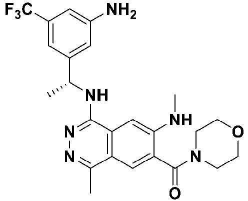 | (R)-(1-((1-(3-amino-5-(trifluoromethyl)phenyl)ethyl)ami no)-4-methyl-7-(methylamino)phthalazin-6-yl)(morpholino)methanone | 489.2 |
| | 1H-NMR (300 MHz, DMSO-d6): δ 7.61 (s, 1H), 7.20 (m, 2H), 6.85 (d, 2H), 6.67 (s, 1H), 6.04 (m, 1H), 5.40 (m, 3H), 3.64 (m, 6H), 3.16 (m, 2H), 2.93 (d, 3H), 2.49 (s, 3H), 1.55 (d, 3H). | | |
| 26 | 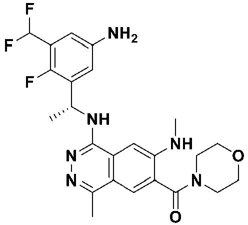 | (R)-(1-((1-(5-amino-3-(difluoromethyl)-2-fluorophenyl)ethyl)amino)-4-methyl-7-(methylamino)phthalazin-6-yl)(morpholino)methanone | 489.2 |
| | 1H-NMR (300 MHz, DMSO-d6): δ 7.63 (s, 1H), 7.29-6.89 (m, 2H), 6.69 (m, 1H), 6.57 (m, 1H), 6.12 (m, 1H), 5.55 (m, 1H), 5.10 (m, 2H), 3.69-3.40 (m 6H), 3.31-3.18 (m, 2H), 2.96 (m, 3H), 1.58 (m, 3H). | | |
| 27 | 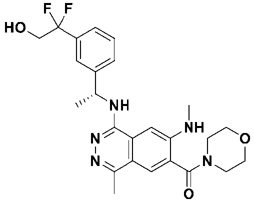 | (R)-(1-((1-(3-(1,1-difluoro-2-hydroxyethyl)phenyl)ethyl)amino) -4-methyl-7-(methylamino)phthalazin-6-yl)(morpholino)methanone | 486.2 |
| | 1H-NMR (300 MHz, DMSO-d6): δ 7.63-7.54 (m, 3H), 7.43-7.32 (m, 3H), 7.24 (s, 1H), 6.15 (m, 1H), 5.59-5.51 (m, 2H), 3.88-3.60 (m, 8H), 3.29-3.23 (m, 2H), 3.21 (m, 2H), 3.18 (m, 3H), 1.62 (d, 3H). | | |
| 28 | 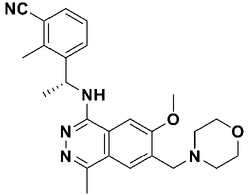 | (R)-3-(1-((7-methoxy-4-methyl-6-(morpholinomethyl)phthalazin-1-yl)amino)ethyl)-2-methylbenzonitrile | 432.2 |
| | 1H-NMR (300 MHz, CDCl3): δ 8.03 (s, 1H), 7.70 (m, 1H), 7.47 (m, 1H), 7.18 (m, 1H), 7.08 (s, 1H), 5.75 (m, 1H), 5.22 (s, 1H), 4.00 (s, 3H), 3.79 (m, 4H), 3.72 (s, 2H), 2.78 (s, 3H), 2.69 (s, 3H), 2.58 (m, 4H), 1.64 (m, 3H). | | |
| 29 | 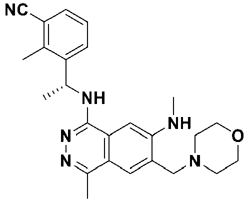 | (R)-2-methyl-3-(1-((4-methyl-7-(methylamino)-6-(morpholinomethyl)phthalazin-1-yl)amino)ethyl)benzonitrile | 431.3 |
| | 1H-NMR (300 MHz, DMSO-d6): δ 7.78-7.75 (m, 1H), 7.59-7.57 (m, 2H), 7.31-7.23 (m, 2H), 7.16 (s, 1H), 6.83-6.78 (m, 1H), 5.58-5.53 (m, 1H), 3.63-3.57 (m, 6H), 3.00-2.98 (m, 3H), 2.67 (s, 3H), 2.48 (s, 3H), 2.38-2.35 (m, 4H), 1.55-1.53 (m, 3H). | | |
| 30 | 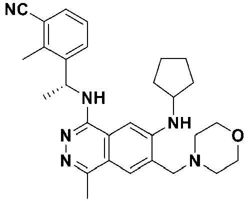 | (R)-3-(1-((7-(cyclopentylamino)-4-methyl-6-(morpholinomethyl)phthalazin-1-yl)amino)ethyl)-2-methylbenzonitrile | 485.3 |
| | 1H-NMR (300 MHz, DMSO-d6): δ 7.77 (d, 1H), 7.60 (m, 2H), 7.32-7.20 (m, 4H), 5.56 (m, 1H), 4.21 (m, 1H), 3.66 (s, 2H), 3.42 (m, 4H), 2.67 (s, 3H), 2.48 (m, 3H), 2.37 (m, 4H), 2.13 (m, 2H), 1.77 (m, 4H), 1.56 (m, 5H). | | |
| 31 | 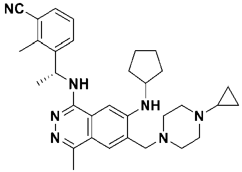 | (R)-3-(1-((7-(cyclopentylamino)-6-((4-cyclopropylpiperazin-1-yl)methyl)-4-methylphthalazin-1-yl)amino)ethyl)-2-methylbenzonitrile | 524.4 |
| | 1H-NMR (300 MHz, DMSO-d6): δ 7.76 (m, 1H), 7.62 (m, 3H), 7.43 (m, 1H), 7.34 (m, 2H), 5.56 (m, 1H), 4.27 (m, 1H), 3.66 (s, 2H), 2.67 (s, 3H), 2.54 (m, 5H), 2.34 (m, 3H), 2.18 (m, 2H), 1.74 (m, 10H), 0.40 (m, 4H). | | |
| 32 | 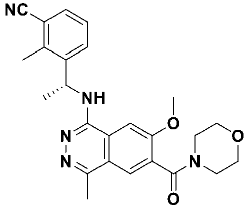 | (R)-3-(1-((7-methoxy-4-methyl-6-(morpholine-4-carbonyl)phthalazin-1-yl)amino)ethyl)-2-methylbenzonitrile | 446.2 |
| | 1H-NMR (300 MHz, DMSO-d6): δ 7.93 (s, 1H), 7.83 (s, 1H), 7.78 (m, 1H), 7.76 (m, 2H), 7.34-7.29 (m, 1H), 5.60 (m, 1H), 4.07 (s, 3H), 3.72-3.67 (m, 4H), 3.65-3.62 (m, 2H), 3.12 (m, 2H), 2.68 (s, 3H), 2.58 (s, 3H), 1.59 (m, 3H). | | |
| 33 | 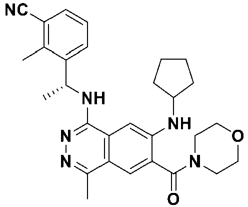 | (R)-3-(1-((7-(cyclopentylamino)-4-methyl-6-(morpholine-4-carbonyl)phthalazin-1-yl)amino)ethyl)-2-methylbenzonitrile | 499.3 |
| | 1H-NMR (300 MHz, DMSO-d6): δ 7.76 (m, 1H), 7.61-7.58 (m, 2H), 7.33-7.28 (m, 3H), 5.76-5.54 (m, 2H), 4.20 (m, 1H), 4.00-3.62 (m, 4H), 3.61-3.29 (m, 2H), 2.54 (s, 3H), 2.48 (m, 3H), 2.15-2.09 (m, 2H), 1.75-1.54 (m, 9H). | | |

### Experimental Example 1: Assay for Inhibitory Activity on SOS1-Mediated GTP Exchange

To evaluate whether the SOS1-mediated nucleotide exchange activity on KRAS G12C is inhibited by the compounds of the above Examples, a homogeneous time-resolved fluorescence (homogenous time-resolved fluorescence, HTRF) assay was performed using GST-KRAS G12C (#MSC-11-538) and SOS1-Strep (#MSC-11-502) obtained from Reaction Biology Corp. (USA, MA). The activity of SOS1 was determined by measuring the amount of GTP bound to the KRAS G12C protein based on the principle of time-resolved fluorescence resonance energy transfer (time-resolved fluorescence energy transfer, TR-FRET). Terbium (Terbium) conjugated to an anti-GST antibody bound to the GST tag of the GST-KRAS G12C protein served as a FRET donor, and GTP bound to the KRAS G12C protein was labeled with the fluorophore DY-647P1, serving as a FRET acceptor. Based on this principle, a higher HTRF or FRET signal is observed as more GTP is bound to KRAS G12C due to the SOS1-mediated exchange reaction in the assay.

In summary, a GST-labeled KRAS G12C protein (comprising amino acid residues 2-169), a streptavidin-tagged SOS1 protein (comprising amino acid residues 564-1049 corresponding to the catalytic domain), an anti-GST antibody, labeled nucleotide GTP-DY-647P1, and the test compound were mixed in a buffer solution containing 20 mM HEPES (pH 7.4), 150 mM NaCl, 5 mM MgCl2, 1 mM dithiothreitol (DTT), 0.05% BSA, and 0.0025% NP40. The mixture was dispensed into a 384-well plate, incubated at room temperature, and the HTRF signal was measured using a PHERAstar microplate reader (BMG Labtech). The excitation wavelength was 337 nm, and emission was measured at 620/665 nm. A background signal value was determined from fluorescence measurements obtained in the absence of SOS1 protein and was subtracted from all measured HTRF values. A control value was determined from fluorescence measurements obtained in the absence of the synthesized compounds and was set as the 100% reference. Fluorescence signals were measured at compound concentrations of 0.1, 1, 10, 100, and 1,000 nM (5 points, 10-fold) or 1.6, 8, 40, 200, and 1,000 nM (5 points, 5-fold). The 50% inhibitory concentration (IC₅₀) values of the compounds were calculated using GraphPad Prism. The results, i.e., the inhibitory activity of the Example compounds against SOS 1-mediated GTP exchange, are shown in Table 2 below.

IC₅₀ values were denoted as +++ when 50 nM or less, ++ when greater than 50 nM and 100 nM or less, and + when greater than 100 nM.

**[Table 2]**

| **Synthesis compound** | **IC₅₀ (nM)** | **Synthesis compound** | **IC₅₀ (nM)** | **Synthesis compound** | **IC₅₀ (nM)** |
|---|---|---|---|---|---|
| Example 1 | +++ | Example 12 | +++ | Example 23 | +++ |
| Example 2 | +++ | Example 13 | +++ | Example 24 | +++ |
| Example 3 | +++ | Example 14 | +++ | Example 25 | +++ |
| Example 4 | +++ | Example 15 | +++ | Example 26 | +++ |
| Example 5 | +++ | Example 16 | +++ | Example 27 | +++ |
| Example 6 | +++ | Example 17 | +++ | Example 28 | +++ |
| Example 7 | +++ | Example 18 | +++ | Example 29 | +++ |
| Example 8 | +++ | Example 19 | +++ | Example 30 | +++ |
| Example 9 | +++ | Example 20 | +++ | Example 31 | +++ |
| Example 10 | +++ | Example 21 | +++ | Example 32 | +++ |
| Example 11 | +++ | Example 22 | +++ | Example 33 | +++ |

The above experimental results suggest that the phthalazine derivative compounds according to the present invention exhibit excellent activity in inhibiting the binding between SOS 1 and RAS family proteins, and thus may be used in the development of various pharmaceuticals for cancers or tumors treatable by such inhibition.

## Claims

1. A compound selected from compounds of Chemical Formula 1 below, optical isomers thereof, diastereomers thereof, hydrates thereof, and solvates thereof , and pharmaceutically acceptable salts thereof: 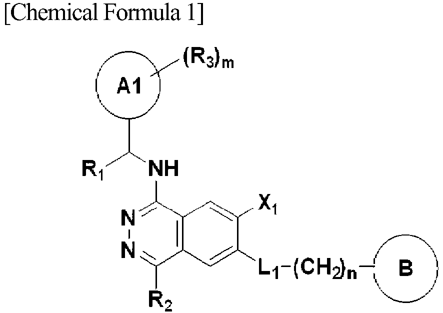 In Chemical Formula 1,
R₁ is hydrogen or C₁₋₄ alkyl;
R₂ is hydrogen, C₁₋₄ alkyl, halo C₁₋₄ alkyl, C₂₋₆ alkenyl or C₂₋₆ alkynyl;
R₃ is R₃ₐ or -L₂- 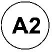 ;
R₃ₐ are each independently halogen, hydroxy, cyano, amino, amine, nitro, oxo (=O), C₁₋₆ alkyl, C₂₋₆ alkenyl, C₂₋₆ alkynyl, halo C₁₋₆ alkyl, amino C₁₋₆ alkyl, C₁₋₆ alkoxy, hydroxy-C₁₋₄ alkyl, - CF₂H, -(CH₂)ᵣ-NH(CO)-Rₐ, or -(CH₂)ᵣ-NRₐR_{b}, and Rₐ and R_{b} are each independently any one selected from the group consisting of hydrogen, C₁₋₆ alkyl, halo C₁₋₆ alkyl, hydroxy-C₁₋₆ alkyl, C₂₋₆ alkenyl, C₂₋₆ alkynyl, and C₃₋₈ carbocyclyl; and wherein the C₁₋₆ alkyl, C₂₋₆ alkenyl, C₂₋₆ alkynyl, amino C₁₋₆ alkyl, C₁₋₆ alkoxy, and hydroxy-C₁₋₄ alkyl are optionally substituted with one or more halogens;
r is an integer in the range of 0 to 1;
m is an integer in the range of 0 to 5;
L₂ is a direct bond, -O-(CH₂)ₚ, or -CH=CH-(CH₂)_{q};
p is an integer in the range of 0 to 3;
q is an integer in the range of 0 to 2;
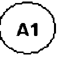 and 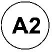 are each independently C₆₋₁₀ aryl, C₄₋₁₀ heteroaryl, C₃₋₁₀ carbocyclyl, C₂₋₁₀ heterocyclyl, or C₉₋₁₂ bicyclic heterocyclyl, wherein C₆₋₁₀ aryl, C₄₋₁₀ heteroaryl, C₃₋₁₀ carbocyclyl, C₂₋₁₀ heterocyclyl, or C₉₋₁₂ bicyclic heterocyclyl of 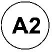 is unsubstituted or substituted with one or more R_{3a,}
X₁ is -O(R₄) or -N(R₅)(R₆);
R₄ is hydrogen, C₁₋₆ alkyl, hydroxy C₁₋₆ alkyl, C₂₋₆ alkenyl, C₂₋₆ alkynyl, C₁₋₆ alkoxy, C₃₋₁₀ carbocyclyl, C₂₋₉ heterocyclyl, C₆₋₁₀ aryl, C₄₋₁₀ heteroaryl, C₈₋₁₆ spirocarbocyclyl, C₆₋₁₄ heterospirocarbocyclyl, C₈₋₁₆ fused carbocyclyl, C₆₋₁₄ fused heterocyclyl, C₈₋₁₆ bridge carbocyclyl, or C₆₋₁₄ bridged heterocyclyl each of which is unsubstituted or substituted with halogen, hydroxy, nitro, oxo (=O), cyano, halo C₁₋₆ alkyl, C₁₋₆ alkyl, C₂₋₆ alkenyl, C₂₋₆ alkynyl, C₁₋₆ alkoxy, halo C₁₋₆ alkoxy, -S(O)-C₁₋₄ alkyl, -S(O)₂-C₁₋₄ alkyl, -C(O)-NR_{c}R_{d}, -C(O)OR_{c}, -OR_{c}, or -NR_{c}R_{d}, wherein R_{c} and R_{d} are each independently hydrogen or C₁₋₆ alkyl;
R₅ and R₆ are each hydrogen, C₁₋₆ alkyl, hydroxy C₁₋₆ alkyl, C₂₋₆ alkenyl, C₂₋₆ alkynyl, C₃₋₁₀ carbocyclyl, C₂₋₉ heterocyclyl, C₆₋₁₀ aryl, C₄₋₁₀ heteroaryl, C₈₋₁₆ spirocarbocyclyl, C₆₋₁₄ heterospirocarbocyclyl, C₈₋₁₆ fused carbocyclyl, C₆₋₁₄ fused heterocyclyl, C₈₋₁₆ bridged carbocyclyl, or C₆₋₁₄ bridged heterocyclyl, wherein C₁₋₆ alkyl, hydroxy C₁₋₆ alkyl, C₂₋₆ alkenyl, C₂₋₆ alkynyl, C₃₋₁₀ carbocyclyl, C₂₋₉ heterocyclyl, C₆₋₁₀ aryl, C₄₋₁₀ heteroaryl, C₈₋₁₆ spirocarbocyclyl, C₆₋₁₄ heterospirocarbocyclyl, C₈₋₁₆ fused carbocyclyl, C₆₋₁₄ fused heterocyclyl, C₈₋₁₆ bridged carbocyclyl, or C₆₋₁₄ bridged heterocyclyl is unsubstituted or substituted with one or more functional groups selected from the group consisting of halogen, hydroxy, nitro, oxo (=O), cyano, halo C₁₋₆ alkyl, C₁₋₆ alkyl, C₂₋₆ alkenyl, C₂₋₆ alkynyl, C₁₋₆ alkoxy, halo C₁₋₆ alkoxy, -S(O)-C₁₋₄ alkyl, -S(O)₂-C₁₋₄ alkyl, - C(O)-NRₑR_{f}, -C(O)ORₑ, -ORₑ, and -NRₑR_{f}, wherein Rₑ and R_{f} are each independently hydrogen or C₁₋₆ alkyl, or
alternatively, the -N(R₅)(R₆) is C₂₋₉ heterocyclyl, C₆₋₁₄ heterospirocarbocyclyl, C₆₋₁₄ fused heterocyclyl, C₆₋₁₄ bridged heterocyclyl, or C₄₋₁₀ heteroaryl in each of which R₅ and R₆ are linked to each other and form a ring in conjunction with a nitrogen atom contained in the -N(R₅)(R₆), in which C₂₋₉ heterocyclyl, C₆₋₁₄ heterospirocarbocyclyl, C₆₋₁₄ fused heterocyclyl, C₆₋₁₄ bridged heterocyclyl, or C₄₋₁₀ heteroaryl is unsubstituted or substituted with one or more functional groups selected from the group consisting of halogen, hydroxy, nitro, oxo (=O), cyano, halo C₁₋₆ alkyl, C₁₋₆ alkyl, C₂₋₆ alkenyl, C₂₋₆ alkynyl, C₁₋₆ alkoxy, halo C₁₋₆ alkoxy, -S(O)-C₁₋₄ alkyl, -S(O)₂-C₁₋₄ alkyl, -C(O)-NR_{g}Rₕ, -C(O)OR_{g}, -OR_{g}, and -NR_{g}Rₕ, wherein R_{g} and Rₕ are each independently hydrogen, C₁₋₆ alkyl, hydroxy-C₁₋₆ alkyl, C₂₋₆ alkenyl, C₂₋₆ alkynyl, C₁₋₆ alkoxy, or C₃₋₈ carbocyclyl;
L₁ is a direct bond, -C(O)-, -O-, -NH-, -C(O)NH- or -C(O)NCH₃- ;
n is an integer in the range of 0 to 2; and
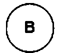 is C₃₋₁₀ carbocyclyl, C₂₋₉ heterocyclyl, C₆₋₁₀ aryl, C₄₋₁₀ heteroaryl, C₄₋₁₄ heterospirocarbocyclyl, C₄₋₁₄ fused heterocyclyl, or C₆₋₁₄ bridged heterocyclyl, wherein C₃₋₁₀ carbocyclyl, C₂₋₉ heterocyclyl, C₆₋₁₀ aryl, C₄₋₁₀ heteroaryl, C₄₋₁₄ heterospirocarbocyclyl, C₄₋₁₄ fused heterocyclyl, or C₆₋₁₄ bridged heterocyclyl is unsubstituted or substituted with one or more functional groups selected from the group consisting of halogen, hydroxy, nitro, oxo (=O), halo C₁₋₆ alkyl, C₁₋₆ alkyl, C₂₋₆ alkenyl, C₂₋₆ alkynyl, C₁₋₆ alkoxy, -S(O)-C₁₋₄ alkyl, -S(O)₂-C₁₋₄ alkyl, -C(O)-NRᵢRⱼ, - C(O)ORᵢ, -ORᵢ,, -NRᵢRⱼ, C₃₋₁₀ carbocyclyl, and C₂₋₉ heterocyclyl, wherein Rᵢ and Rⱼ are each independently hydrogen or C₁₋₆ alkyl.

2. The compound according to claim 1, wherein 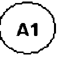 and 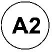 are each independently C₆₋₁₀ aryl or C₄₋₁₀ heteroaryl.

3. The compound according to claim 1, wherein R₃ₐ is each independently halogen, hydroxy, cyano, amino, amine, nitro, C₁₋₆ alkyl, halo C₁₋₆ alkyl, amino C₁₋₆ alkyl, C₁₋₆ alkoxy, -CF₂H, -(CH₂)ᵣ-NH(CO)-Rₐ or -(CH₂)ᵣ-NRₐR_{b}, wherein Rₐ and R_{b} are each independently hydrogen, C₁₋₆ alkyl, -CF₃, or -CF₂H, and wherein the C₁₋₆ alkyl is optionally substituted with one or more halogens.

4. The compound according to claim 1, wherein R₄ is hydrogen, C₁₋₆ alkyl, C₂₋₆ alkenyl, C₂₋₆ alkynyl, C₃₋₁₀ carbocyclyl, or C₂₋₉ heterocyclyl, and
R₅ and R₆ are each independently hydrogen, C₁₋₆ alkyl, C₂₋₆ alkenyl, C₂₋₆ alkynyl, C₃₋₁₀ carbocyclyl, or C₂₋₉ heterocyclyl, or the -N(R₅)(R₆) is C₂₋₉ heterocyclyl.

5. The compound according to claim 1, wherein the compound represented by Chemical Formula 1 is a compound represented by Chemical Formula 2 below: 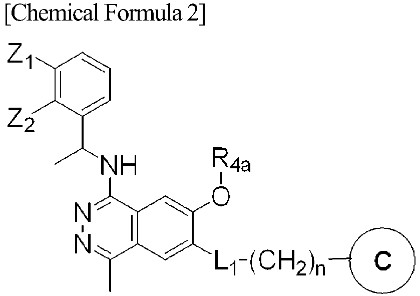 In Chemical Formula 2,
L₁ is a direct bond, -C(O)-, -O-, -NH-, -C(O)NH- or -C(O)NCH₃;
n is an integer in the range of 0 to 2; and
Z₁ and Z₂ are each independently hydrogen, -CN, -F, -CF₂H, -CF₃, -CH₃, or -NH₂, but for the case in which Z₁ and Z₂ are both hydrogen;
R₄ₐ is hydrogen, C₁₋₆ alkyl, C₃₋₁₀ carbocyclyl, or C₂₋₉ heterocyclyl;
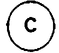 is morpholinyl, thiomorpholinyl, dioxothiomorpholinyl, piperazinyl, thiazolidinyl, tetrahydropyranyl, hexahydro-1*H*-furo[3,4-c]pyrrolyl, oxetanyl or azetidinyl, wherein morpholinyl, thiomorpholinyl, dioxothiomorpholinyl, piperazinyl, thiazolidinyl, tetrahydropyranyl, hexahydro-1*H*-furo[3,4-c]pyrrolyl, oxetanyl or azetidinyl is unsubstituted or substituted with one or more functional groups selected from the group consisting of halogen, -C₁₋₃ alkyl , C₃₋₁₀ carbocyclyl, and C₂₋₉ heterocyclyl.

6. The compound according to claim 1, wherein the compound represented by Chemical Formula 1 is a compound represented by Chemical Formula 3 below: 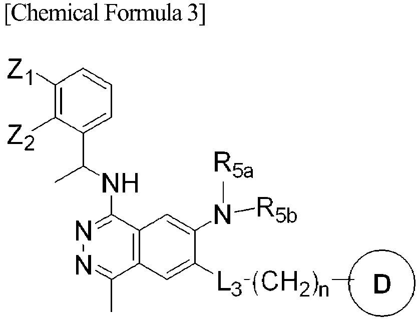 In Chemical Formula 3,
L₃ is a direct bond, -C(O)-, -C(O)NH- or -C(O)NCH₃;
n is an integer in the range of 0 to 2; and
Z₁ and Z₂ are each independently hydrogen, -CN, -F, -CF₂H, -CF₃, -CH₃, or -NH₂, but for the case in which Z₁ and Z₂ are both hydrogen;
R₅ₐ and R_{5b} are each independently hydrogen, C₁₋₆ alkyl, C₃₋₁₀ carbocyclyl, or C₂₋₉ heterocyclyl, or
alternatively the -N(R₅ₐ)(R_{5b}) is a C₂₋₉ heterocyclyl in which R₅ₐ and R_{5b} are linked to each other and form a ring in conjunction with a nitrogen atom contained in the -N(R₅ₐ)(R_{5b});
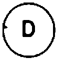 is morpholinyl, dioxothiomorpholinyl, thiomorpholinyl, piperazinyl, thiazolidinyl, tetrahydropyranyl, hexahydro-1*H*-furo[3,4-c]pyrrolyl, oxetanyl or azetidinyl, wherein morpholinyl, dioxothiomorpholinyl, thiomorpholinyl, piperazinyl, thiazolidinyl, tetrahydropyranyl, hexahydro-1*H*-furo[3,4-c]pyrrolyl, oxetanyl or azetidinyl is unsubstituted or substituted with one or more functional groups selected from the group consisting of halogen, -C₁₋₃ alkyl , C₃₋₁₀ carbocyclyl, and C₂₋₉ heterocyclyl.

7. The compound according to claim 5, wherein the compound represented by Chemical Formula 2 is a compound represented by Chemical Formula 4 below: 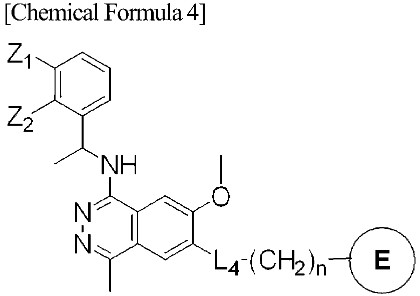 In Chemical Formula 4,
L₄ is a direct bond, -C(O)- , -O-, -C(O)NH- or -C(O)NCH₃;
n is an integer in the range of 0 to 2; and
Z₁ and Z₂ are each independently hydrogen, -CN, -F, -CF₂H, -CF₃, -CH₃, or -NH₂, but for the case in which Z₁ and Z₂ are both hydrogen;
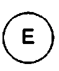 is morpholinyl, thiomorpholinyl, piperazinyl, thiazolidinyl, or tetrahydropyranyl, wherein the morpholinyl, thiomorpholinyl, piperazinyl, thiazolidinyl, or tetrahydropyranyl is unsubstituted or substituted with one or more functional groups selected from the group consisting of halogen, -C₁₋₃ alkyl , C₃₋₁₀ carbocyclyl, and C₂₋₉ heterocyclyl.

8. The compound according to claim 6, wherein the compound represented by Chemical Formula 3 is a compound represented by Chemical Formula 5 below: 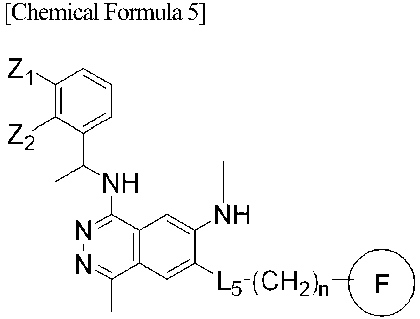 In Chemical Formula 5,
L₅ is a direct bond, -C(O)- , -O-, -C(O)NH- or -C(O)NCH₃;
n is an integer in the range of 0 to 2; and
Z₁ and Z₂ are each independently hydrogen, -F, -CN, -CF₂H, -CF₃, -CH₃, or -NH₂, but for the case in which Z₁ and Z₂ are both hydrogen;
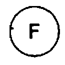 is morpholinyl, dioxothiomorpholinyl, thiomorpholinyl, piperazinyl, thiazolidinyl, or hexahydro-1*H*-furo[3,4-c]pyrrolyl, wherein the morpholinyl, dioxothiomorpholinyl, thiomorpholinyl, piperazinyl, thiazolidinyl, or hexahydro-1*H*-furo[3,4-c]pyrrolyl is unsubstituted or substituted with one or more functional groups selected from the group consisting of halogen, -C₁₋₃ alkyl , C₃₋₁₀ carbocyclyl, and C₂₋₉ heterocyclyl.

9. The compound according to claim 1, wherein the compound of Chemical Formula 1 is a compound selected from the group consisting of:
(*R*)-2-methyl-3-(1-((4-methyl-7-(methylamino)-6-(morpholine-4-carbonyl)phthalazin-1-yl)amino)ethyl)benzonitrile;
(*R*)-2-methyl-3-(1-((4-methyl-7-(methylamino)-6-(thiomorpholine-4-carbonyl)phthalazin-1-yl)amino)ethyl)benzonitrile;
(*R*)-2-methyl-3-(1-((4-methyl-7-(methylamino)-6-(thiazolidine-3-carbonyl)phthalazin-1-yl)amino)ethyl)benzonitrile;
(*R*)-3-(1-((6-(1,1-dioxothiomorpholine-4-carbonyl)-4-methyl-7-(methylamino)phthalazin-1-yl)amino)ethyl)-2-methylbenzonitrile;
(*R*)-1-((1-(3-cyano-2-methylphenyl)ethyl)amino)-4-methyl-7-(methylamino)-N-(tetrahydro-2H-pyran-4-yl)phthalazin-6-carboxamide;
(*R*)-1-((1-(3-cyano-2-methylphenyl)ethyl)amino)-N,4-dimethyl-7-(methylamino)-N-(tetrahydro-2H-pyran-4-yl)phthalazin-6-carboxamide;
3-((1R)-1-((6-(hexahydro-1H-furo[3,4-c]pyrrole-5-carbonyl)-4-methyl-7-(methylamino)phthalazin-1-yl)amino)ethyl)-2-methylbenzonitrile;
(*R*)-2-methyl-3-(1-((4-methyl-7-(methylamino)-6-(4-methylpiperazin-1-carbonyl)phthalazin-1-yl)amino)ethyl)benzonitrile;
(*R*)-2-methyl-3-(1-((4-methyl-7-(methylamino)-6-(piperazin-1-carbonyl)phthalazin-1-yl)amino)ethyl)benzonitrile;
(*R*)-3-(1-((6-(4,4-difluoropiperidin-1-carbonyl)-4-methyl-7-(methylamino)phthalazin-1-yl)amino)ethyl)-2-methylbenzonitrile;
(*R*)-2-methyl-3-(1-((4-methyl-7-(methylamino)-6-(2-oxa-6-azaspiro[3.3]heptane-6-carbonyl)phthalazin-1-yl)amino)ethyl)benzonitrile;
(*R*)-3-(1-((6-(4-ethylpiperazin-1-carbonyl)-4-methyl-7-(methylamino)phthalazin-1-yl)amino)ethyl)-2-methylbenzonitrile;
(*R*)-3-(1-((6-(4-cyclopropylpiperazin-1-carbonyl)-4-methyl-7-(methylamino)phthalazin-1-yl)amino)ethyl)-2-methylbenzonitrile;
(*R*)-2-methyl-3-(1-((4-methyl-7-(methylamino)-6-(pyrrolidin-1-carbonyl)phthalazin-1-yl)amino)ethyl)benzonitrile;
(*R*)-2-methyl-3-(1-((4-methyl-7-(methylamino)-6-(piperidin-1-carbonyl)phthalazin-1-yl)amino)ethyl)benzonitrile;
(*R*)-3-(1-((6-(azetidin-1-carbonyl)-4-methyl-7-(methylamino)phthalazin-1-yl)amino)ethyl)-2-methylbenzonitrile;
(*R*)-3-(1-((6-(3-fluoroazetidin-1-carbonyl)-4-methyl-7-(methylamino)phthalazin-1-yl)amino)ethyl)-2-methylbenzonitrile;
(*R*)-2-methyl-3-(1-((4-methyl-7-(methylamino)-6-(5-azaspiro[2.4]heptane-5-carbonyl)phthalazin-1-yl)amino)ethyl)benzonitrile;
3-((1R)-1-((6-(2-oxa-5-azabicyclo[2.2.1]heptane-5-carbonyl)-4-methyl-7-(methylamino)phthalazin-1-yl)amino)ethyl)-2-methylbenzonitrile;
(*R*)-3-(1-((6-(4-cyclohexylpiperazin-1-carbonyl)-4-methyl-7-(methylamino)phthalazin-1-yl)amino)ethyl)-2-methylbenzonitrile;
(*R*)-3-(1-((6-(4-isopropylpiperazin-1-carbonyl)-4-methyl-7-(methylamino)phthalazin-1-yl)amino)ethyl)-2-methylbenzonitrile;
(*R*)-2-methyl-3-(1-((4-methyl-7-(methylamino)-6-(4-morpholinopiperidin-1-carbonyl)phthalazin-1-yl)amino)ethyl)benzonitrile;
(*R*)-1-((1-(3-cyano-2-methylphenyl)ethyl)amino)-4-methyl-7-(methylamino)-N-(2-morpholinoethyl)phthalazin-6-carboxamide;
(*R*)-2-methyl-3-(1-((4-methyl-7-(methylamino)-6-(1,4-oxazepane-4-carbonyl)phthalazin-1-yl)amino)ethyl)benzonitrile;
(*R*)-(1-((1-(3-amino-5-(trifluoromethyl)phenyl)ethyl)amino)-4-methyl-7-(methylamino)phthalazin-6-yl)(morpholino)methanone;
(*R*)-(1-((1-(5-amino-3-(difluoromethyl)-2-fluorophenyl)ethyl)amino)-4-methyl-7-(methylamino)phthalazin-6-yl)(morpholino)methanone;
(*R*)-(1-((1-(3-(1,1-difluoro-2-hydroxyethyl)phenyl)ethyl)amino)-4-methyl-7-(methylamino)phthalazin-6-yl)(morpholino)methanone;
(*R*)-3-(1-((7-methoxy-4-methyl-6-(morpholinomethyl)phthalazin-1-yl)amino)ethyl)-2-methylbenzonitrile;
(*R*)-2-methyl-3-(1-((4-methyl-7-(methylamino)-6-(morpholinomethyl)phthalazin-1-yl)amino)ethyl)benzonitrile;
(*R*)-3-(1-((7-(cyclopentylamino)-4-methyl-6-(morpholinomethyl)phthalazin-1-yl)amino)ethyl)-2-methylbenzonitrile;
(*R*)-3-(1-((7-(cyclopentylamino)-6-((4-cyclopropylpiperazin-1-yl)methyl)-4-methylphthalazin-1-yl)amino)ethyl)-2-methylbenzonitrile;
(*R*)-3-(1-((7-methoxy-4-methyl-6-(morpholine-4-carbonyl)phthalazin-1-yl)amino)ethyl)-2-methylbenzonitrile;
(*R*)-3-(1-((7-(cyclopentylamino)-4-methyl-6-(morpholine-4-carbonyl)phthalazin-1-yl)amino)ethyl)-2-methylbenzonitrile.

10. A pharmaceutical composition for preventing or treating a disease associated with inhibition of SOS1 binding to RAS family proteins and/or RAC1, comprising, as an active ingredient, a compound selected from the group consisting of the compound according to any one of claims 1 to 9, optical isomers thereof, diastereomers thereof, hydrates thereof, solvates thereof, and pharmaceutically acceptable salts thereof.

11. The pharmaceutical composition according to claim 10, wherein the pharmaceutical composition is used for preventing or treating cancer or a tumor treatable by inhibiting binding of SOS1 to RAS family proteins and/or RAC1.

12. A method of inhibiting SOS1 binding to RAS family proteins and/or RAC1 in a subject or cell, comprising administering to the subject a pharmaceutically effective amount of the compound of claim 1.

13. A method of preventing or treating cancer in a subject, comprising administering to the subject a pharmaceutically effective amount of the compound of claim 1.

14. Use of the compound of claim 1, or a pharmaceutically acceptable salt thereof, for preventing or treating cancer or a tumor.
